# EUROPÄISCHE PATENTSCHRIFT

(11) **EP 2 097 059 B1**
(45) Veröffentlichungstag und Bekanntmachung des Hinweises auf die Patenterteilung: **18.04.2012**
(21) Anmeldenummer: 07847341.0
(22) Anmeldetag: 26.11.2007
(51) Int. Cl.: A61K 8/02, A61Q 5/02, A61Q 19/10, C11D 3/02, C11D 3/12, C11D 17/04, C11D 3/00, C11D 3/04, C11D 3/50, C11D 3/20, C11D 3/10, C11D 3/06, A61K 8/25, A61K 8/23

(54) **REINIGUNGS- ODER PFLEGEPRODUKT**
CLEANSING OR CARE PRODUCT
PRODUIT D'HYGIÈNE OU DE SOIN

(30) Priorität: 27.11.2006 DE 102006056249
(43) Veröffentlichungstag der Anmeldung: 09.09.2009
(73) Patentinhaber: Henkel Kommanditgesellschaft auf Aktien, 40589 Düsseldorf (DE)
(72) Erfinder: TAYLOR, Tim, Phoenix, AZ 85013 (US); SCHELGES, Heike, 47877 Willich (DE)
(86) Internationale Anmeldenummer: PCT/EP2007/062808
(87) Internationale Veröffentlichungsnummer: WO 2008/065072

(56) Entgegenhaltungen:
- EP-A- 1 172 088
- EP-A- 1 186 650
- EP-A- 1 407 762
- EP-A- 1 634 568
- WO-A-01/12133
- WO-A-01/12147
- WO-A-02/079363
- WO-A-2004/105709
- DE-A1- 10 234 257

## Beschreibung

Die Erfindung betrifft ein Reinigungs- und/oder Pflegeprodukt, umfassend ein Substrat, wie beispielsweise Beutel, Pads, Tücher, insbesondere Vliese, Netze und Schwämme, in Kombination mit einer kosmetischen oder dermatologischen Zusammensetzung, die bei Kontakt mit Wasser Hydratationswärme entwickelt, dabei aber kein Kohlendioxid freisetzt.

Die erfindungsgemäßen Produkte sind für die persönliche Körperpflege geeignet.

Der Markt kennt eine Vielzahl von Reinigungs- und Pflegeprodukten. Für die persönliche Hygiene werden diese in Form von Seifenriegeln, Duschgelen und Moussen vermarktet, wobei die Moussen durch mechanische Spender oder Aerosolspender erzeugt werden. Für die Reinigung harter Oberflächen in Haushalt, Industrie und institutioneller Reinigung werden die Reinigungszusammensetzungen häufig in Kombination mit einem mechanischen Hilfsmittel, beispielsweise einem Schwamm oder Lappen, verwendet, um die Schaumbildung und physikalische Entfernung von Schmutz durch Schrubben zu unterstützen. Auch Pflegezusammensetzungen für harte Oberflächen, beispielsweise Möbel- oder Lackpolituren, werden meist mit einem Lappen oder Schwamm aufgetragen. Üblicherweise werden beide Reinigungsgegenstände - Reinigungszusammensetzung und Träger - im Handel getrennt vertrieben. Erst im Bedarfsfall appliziert der Verbraucher die Reinigungszusammensetzung auf den Träger bzw. tränkt den Träger mit einer Reinigungszusammensetzung.

### Stand der Technik

Mittlerweile haben sich im Markt Reinigungs- und Pflegeprodukte etabliert, die als vorgefertigte, mit der Reinigungs- oder Pflegezusammensetzung imprägnierte Trägersubstrate vorliegen. US 5980931 beschreibt ein trockenes Körperpflegeprodukt, umfassend ein in Wasser unlösliches Vlies oder ähnliches Substrat, das mit einer Tensidlösung getränkt, anschließend getrocknet und in getrockneter Form mit der Anweisung an den Verbraucher vermarktet wird, das trockene Trägersubstrat direkt vor Gebrauch mit Wasser zu benetzen. Eine Wärmeentwicklung ist nicht offenbart. Für die Reinigung von Geschirr und Töpfen im Haushalt sind trockene, mit Tensiden und/oder Abrasivpartikeln ausgerüstete Schwämme ebenfalls schon seit langem bekannt. In US 3250680 sind creme- oder pastenförmige Hautreinigungsmittel offenbart, die als Wärme freisetzende Komponente ein entwässertes Molekularsieb, das heißt einen Zeolith, in einem wasserfreien Träger enthalten. US 5747004 offenbart Zahnpasten, die als Hydratationswärme freisetzendes Salz wasserfreies Natriumcarbonat enthalten, das in Wasser hydrolysiert und einen stark basischen pH-Wert einstellt (pH 9,36 - 10,7). Derart hohe pH-Werte sind für die Anwendung auf der Haut, insbesondere der Gesichtshaut, weniger geeignet und für empfindliche Haut sogar ungeeignet.

Selbsterwärmende Pflegezusammensetzungen, die nicht auf der Entwicklung von Hydratationswärme, sondern auf der Reaktionswärme aus Redoxreaktionen beruhen, sind im Stand der Technik ebenfalls bekannt, beispielsweise aus US 6287580. Diese Zusammensetzungen benötigen zur Ingangsetzung der Reaktion ebenfalls Wasser, derartige Systeme, in denen einzelne Komponenten exotherm mit Wasser reagieren unter Bildung von Reaktionsprodukten aus Wasser, deren Redoxstatus sich dabei gegenüber dem H₂O-Molekül ändert (beispielsweise bei der Bildung von Wasserstoff oder Sauerstoff), fallen aber nicht unter die vorliegend beanspruchten, durch Hydratationswärme selbsterwärmenden Systeme.

DE 102 34 257 A1 offenbart Substrate, insbesondere Tücher, die mit unter Normalbedingungen flüssigen, wasserfreien Lösungen auf der Basis von Polyalkoholen getränkt oder imprägniert sind, die beim Kontakt mit Wasser Wärme freisetzen und zur kosmetischen Reinigung geeignet sind. Derartige Substrate müssen allerdings mit einer relativ hohen Menge an Polyalkoholen getränkt oder imprägniert sein, damit der Wärmeeffekt für den Verbraucher tatsächlich wahrmehmbar ist. Ein einzelnes Substrat kann außerdem nur mit einer begrenzten Menge an Wärme freisetzendem Polyalkohol beaufschlagt werden, so dass die Wärmeentwicklungskapazität eines solchen Substrates von vornherein sehr begrenzt ist. Weiterhin können einige flüssige Polyalkohole, beispielsweise das 1,2-Propylenglycol, Hautunverträglichkeiten hervorrufen. Flüssige Polyalkohole weisen außerdem bekanntermaßen ein sehr klebriges Hautgefühl auf. Ein mit größeren Mengen Polyalkohol ausgerüstetes Substrat gemäß DE 10234257 A1 fühlt sich unangenehm feucht und klebrig an und ist für den Verbraucher nicht attraktiv.

Durch die Wärmeentwicklung eines "self-warming" Produktes wird eine bessere Reinigungsleistung erzielt, beispielsweise durch eine bessere Schmutzan- und ablösung. Bei Anwendungen auf der menschlichen Haut ist ein weiterer Vorteil, dass sich durch die Wärmeentwicklung die Hautporen kurzzeitig erweitern und die pflegenden Effekte kosmetischer und dermatologischer Wirkstoffe gesteigert werden können. Ein weiterer Vorteil selbsterwärmender Zusammensetzungen ist, dass eine Wärmereinigung auch dort erfolgen kann, wo nur kaltes Wasser bereitsteht, beispielsweise unterwegs auf Reisen oder in öffentlichen Waschräumen. Dort, wo im Prinzip auch warmes Wasser zur Verfügung steht, erspart das erfindungsgemäße Produkt das Vorheizen des Wassers.

Die Konfektionierung als trockenes Produkt bietet ebenfalls zahlreiche Vorteile. Wasserfreie oder wasserreduzierte Produkte senken Transport- und Verpackungskosten. Außerdem sind wasserfreie oder wasserreduzierte Produkte wenig anfällig gegenüber mikrobiellem Verderb- Dies ist bedeutsam insbesondere für Nonwoven-Tuchsubstrate und Schwämme, die aufgrund ihrer Porenstruktur und der damit verbundenen sehr großen Oberfläche im feuchten Zustand leicht von Mikroben und Pilzen besiedelt werden und oft nur schwierig zu konservieren sind. Der geringe Wassergehalt der Substrate vermeidet auch das Problem, dass einige Substratmaterialien, beispielsweise Cellulosen, bei längerer Lagerung im feuchten Zustand den pH der Benefit-Zusammensetzung beeinflussen können, was als Präventivmaßnahme eine ausreichende pH-Pufferung der Benefit-Zusammensetzung erfordert. Außerdem ruft ein trockenes Produkt beim ersten Kontakt mit den Händen beim Verbraucher ein angenehmeres Gefühl hervor als ein feucht-kühles Produkt.

Bekannte selbsterwärmende Zusammensetzungen in Form flüssiger oder pastenförmiger Zubereitungen vom Typ einer Flüssigseife oder einer Waschpaste, die in Tuben verpackt sind, können nach den Beobachtungen, die dieser Anmeldung zugrunde liegen, formulierungstechnische Schwierigkeiten aufwerfen, beispielsweise nachträglich so stark verdicken, dass sie nicht mehr aus der Verpackung entnehmbar sind. Dieses Problem kann insbesondere bei Lagerung unter höheren Temperaturen auftreten, einem Problem, das z. B. beim Vertrieb in wärmeren Ländern, während der warmen Jahreszeiten, bei Transport und Lagerung unter unzureichend klimatisierten Bedingungen relevant ist.

Eine weitere Form eines kosmetischen Reinigungsproduktes, das in der letzten Zeit stark an Bedeutung gewonnen hat, ist das bei Kontakt mit Wasser aufschäumende Reinigungskissen oder Reinigungssachet ("pouch, pillow"). Diese für den Verbraucher hochinteressante Applikationsform hat den Vorteil, dass eine fein dosierte Abgabe des Reinigungsmittels während der Anwendung möglich ist.

Aus US 6063390 sind solche Reinigungskissen bekannt. US 6063390 offenbart ein Reinigungsprodukt, das eine, vorzugsweise pulverisierte, Reinigungszusammensetzung mit einer Säure und einem Carbonathaltigen Material, die in einem für Luft/Wasser durchlässigen Beutel oder Sachet enthalten ist, umfasst. Bei Kontakt mit Wasser entwickelt sich aus der Reaktion einer Säure mit einem Carbonat- bzw. Hydrogencarbonat-haltigen Material Kohlendioxid, wodurch die Zusammensetzung aufschäumt und das Reinigungskissen ähnlich einem Schwamm zur Körperreinigung verwendet werden kann. Die Reinigungskissen gemäß US 6063390 zeigen allerdings keinen für den Verbraucher wahrnehmbaren Wärmeeffekt beim Kontakt mit Wasser. CA 2325037 A1 sowie weitere Schriften offenbaren Tabletten, die eine Säure, ein Carbonat-haltiges Material und ein Salz enthalten, das im Kontakt mit Wasser Hydratationswärme entwickelt. Diese Tabletten entwickeln beim Kontakt mit Wasser neben Wärme auch Kohlendioxd. Bei der Entwicklung der erfindungsgemäßen Produkte wurde jedoch beobachtet, dass diese gekoppelte CO₂/Wärmeentwicklung auf einem Substrat, beispielsweise einem Tuch, Schwamm oder Pad, nicht zu einem signifikanten Wärmeempfinden des Verbrauchers führt und kein zufriedenstellendes Produkt ergibt.

### Aufgabenstellung

Eine Aufgabe der vorliegenden Erfindung war es, die für den Verbraucher attraktiven Angebotsformen der Pads, Tücher und "Reinigungskissen" weiter zu optimieren und ihre Akzeptanz durch den Anwender zu erhöhen. Eine weitere Aufgabe der vorliegenden Erfindung war es, die verbraucherattraktive Angebotsform der Pads, Tücher und "Reinigungskissen" mit dem Benefit des "self-warming"-Effekts auszustatten. Hierbei sollten insbesondere das Hautgefühl und/oder die Lagerstabilität erhöht werden. In Verbesserung bekannter Produkte sollte weiterhin eine lang anhaltende, ausreichend hohe, aber dennoch kontrollierte und als angenehm zu empfindende Wärmeentwicklung erzielt werden.

Überraschenderweise wurden nun Produkte gefunden, die die Nachteile des Standes der Technik überwinden. Die Konfektionierung einer selbsterwärmenden Zusammensetzung als gebrauchsfertiges Produkt mit Träger (Beutel, Pad, Schwamm etc.) und Reinigungszusammensetzung erleichtert dem Verbraucher die Anwendung und vermeidet darüber hinaus formulierungstechnische Probleme, die bei einer Darreichung in Bulk-Form auftreten können.

Ein erster Gegenstand der vorliegenden Anmeldung ist ein Produkt zur persönlichen Reinigung und/oder zur persönlichen Pflege, umfassend
a) eine Benefit-Zusammensetzung, die bei Kontakt mit Wasser Hydratationswärme freisetzt und die bei Kontakt mit Wasser kein Kohlendioxid freisetzt, enthaltend
   i) mindestens 1 Gew.-% mindestens einer unter Normalbedingungen festen Substanz, die sich in Wasser mit negativer Lösungsenthalpie löst oder in Kontakt mit Wasser Hydratationswärme freisetzt, ausgewählt aus
      A) anorganischen Salzen,
      B) organischen Salzen,
      C) sowie Mischungen hiervon,
   ii) und mindestens einen Wirkstoff, ausgewählt aus Tensiden, kosmetischen Wirkstoffen und dermatologischen Wirkstoffen, antibakteriellen Stoffen, Antioxidantien, Blondier- oder Bleichwirkstoffen, Enzymen, Farbstoff(vorprodukt)en, Fettstoffen, Fungiziden, Germiziden, geruchskomplexierenden Substanzen, Hydrophobiermitteln, Riechstoffen, UV-Schutz-Substanzen und/oder weichmachenden Komponenten,
   iii) 0 - 10 Gew.-%, bevorzugt 0,5 - 7 Gew.-%, besonders bevorzugt 1 - 5 Gew.-% und außerordentlich bevorzugt 2 - 3 Gew.-% Wasser, jeweils bezogen auf das Gesamtgewicht der Benefit-Zusammensetzung,
   iv) mindestens einen unter Normalbedingungen festen, teilchenförmigen inerten Füllstoff, wobei in der Benefit-Zusammensetzung die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an festen, teilchenförmigen inerten Füllstoffen in einem Gewichtsverhältnis zueinander von 0,1 - 20 enthalten sind; und
b) ein Trägersubstrat,
   dadurch gekennzeichnet, dass die Benefit-Zusammensetzung pulverförmig und/oder agglomeriert und/oder granuliert vorliegt und weiterhin dadurch gekennzeichnet, dass die Benefit-Zusammensetzung nicht gleichzeitig eine oder mehrere Säuren und ein Carbonat- und/oder Hydrogencarbonat-haltiges Material umfasst, und weiterhin dadurch gekennzeichnet, dass die Benefit-Zusammensetzung keine Zeolithe enthält.

"Normalbedingungen" sind im Sinne der vorliegenden Anmeldung eine Temperatur von 20 °C und ein Druck von 1013,25 mbar.

Damit der Effekt der Selbsterwärmung optimal zu Tage tritt, sollte die Benefit-Zusammensetzung nur einen begrenzten Anteil an Wasser enthalten. Erfindungsgemäße Reinigungs- und Pflegeprodukte sind daher dadurch gekennzeichnet, dass die Benefit-Zusammensetzung 0 - 10 Gew.-%, bevorzugt 0,5 - 7 Gew.-%, besonders bevorzugt 1 - 5 Gew.-% und außerordentlich bevorzugt 2 - 3 Gew.-% Wasser, jeweils bezogen auf das Gesamtgewicht der Benefit-Zusammensetzung, enthält. Kristallwasser wird nicht als Wasser für die Zwecke der Definition des Wassergehaltes angesehen. Jedoch ist es bevorzugt, auch den Kristallwassergehalt zu minimieren, vorzugsweise zu entfernen. Eine bevorzugte Methode zur Wassergehaltsbestimmung ist das Trocknen der Benefit-Zusammensetzung bei 110° C bis zur Gewichtskonstanz.

Die erfindungsgemäßen Reinigungs- oder Pflegeprodukte sind dadurch gekennzeichnet, dass die mindestens eine unter Normalbedingungen feste Substanz, die sich in Wasser mit negativer Lösungsenthalpie löst oder in Kontakt mit Wasser Hydratationswärme freisetzt, ausgewählt ist aus anorganischen Salzen, organischen Salzen sowie Mischungen hiervon.

Erfindungsgemäß bevorzugte anorganische Salze, die sich in Wasser mit negativer Lösungsenthalpie lösen oder in Kontakt mit Wasser Hydratationswärme freisetzen, sind ausgewählt aus den Ortho- und Pyrophosphaten, Carbonaten, Sesquicarbonaten, Boraten, Chloriden und Sulfaten der Alkali-, Erdalkali- und Erdmetalle sowie des Zinks, jeweils in ganz oder teilweise dehydratisierter Form, sofern diese in Kontakt mit Wasser Hydratationswärme freisetzen. Natriumchlorid beispielsweise weist eine positive Lösungsenthalpie auf und ist daher erfindungsgemäß nicht geeignet. Auch die in Wasser eher schwer löslichen Salze wie Calciumsulfat und Calciumcarbonat sind erfindungsgemäß nicht geeignet. Erfindungsgemäß bevorzugt sind Natriumcarbonat, Calciumchlorid (nur vollständig dehydratisiertes CaCl₂), Magnesiumchlorid (besonders bevorzugt MgCl₂, auch MgCl₂ · 6 H₂O), Magnesiumsulfat (besonders bevorzugt MgSO₄, auch MgSO₄ · H₂O, nicht geeignet MgSO₄ · 6 H₂O), Zinksulfat (besonders bevorzugt ZnSO₄, auch ZnSO₄ · H₂O, nicht geeignet ZnSO₄ · 7 H₂O) und Aluminiumsulfat (besonders bevorzugt Al₂(SO₄)₃ · 6 H₂O, auch Al₂(SO₄)₃ · 18 H₂O). Besonders bevorzugt ist zumindest teilweise dehydratisiertes Magnesiumsulfat, außerordentlich bevorzugt ist vollständig dehydratisiertes Magnesiumsulfat. Erfindungsgemäß bevorzugte organische Salze, die sich in Wasser mit negativer Lösungsenthalpie lösen oder in Kontakt mit Wasser Hydratationswärme freisetzen, sind ausgewählt aus den Citraten und Acetaten der Alkali-, Erdalkali- und Erdmetalle sowie des Zinks, jeweils in ganz oder teilweise dehydratisierter Form, sofern diese in Kontakt mit Wasser Hydratationswärme freisetzen. Besonders bevorzugt sind die Alkalimetall-citrate und -acetate sowie Zinkcitrat, jeweils in ganz oder teilweise dehydratisierter Form. Für die meisten Substanzen ist die Hydratationswärme oder Lösungsenthalpie in Handbüchern der physikalischen Chemie tabelliert, beispielsweise in Lange's Handbook of Chemistry, 11. Auflage, 1973, Seiten 9-106 bis 9-115.

Die oben erwähnten anorganischen und organischen Salze von schwachen Säuren hydrolysieren beim Lösen in Wasser und beeinflussen damit den pH-Wert der kosmetischen Zusammensetzung bei der Anwendung mit Wasser. Dies kann unter Umständen erfindungsgemäß unerwünscht sein. Darüber hinaus sind beispielsweise Acetate und die bei der Hydrolyse entstehende Essigsäure wegen des Geruchs zumindest für die Anwendung in kosmetischen oder dermatologischen Produkten weniger bevorzugt. Daher sind erfindungsgemäß besonders bevorzugte Salze ausgewählt aus den Salzen starker Säuren, insbesondere ausgewählt aus den Sulfaten und Chloriden.

Besonders bevorzugte erfindungsgemäße Produkte sind dadurch gekennzeichnet, dass die Benefit-Zusammensetzungen mindestens eine unter Normalbedingungen feste Substanz, die sich in Wasser mit negativer Lösungsenthalpie löst oder in Kontakt mit Wasser Hydratationswärme freisetzt, in einer Gesamtmenge von 5 - 90 Gew.-%, bevorzugt 8 - 70 Gew.-%, besonders bevorzugt 10 - 30 Gew.-% und außerordentlich bevorzugt 15 - 25 Gew.-%, enthalten.

Weitere besonders bevorzugte erfindungsgemäße Produkte sind dadurch gekennzeichnet, dass die Benefit-Zusammensetzungen das mindestens eine anorganische Salze, das sich in Wasser mit negativer

Lösungsenthalpie löst oder in Kontakt mit Wasser Hydratationswärme freisetzt, in einer Gesamtmenge von 5 - 90 Gew.-%, bevorzugt 8 - 70 Gew.-%, besonders bevorzugt 10 - 30 Gew.-% und außerordentlich bevorzugt 15 - 25 Gew.-%, enthalten.

Weitere besonders bevorzugte erfindungsgemäße Produkte sind dadurch gekennzeichnet, dass die Benefit-Zusammensetzungen das mindestens eine organische Salze, das sich in Wasser mit negativer Lösungsenthalpie löst oder in Kontakt mit Wasser Hydratationswärme freisetzt, in einer Gesamtmenge von 5 - 90 Gew.-%, bevorzugt 8 - 70 Gew.-%, besonders bevorzugt 10 - 30 Gew.-% und außerordentlich bevorzugt 15 - 25 Gew.-%, enthalten.

Weitere besonders bevorzugte erfindungsgemäße Produkte sind dadurch gekennzeichnet, dass die Benefit-Zusammensetzungen eine Mischung aus mindestens einem anorganischen Salz, das sich in Wasser mit negativer Lösungsenthalpie löst oder in Kontakt mit Wasser Hydratationswärme freisetzt und mindestens einem organischen Salz, das sich in Wasser mit negativer Lösungsenthalpie löst oder in Kontakt mit Wasser Hydratationswärme freisetzt, in einer Gesamtmenge von 5 - 90 Gew.-%, bevorzugt 8 - 70 Gew.-%, besonders bevorzugt 10 - 30 Gew.-% und außerordentlich bevorzugt 15 - 25 Gew.-%, enthalten.

Die erfindungsgemäßen Reinigungs- oder Pflegeprodukte sind dadurch gekennzeichnet, dass die Benefit-Zusammensetzung nicht gleichzeitig eine oder mehrere Säuren und ein Carbonat- und/oder Hydrogencarbonat-haltiges Material umfasst. Säure/(Hydrogen)Carbonat-Kombinationen werden im Stand der Technik eingesetzt, um in situ CO₂ als Schaumbildner und/oder Treibmittel zu erzeugen. Es wurde jedoch festgestellt, dass die erfindungsgemäße Lehre der für den Verbraucher wahrnehmbaren Wärmeentwicklung nicht zufriedenstellend umgesetzt werden kann, wenn sie mit einer gleichzeitigen CO₂-Erzeugung einhergeht.

Die erfindungsgemäße Benefit-Zusammensetzung liegt pulverförmig und/oder agglomeriert und/oder granuliert vor.

Unter "Pulver" ist erfindungsgemäß die allgemeine Bezeichnung für eine Form der Zerteilung trockener fester Stoffe, die man durch Zerkleinern, das heißt Zerreiben oder Zerstoßen in der Reibschale (Pulverisieren), Mahlen in Mühlen oder als Folge von Zerstäubungstrocknungen oder Gefriertrocknungen erhält, zu verstehen. Eine besonders feine Zerteilung nennt man oft Atomisierung oder Mikronisierung. Pulver können sich physikalisch recht unterschiedlich verhalten: in Form von Staub oder Rauch ähneln sie Gasen, im Fließverhalten (Sand), beim pneumatischen Transport in Rohrleitungen oder beim Brodeln der Wirbelschicht erinnern Pulver an Flüssigkeiten, und sehr dicht gepackte Pulver können Eigenschaften von Festkörpern zeigen (Tabletten, pulvermetallurgische Produkte, Kohlebriketts etc.). Nach der Korngröße ist eine grobe Einteilung der Pulver in Grobpulver, Feinpulver und Feinstpulver üblich; eine genauere Klassifizierung pulverförmiger Schüttgüter erfolgt über ihre Schüttdichte und durch Siebanalyse. Auch zur Bestimmung der Pulveroberfläche existieren Methoden, z. B. die BET-Methode. Zur Kontrolle der Gleichmäßigkeit von Pulvern bestimmt man die Schüttdichte nach DIN ISO 697: 1984-01. Agglomerate sind Zusammenballungen aus Primärteilchen, Aggregaten oder einer Mischung aus beiden deren Zusammenhalt so gering ist, dass sie mit den üblichen Verfahren der Herstellung von Beschichtungsstoffen oder Druckfarben zerteilt werden können. Sie sind über Kanten und Ecken brückenartig miteinander verbunden. Ihre Oberfläche unterscheidet sich nicht wesentlich von der Summe der Oberflächen aller sie aufbauenden Teilchen (DIN 53206-1: 1972-08). Agglomerate von Pigmenten und Füllstoffen entstehen vornehmlich bei der Trocknung im Verlauf der Pigment- oder Füllstoffherstellung.

Ein Granulatkorn (Granalie) ist ein asymmetrisches Aggregat aus Pulverpartikeln (ganzen Kristallen, Kristallbruchstücken oder Drogenpartikeln). Es weist - im Gegensatz zum Pellet, aber ähnlich wie ein Agglomerat - keine harmonisch geometrische Form auf; die Form einer Kugel, eines Stäbchens, eines Zylinders usw. ist nur ungefähr und andeutungsweise erhalten. Die Oberfläche ist in der Regel uneben und zackig, die Masse in vielen Fällen mehr oder weniger porös. Unter *Granulieren* versteht man das Überführen von Pulverteilchen in Granulatkörner, was z.B. zur Konfektionierung der Arzneiformen in der pharmazeutischen Industrie, für die Düngemittelindustrie, die Kunststoffindustrie und die Waschmittelindustrie wichtig ist. Technische Ausführungen machen häufig von Wirbelschichtverfahren - man spricht heute oft von Prills - Gebrauch. Als Kriterium für den Gebrauchswert von Granulaten können die Bestimmung der Schüttdichte nach DIN-ISO 697: 1984-01 und die Bestimmung des Schüttwinkels nach DIN ISO 4324: 1983-12 herangezogen werden.

Pulverförmige und/oder agglomerierte und/oder granulierte erfindungsgemäße Benefit-Zusammensetzungen enthalten besonders bevorzugt mindestens ein anorganisches Salz und/oder mindestens ein organisches Salz als selbsterwärmende Komponente, die unter Normalbedingungen als Feststoff, insbesondere als pulverförmiger Feststoff, vorliegt. Ein zusätzlicher Gehalt an wasserfreien mehrwertigen C₂ - C₉-Alkanolen mit 2 - 6 Hydroxylgruppen und/oder wasserfreien Polyethylenglycolen mit 3 - 20 Ethylenoxid-Einheiten ist hierbei weniger bevorzugt, da diese Verbindungen unter Normalbedingungen üblicherweise als Flüssigkeit vorliegen. Mit einem zu hohen Anteil an flüssigen Verbindungen lassen sich jedoch keine Benefit-Zusammensetzungen mit zufrieden stellenden pulverförmigen und/oder agglomerierten und/oder granulierten Eigenschaften herstellen.

Pulverförmige und/oder agglomerierte und/oder granulierte Benefit-Zusammensetzungen werden erfindungsgemäß besonders bevorzugt mit einem Beutel als Trägersubstrat kombiniert. Beutel oder auch Kissen ("pouch") als Trägersubstrat werden weiter unten ausführlicher diskutiert.

Bevorzugte erfindungsgemäße Benefit-Zusammensetzungen sind dadurch gekennzeichnet, dass sie mindestens ein Tensid enthalten. Besonders bevorzugt ist das enthaltene Tensid wasserfrei. In einer weiteren bevorzugten Ausführungsform liegt das mindestens eine Tensid unter Normalbedingungen in fester Form vor. Das enthaltene Tensid ist ausgewählt aus anionischen, nichtionischen, zwitterionischen, ampholytischen oder kationischen Tensiden sowie kompatiblen Mischungen hiervon.

Als anionische Tenside eignen sich alle oberflächenaktiven Stoffe, deren Oberflächenaktivität durch ein Anion bedingt ist, das sich durch eine lineare oder verzweigte Alkyl- oder Acylgruppe mit 8 - 20, bevorzugt 10 - 18, besonders bevorzugt 12,13, 14 oder 16 Kohlenstoffatomen auszeichnet, die mit einer Sulfat-, Sulfonat-, Phosphat- oder Carboxylat-Gruppe verknüpft ist, wobei die linearen Alkyl- oder Acylgruppen besonders bevorzugt sind. Weiterhin ist es bevorzugt, dass (technische) Mischungen von Verbindungen mit unterschiedlich langen Alkyl- oder Acylgruppen eingesetzt werden. Ein herausragendes Beispiel sind die von Kokosfettsäuren bzw. Kokosfettalkoholen abgeleiteten Tenside mit einer C-Zahl-Verteilung von 8 - 18. Bevorzugt, insbesondere für kosmetische und/oder dermatologische Anwendungen, eignen sich schäumende anionische Tenside wie z. B.
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobemsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglycolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- sulfatierte Hydroxyalkylpolyethylen- und/oder Hydroxyalkylenpropylenglycolether
- Sulfonate ungesättigter Fettsäuren mit 8 bis 24 C-Atomen und 1 bis 6 Doppelbindungen
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel R¹(OCH₂CH₂)ₙ-O-P(O)(OX)-OR² ,
   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglycolester der Formel R⁷CO(AlkO)ₙSO₃M, in der R⁷CO- für einen linearen oder verzweigten,aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (E1-III) 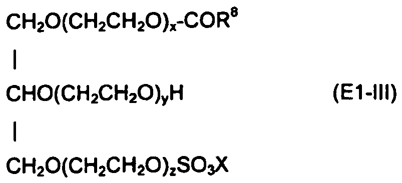 in der R³CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweißfettsäurekondensate bekannt sind, wie beispielsweise die Lamepon^{®} - Typen, Gluadin^{®} - Typen, Hostapon^{®} KCG oder die Amisoft^{®} - Typen.

Die Aniontenside werden jeweils in Form ihrer Salze eingesetzt. Die Salze dieser Tenside sind bevorzugt ausgewählt aus den Natrium-, Kalium- und Ammonium- sowie den Mono-, Di- und Trialkanolammoniumsalzen mit 2 - 4 C-Atomen in der Alkanolgruppe. In der Regel sind die Natriumsalze der genannten Aniontenside unter Normalbedingungen fest, wohingegen die Mono-, Di- und Trialkanolammoniumsalze mit 2 - 4 C-Atomen in der Alkanolgruppe in der Regel unter Normalbedingungen flüssig oder in pastöser, fließfähiger Form vorliegen. Besonders bevorzugt sind die Natriumsalze der genannten Aniontenside, da diese meistens in unter Normalbedingungen fester Form vorliegen. Weniger bevorzugt sind die Alkanolammoniumsalze der genannten Aniontenside, da diese meistens nicht in unter Normalbedingungen fester Form vorliegen. Erfindungsgemäß besonders bevorzugt sind die Sulfobernsteinsäuremonoalkylester-Salze, insbesondere die Natriumsalze und insbesondere mit einem linearen Alkylrest mit 10 - 18, bevorzugt 12 Kohlenstoffatomen, weiterhin die Acylisethionate, insbesondere Natriumcocoylisethionat, die Alkylsulfate, die Alkylethersulfate, insbesondere die Natriumalkylethersulfate mit bevorzugt 1 - 4 Ethylenoxideinheiten, besonders bevorzugt 3 Ethylenoxideinheiten, insbesondere Natriumlaurylethersulfat, die Ethercarbonsäuren mit 10 bis 18 C-Atomen in der Alkylgruppe und bis zu 12 Glycolethergruppen im Molekül, Sulfobernsteinsäuremonoalkylpolyoxyethylester mit 8 bis 18 C-Atomen in der Alkylgruppe und 1 bis 6 Oxyethylgruppen, Monoglyceridsulfate, Alkyl- und Alkenyletherphosphate sowie Eiweißfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders bevorzugte, insbesondere für kosmetische und/oder dermatologische Anwendungen bevorzugte, zwitterionische Tenside sind die sogenannten Betaine, wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, 2-Alkyl-3-carboxymethyl-3-hydroxyethyl-imidazoline und Sulfobetaine mit jeweils 8 bis 18 C-Atomen in der - bevorzugt linearen - Alkyl- oder Acylgruppe sowie das Kokosacylaminoethylhydroxyethylcarboxymethylglycinat. Ein außerordentlich bevorzugtes zwitterionisches Tensid ist das unter der INCl-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder C₈ - C₂₄ - Acylgruppe im Molekül mindestens eine freie Aminogruppe und mindestens eine -COOH- oder -SO₃H-Gruppe enthalten und zur Ausbildung innerer Salze befähigt sind. Beispiele für erfindungsgemäß bevorzugte, insbesondere für kosmetische und/oder dermatologische Anwendungen erfindungsgemäß bevorzugte, ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin. Bevorzugte erfindungsgemäße Produkte sind dadurch gekennzeichnet, dass sie mindestens ein zwitterionisches oder amphoteres Tensid enthalten, das ausgewählt ist aus den Betainen, vorzugsweise N-Alkyl-N,N-dimethylammonium-glycinaten, N-Acyl-aminopropyl-N,N-dimethylammoniumglycinaten, 2-Alkyl-3-carboxymethyl-3-hydroxyethyl-imidazolinen mit jeweils 8 bis 18 C-Atomen in der Alkyl- oder Acylgruppe sowie den unter der INCl-Bezeichnung Cocamidopropyl Betaine bekannten Fettsäureamid-Derivaten und/oder den N-Alkylglycinen, N-Alkylpropionsäuren, N-Alkylaminobuttersäuren, N-Alkyliminodipropionsäuren, N-Hydroxyethyl-N-alkylamidopropylglycinen, N-Alkyltaurinen, N-Alkylsarcosinen, 2-Alkylaminopropionsäuren und Alkylaminoessigsäuren mit jeweils 8 bis 24 C-Atomen in der Alkylgruppe.

Unter nichtionischen Tensiden werden solche Tenside verstanden, die eine lipophile, bevorzugt lineare Alkyl- oder Acylgruppe mit 8-22 C-Atomen und als hydrophile Gruppe einen Glucosid- oder Polyglucosidrest, einen Glycerin- oder Polyglycerinrest, einen Sorbitanrest oder einen Polyglycoletherrest oder mehrere dieser Reste enthalten. Erfindungsgemäß bevorzugte nichtionische Tenside sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, wie beispielsweise die unter der Verkaufsbezeichnung Dehydol^{®} LT (Cognis) erhältlichen Typen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter der Verkaufsbezeichnung Dehydol^{®} LS (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol-Typen (Cognis),
- alkoxylierte Triglyceride,
- alkoxylierte Fettsaurealkylester der Formel R¹²CO-(OCH₂CHR¹³)_{w}OR¹⁴, in der R¹²CO für einen linearen oder verzweigten, gesättigten und/oder ungesättigten Acylrest mit 6 bis 22 Kohlenstoffatomen, R¹³ für Wasserstoff oder Methyl, R¹⁴ für lineare oder verzweigte Alkylreste mit 1 bis 4 Kohlenstoffatomen und w für Zahlen von 1 bis 20 steht,
- Aminoxide, z. B. N,N-Dimethyl-N-kokosalkylaminoxid,
- Amidoalkylaminoxide, z. B. Cocamidopropyldimethylaminoxid,
- Hydroxymischether, beispielsweise gemäß US 2005/0130865 A1,
- Sorbitanfettsäureester und Anlagerungsprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglycoside gemäß Formel R¹⁵O-[G]ₚ, in der R¹⁵ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Die Alkyl- und Alkenyloligoglycoside können sich von Aldosen bzw. Ketosen mit 5 oder 6 Kohlenstoffatomen, vorzugsweise von Glucose, ableiten. Vorzugsweise werden Alkyl- und/oder Alkenyloligoglycoside mit einem mittleren Oligomerisierungsgrad p von 1,1 bis 3,0, bevorzugt kleiner als 1,7, insbesondere 1,2 -1,4 eingesetzt.
- Zuckertenside vom Typ der Fettsäure-N-alkylpolyhydroxyalkylamide der Formel R¹⁶CO-NR¹⁷Z, in der R¹⁶CO für einen aliphatischen Acylrest mit 6 bis 22 Kohlenstoffatomen, R¹⁷ für Wasserstoff, einen Alkyl- oder Hydroxyalkylrest mit 1 bis 4 Kohlenstoffatomen und Z für einen linearen oder verzweigten Polyhydroxyalkylrest mit 3 bis 12 Kohlenstoffatomen und 3 bis 10 Hydroxylgruppen steht.

Als nichtionische Tenside sind nur solche geeignet, die in wasserfreier Form zur Verfügung stehen. Aufgrund ihrer schäumenden und/oder hautverträglichen Eigenschaften besonders bevorzugt sind die Anlagerungsprodukte von Ethylenoxid an Fettalkohole, an Fettsäuren, an Fettsäuremono- oder -diglyceride, an Fettsäurealkanolamide, an Methylglucosidfettsäureester oder an Alkyl- und Alkenyloligoglycoside sowie die Alkyl- und Alkenyloligoglycoside selbst, weiterhin Aminoxide und Amidoalkylaminoxide, wobei die Fettketten lineare oder verzweigte Alkyl- oder Alkenylgruppen mit 5 oder 6 bis 30, bevorzugt 7 oder 8 bis 22 und besonders bevorzugt 11 oder 12 bis 17 oder 18 Kohlenstoffatomen darstellen. Besonders bevorzugt sind die Anlagerungsprodukte von Ethylenoxid an C₁₂-C₁₈-Fettalkohole, die einen durchschnittlichen Ethoxylierungsgrad von 7 - 10 aufweisen, insbesondere Laureth-4, Laureth-7 und Laureth-10 sowie Mischungen dieser Substanzen mit entsprechend ethoxylierten C₁₂ -C₁₈-Fettalkoholen, insbesondere mit ethoxyliertem Myristylalkohol, ethoxyliertem Palmitylalkohol, ethoxyliertem Cetylalkohol und ethoxyliertem Stearylalkohol. Diese Substanzen liegen bei 20°C in flüssiger oder pastöser, fließfähiger Form vor. Entsprechende bevorzugte Handelsprodukte sind beispielsweise Dehydol 100 oder Dehydol LT 7 von Cognis. Weiterhin bevorzugt sind die Alkyl- und Alkenyloligoglycoside, besonders bevorzugt die Alkyloligoglucoside, insbesondere C₁₀-C₁₆- Alkylglucoside mit einer Mischung von Alkylglucosiden mit Alkylkettenlängen mit 10 - 16 Kohlenstoffatomen.

Besonders bevorzugte Zuckertenside vom Typ der Alkyl- und Alkenyloligoglykoside gemäß der vorstehenden Formel R¹⁵O-[G]ₚ weisen einen Alkyl- oder Alkenylrest mit 4 bis 22 Kohlenstoffatomen auf, während G für Glucose steht. Die mittleren Oligomerisierungsgrade p liegen vorzugsweise zwischen 1,1 bis 3,0, besonders bevorzugt kleiner 1,7. Besonders bevorzugte feste Reinigungsmittel a) sind Butylglucosid [dp = 1,0], Capronylglucosid [dp = 1,0], Caprylglucosid [dp = 1,0], Caprinylglucosid [dp = 1,0] und Undecylglucosid [dp = 1,0], Laurylglucosid [dp = 1,0], Myristylglucosid [dp = 1,0]. Cetylglucosid [dp = 1,0], Palmitoleylglucosid [dp = 1,0], Stearylglucosid [dp = 1,0], Isostearylglucosid [dp = 1,0], Oleylglucosid [dp = 1,0], Elaidylglucosid [dp = 1,0], Petroselinylglucosid [dp = 1,0], Arachylglucosid [dp = 1,0], Gadoleylglucosid [dp = 1,0], Behenylglucosid [dp = 1,0], Erucylglucosid [dp = 1,0], Brassidylglucosid [dp = 1,0], Butylglucosid [dp = 1,1], Capronylglucosid [dp = 1,1], Caprylglucosid [dp = 1,1], Caprinylglucosid [dp = 1,1] und Undecylglucosid [dp = 1,1], Laurylglucosid [dp = 1,1], Myristylglucosid [dp = 1,1], Cetylglucosid [dp = 1,1], Palmitoleylglucosid [dp = 1,1], Stearylglucosid [dp = 1,1], Isostearylglucosid [dp = 1,1], Oleylglucosid [dp = 1,1], Elaidylglucosid [dp = 1,1], Petroselinylglucosid [dp = 1,1], Arachylglucosid [dp = 1,1], Gadoleylglucosid [dp = 1,1], Behenylglucosid [dp = 1,1], Erucylglucosid [dp = 1,1], Brassidylglucosid [dp = 1,1], Butylglucosid [dp = 1,2], Capronylglucosid [dp = 1,2], Caprylglucosid [dp = 1,2], Caprinylglucosid [dp = 1,2] und Undecylglucosid [dp = 1,2], Laurylglucosid [dp = 1,2], Myristylglucosid [dp = 1,2], Cetylglucosid [dp = 1,2], Palmitoleylglucosid [dp = 1,2], Stearylglucosid [dp = 1,2], Isostearylglucosid [dp = 1,2], Oleylglucosid [dp = 1,2], Elaidylglucosid [dp = 1,2], Petroselinylglucosid [dp = 1,2], Arachylglucosid [dp = 1,2], Gadoleylglucosid [dp = 1,2], Behenylglucosid [dp = 1,2], Erucylglucosid [dp = 1,2], Brassidylglucosid [dp = 1,2], Butylglucosid [dp = 1,3], Capronylglucosid [dp = 1,3], Caprylglucosid [dp = 1,3], Caprinylglucosid [dp = 1,3] und Undecylglucosid [dp = 1,3], Laurylglucosid [dp = 1,3], Myristylglucosid [dp = 1,3], Cetylglucosid [dp = 1,3], Palmitoleylglucosid [dp = 1,3], Stearylglucosid [dp = 1,3], Isostearylglucosid [dp = 1,3], Oleylglucosid [dp = 1,3], Elaidylglucosid [dp = 1,3], Petroselinylglucosid [dp = 1,3], Arachylglucosid [dp = 1,3], Gadoleylglucosid [dp = 1,3], Behenylglucosid [dp = 1,3], Erucylglucosid [dp = 1,3], Brassidylglucosid [dp = 1,3], Butylglucosid [dp = 1,4], Capronylglucosid [dp = 1,4], Caprylglucosid [dp = 1,4], Caprinylglucosid [dp = 1,4] und Undecylglucosid [dp = 1,4], Laurylglucosid [dp = 1,4], Myristylglucosid [dp = 1,4], Cetylglucosid [dp = 1,4], Palmitoleylglucosid [dp = 1,4], Stearylglucosid [dp = 1,4], Isostearylglucosid [dp = 1,4], Oleylglucosid [dp = 1,4], Elaidylglucosid [dp = 1,4], Petroselinylglucosid [dp = 1,4], Arachylglucosid [dp = 1,4], Gadoleylglucosid [dp = 1,4], Behenylglucosid [dp = 1,4], Erucylglucosid [dp = 1,4], Brassidylglucosid [dp = 1,4], Butylglucosid [dp = 1,5], Capronylglucosid [dp = 1,5], Caprylglucosid [dp = 1,5], Caprinylglucosid [dp = 1,5] und Undecylglucosid [dp = 1,5], Laurylglucosid [dp = 1,5], Myristylglucosid [dp = 1,5], Cetylglucosid [dp = 1,5], Palmitoleylglucosid [dp = 1,5], Stearylglucosid [dp = 1,5], Isostearylglucosid [dp = 1,5], Oleylglucosid [dp = 1,5], Elaidylglucosid [dp = 1,5], Petroselinylglucosid [dp = 1,5], Arachylglucosid [dp = 1,5], Gadoleylglucosid [dp = 1,5], Behenylglucosid [dp = 1,5], Erucylglucosid [dp = 1,5], Brassidylglucosid [dp = 1,5], Butylglucosid [dp = 1,6], Capronylglucosid [dp = 1,6], Caprylglucosid [dp = 1,6], Caprinylglucosid [dp = 1,6] und Undecylglucosid [dp = 1,6], Laurylglucosid [dp = 1,6], Myristylglucosid [dp = 1,6], Cetylglucosid [dp = 1,6], Palmitoleylglucosid [dp = 1,6], Stearylglucosid [dp = 1,6], Isostearylglucosid [dp = 1,6], Oleylglucosid [dp = 1,6], Elaidylglucosid [dp = 1,6], Petroselinylglucosid [dp = 1,6], Arachylglucosid [dp = 1,6], Gadoleylglucosid [dp = 1,6], Behenylglucosid [dp = 1,6], Erucylglucosid [dp = 1,6], Brassidylglucosid [dp = 1,6], Butylglucosid [dp = 1,7], Capronylglucosid [dp = 1,7], Caprylglucosid [dp = 1,7], Caprinylglucosid [dp = 1,7] und Undecylglucosid [dp = 1,7], Laurylglucosid [dp = 1,7], Myristylglucosid [dp = 1,7], Cetylglucosid [dp = 1,7], Palmitoleylglucosid [dp = 1,7], Stearylglucosid [dp = 1,7], Isostearylglucosid [dp = 1,7], Oleylglucosid [dp = 1,7], Elaidylglucosid [dp = 1,7], Petroselinylglucosid [dp = 1,7], Arachylglucosid [dp = 1,7], Gadoleylglucosid [dp = 1,7], Behenylglucosid [dp = 1,7], Erucylglucosid [dp = 1,7], Brassidylglucosid [dp = 1,7].

Eine weitere besonders geeignete Gruppe nichtionischer Tenside sind die Poly-(C₂-C₃)-alkylenglycol-modifizierten Silicone, deren frühere INCl-Bezeichnung Dimethicone Copolyol lautete, mit den aktuellen INCl-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPGe/f PEG/PPG g/h Dimethicone (wobei e, f, g und h unabhängig voneinander für Zahlen von 10 - 20, bevorzugt 14 - 18 und besonders bevorzugt 16 stehen), die z. B. unter den Handelsbezeichnungen Dow Corning 193 (PEG-12 Dimethicone von Dow Corning) oder Abil Care 85 (Bis-PEG/PPG-16/16 PEG/PPG 16/16 Dimethicone von Goldschmidt) im Handel sind.

Erfindungsgemäß bevorzugte kationische Tenside sind ausgewählt aus Tensiden vom Typ der quaternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten der genannten Tenside weisen bevorzugt 10 bis 18 Kohlenstoffatome auf, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quaternium-83 bekannten Imidazolium-Verbindungen.

Besonders bevorzugte kationische Tenside sind sogenannte Esterquats. Hierbei handelt es sich um Verbindungen, die sowohl mindestens eine Esterfunktion als auch mindestens eine quartäre Ammoniumgruppe als Strukturelement enthalten. Erfindungsgemäß bevorzugte Esterquats sind quaternierte Estersalze von C₁₀-C₁₈-Fettsäuren mit Triethanolamin, quaternierte Estersalze von C₁₀-C₁₈-Fettsäuren mit Diethanolalkylaminen und quaternierten Estersalzen von C₁₀-C₁₈-Fettsäuren mit 1,2-Dihydroxypropyldialkylaminen. Solche Produkte werden zum Beispiel unter den Handelsnamen Stepantex^{®}, Dehyquart^{®} und Armocare^{®} vertrieben.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders bevorzugte Verbindung aus dieser Substanzgruppe stellt das unter der Bezeichnung Tegoamid^{®} S 18 im Handel erhältliche Stearamidopropyldimethylamin dar.

Bevorzugte erfindungsgemäße Produkte sind dadurch gekennzeichnet, dass die Benefit-Zusammensetzungen mindestens ein Tensid in einer Gesamtmenge von 0,01 - 95 Gew.-%, bevorzugt 0,5 - 70 Gew.-%, besonders bevorzugt 3 - 50 Gew -% und außerordentlich bevorzugt 10 - 30 Gew.-% jeweils bezogen auf das Gewicht der Benefit-Zusammensetzung, enthalten. Tensidmengen von 15 - 25 Gew.-% oder auch 16 - 20 Gew.-%, jeweils bezogen auf das Gewicht der Benefit-Zusammensetzung, können ebenfalls bevorzugt sein.

Weitere bevorzugte erfindungsgemäße Benefit-Zusammensetzungen sind dadurch gekennzeichnet, dass ein Gewichtsanteil von mindestens 70 %, bevorzugt mindestens 80 %, besonders bevorzugt mindestens 90 % der gesamten Tensidmenge in unter Normalbedingungen fester und/oder granulierter und/oder geträgerter Form vorliegt.

Der Einsatz granulierter und/oder geträgerter Tenside hat erfindungsgemäß den Vorteil, dass so auch solche Tenside in wasserfreier, rieselfähiger und/oder pulverförmiger Form eingesetzt werden können, die unter Normalbedingungen bei der Herstellung pastös oder wachsartig anfallen und in dieser Form schwierig in Einzelportionen für eine Substrat-Benefitzusammensetzung zu dosieren sind. Ein erfindungsgemäß bevorzugtes Beispiel für ein geträgertes Tensid ist der Rohstoff Glucopon 50 G der Firma Cognis, enthaltend 45 - 55 Gew.-% Alkylpolyglucoside, 27 - 32 Gew.-% Natriumsulfat und maximal 5 Gew.-% Wasser.

Weitere bevorzugte erfindungsgemäße Benefit-Zusammensetzungen sind dadurch gekennzeichnet, dass der mindestens eine kosmetische oder dermatologische Wirkstoff ausgewählt ist aus
a) alpha-Hydroxycarbonsäuren, ihren Salzen, Lactonen, Amiden und Estern,
b) beta-Hydroxycarbonsäuren, ihren Salzen, Lactonen, Amiden und Estern,
c) alpha-Ketocarbonsäuren, ihren Salzen, Lactonen, Amiden und Estern,
d) omega-Hydroxycarbonsäuren, ihren Salzen, Lactonen, Amiden und Estern,
e) linearen Dicarbonsäuren, ihren Salzen, Lactonen, Amiden und Estern,
f) Pflanzenextrakten,
g) Monomeren, Oligomeren und Polymeren von Aminosäuren, N-C₂-C₂₄-Acylaminosäuren, den Estern und/oder den physiologisch verträglichen Metallsalzen dieser Substanzen,
h) DNA- oder RNA-Oligonucleotiden,
i) natürlichen Betainverbindungen,
j) Vitaminen, Provitaminen und Vitaminvorstufen der Gruppen A, B, C, E, H und K und den Estern der vorgenannten Substanzen,
k) Flavonoiden und Flavonoid-reichen Pflanzenextrakten,
l) Isoflavonoiden und Isoflavonoid-reichen Pflanzenextrakten,
m) Ubichinon und Ubichinol sowie deren Derivaten,
n) Silymarin,
o) natürlich vorkommenden Xanthin-Derivaten, ausgewählt aus Coffein, Theophyllin, Theobromin und Aminophyllin,
p) Ectoin,
q) Kreatin,
r) Olivenblattextrakten, Ursolsäure, Oleanol und/oder Oleanolsäure,
s) Mono- und Polyhydroxystilbenen und deren Estern,
t) Derivaten von methyliertem Silanol,
u) Phytinsäure,
v) Wirkstoffen, die die beta-Endorphinsynthese in Keratinozyten stimulieren,
w) selbstbräunenden Wirkstoffen,
x) hautaufhellenden Wirkstoffen,
y) Wirkstoffen, die die Prostaglandinsynthese und/oder die Leukotrien-Synthese inhibieren,
z) sebumregulierenden Wirkstoffen,
aa) haarwuchsinhibierenden Wirkstoffen,
bb) Desoxyzuckern oder Desoxyzucker-Bausteine enthaltenden Polysacchariden,
cc) Antischuppenwirkstoffen, sowie
dd) hautberuhigenden Wirkstoffen.

Überraschend zeigte sich, dass die Kombination mit einem durch Wasserzusatz aktivierbaren Selbsterwärmungssystem auf Basis fester Komponenten zu einer unerwarteten Wirksamkeitssteigerung der genannten Wirkstoffe führt. Ohne an diese Hypothese gebunden sein zu wollen, wird vermutet, dass bei der Verwendung flüssiger Wärme entwickelnder Komponenten störende Verdünnungs- oder Kompatibilitätseffekte auftreten, die die Effizienz der Wirkstoffe gegenüber einer Verwendung fester Wärme entwickelnder Komponenten beeinträchtigen. Andererseits ist die geringere Wirksamkeit tatsächlich überraschend, da einige der flüssigen Wärme entwickelnden Komponenten dem Fachmann üblicherweise als Penetrationsverstärker bekannt sind; sie hätten also die Effizienz der Wirkstoffe nicht beeinträchtigen, sondern eher fördern sollen. Anmerkung: die Attribute "fest" und "flüssig" beziehen sich auf den Aggregatzustand bei Normalbedingungen.

Erfindungsgemäß bevorzugte alpha-Hydroxycarbonsäuren und alpha-Ketocarbonsäuren sind ausgewählt aus Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte alpha-Hydroxycarbonsäuren sind Milchsäure, Citronensäure, Glycolsäure und Gluconsäure.

Eine besonders bevorzugte beta-Hydroxycarbonsäure ist Salicylsäure.

Erfindungsgemäß bevorzugte omega-Hydroxycarbonsäuren sind ausgewählt aus 3-Hydroxypropansäure, 4-Hydroxybutansäure, 5-Hydroxypentansäure, 6-Hydroxyhexansäure, 7-Hydroxyheptansäure, 8-Hydroxyoctansäure, 10-Hydroxydecansäure, 12-Hydroxydodecansäure, 14-Hydroxytetradecansäure, 16-Hydroxyhexadecansäure und 18-Hydroxyoctadecansäure, wobei 10-Hydroxydecansäure erfindungsgemäß besonders bevorzugt ist.

Lineare Dicarbonsäuren sind erfindungsgemäß bevorzugt ausgewählt aus Malonsäure, Bernsteinsäure, Glutarsäure, Adipinsäure, Pimelinsäure, Nonandisäure (Azelainsäure), Decandisäure (Sebacinsäure) und 8-Hexadecen-1,16-dicarbonsäure, wobei Azelainsäure, Decandisäure (Sebacinsäure) und 8-Hexadecen-1,16-dicarbonsäure erfindungsgemäß besonders bevorzugt sind.

Die Ester der genannten Säuren sind erfindungsgemäß bevorzugt ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern.

Erfindungsgemäß bevorzugte Salze der vorgenannten Säuren sind ausgewählt aus den physiologisch verträglichen Salzen, besonders bevorzugt aus den Ammonium-, Alkalimetall-, Magnesium-, Calcium-, Aluminium-, Zink-, Kupfer- und Mangan-Salzen. Erfindungsgemäß außerordentlich bevorzugt sind die Natrium-, Kalium-, Magnesium-, Aluminium-, Zink- und Mangan-Salze.

Erfindungsgemäß besonders bevorzugte Benefit-Zusammensetzungen sind dadurch gekennzeichnet, dass sie mindestens eine alpha-Hydroxycarbonsäure, alpha-Ketocarbonsäure, beta-Hydroxycarbonsäure, omega-Hydroxycarbonsäure und/oder lineare Dicarbonsäure und/oder einen Ester, ein Lacton, ein Amid und/oder ein Salz hiervon in einer Gesamtmenge von 0,0001 - 10 Gew.-%, bevorzugt 0,1 - 5 Gew.-%, besonders bevorzugt 0,5 - 1 - 2 Gew.-%, jeweils bezogen auf die gesamte Benefit-Zusammensetzung, enthalten.

Weitere erfindungsgemäß bevorzugte Benefit-Zusammensetzungen sind dadurch gekennzeichnet, dass sie mindestens einen Pflanzenextrakt enthalten.

Erfindungsgemäß bevorzugte Pflanzenextrakte werden üblicherweise durch Extraktion der gesamten Pflanze, in einzelnen Fällen aber auch ausschließlich aus Blüten und/oder Blättern und/ oder Samen und/oder anderen Pflanzenteilen, hergestellt. Erfindungsgemäß besonders bevorzugt sind die Extrakte aus dem Meristem, also dem teilungsfähigen Bildungsgewebe der Pflanzen, und speziellen Pflanzen wie Grünem Tee, Hamamelis, Kamille, Ringelblume, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch (Althaea), Malve (Malva sylvestris), Veilchen, Blättern der Schwarzen Johannisbeere, Huflattich, Fünffingerkraut, Ginseng, Ingwerwurzel und Süßkartoffel. Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden.

Die Extrakte können durch Auspressen der Pflanzen oder einzelner Pflanzenteile gewonnen werden. Weiterhin können sie mit Hilfe von Extraktionsmitteln gewonnen werden. Als Extraktionsmittel zur Herstellung der genannten Pflanzenextrakte können u. a. Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglycol, Propylenglycol und Butylenglycol und zwar sowohl als alleiniges Extraktionsmittel als auch in Mischung mit Wasser, bevorzugt. Pflanzenextrakte auf Basis von Wasser/Propylenglycol im Verhältnis 1:10 bis 10:1 haben sich als besonders geeignet erwiesen. Die Wasserdampfdestillation fällt erfindungsgemäß unter die bevorzugten Extraktionsverfahren.

Die Pflanzenextrakte können erfindungsgemäß sowohl in reiner, getrockneter als auch in verdünnter oder gelöster Form eingesetzt werden. Sofern sie in verdünnter oder gelöster Form eingesetzt werden, enthalten sie üblicherweise ca. 2 - 80 Gew.-% Aktivsubstanz und ein wasserfreies Lösemittel, beispielsweise wasserfreie Alkohole und/oder Glycole oder, besonders bevorzugt, mindestens ein Öl. Je nach Wahl des Lösemittels kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglycolcapryl-/caprinsäureglyceride, Polyoxyethylenglycerinmonolaurat und Polyoxyethylenglycolkokosfettsäureglyceride.

Weiterhin kann es bevorzugt sein, in den erfindungsgemäßen Benefit-Zusammensetzungen Mischungen aus mehreren, insbesondere aus zwei, drei oder vier verschiedenen Pflanzenextrakten einzusetzen. Besonders bevorzugt ist der Einsatz getrockneter, pulverförmiger Pflanzenextrakte.

Weitere erfindungsgemäß bevorzugte Benefit-Zusammensetzungen sind dadurch gekennzeichnet, dass sie mindestens ein Monomer, Oligomer oder Polymer von Aminosäuren, N-C₂-C₂₄-Acylaminosäuren, den Estern und/oder den physiologisch verträglichen Metallsalzen dieser Substanzen, enthalten.

Die Monomere der Aminosäuren und/oder der N-C₂-C₂₄-Acylaminosäuren sind erfindungsgemäß bevorzugt ausgewählt aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, N-Acetyl-L-cystein, Zink- und Natriumpyroglutamat und Natriumlauroylglutamat.

Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere können als Wirkstoffe gegen die Hautalterung verwendet werden.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCl-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arghexyldecylester (z. B. Calmosensine von Sederma), Carnosin (ß-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acvlierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).

Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.

Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist.

Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.

Proteinhydrolysate können naturgemäß auch monomere Aminosäuren und Oligopeptide enthalten; ihre Zusammensetzung ist normalerweise nicht definiert.

Ebenfalls bevorzugt ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß bevorzugt sind auch kationisierte Proteinhydrolysate. Besondes bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.

Erfindungsgemäß bevorzugte DNA-Reparaturenzyme sind Photolyase und T4 Endonuclease V, letztere im Weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A*. *nidulans* wird in technisch relevanten Mengen mittlerweile aus E. coli gewonnen. Photolyase ist zur Aktivierung auf Licht angewiesen.

Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.

Erfindungsgemäß besonders bevorzugte Benefit-Zusammensetzungen sind dadurch gekennzeichnet, dass sie mindestens eines der Handelsprodukte Photosomes™ oder Ultrasomes™ in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Benefit-Zusammensetzung, enthalten.

Erfindungsgemäß besonders bevorzugte Benefit-Zusammensetzungen sind dadurch gekennzeichnet, dass sie mindestens ein Monomer, Oligomer oder Polymer von Aminosäuren, N-C₂-C₂₄-Acylaminosäuren und/oder den Estern und/ oder den physiologisch verträglichen Metallsalzen dieser Substanzen in Gesamtmengen von 0,0000001 - 10 Gew.-%, bevorzugt 0,001 - 5 Gew.-% und besonders bevorzugt 0,01 - 1 - 2 - 3 Gew.-%, jeweils bezogen auf den Aktivsubstanzgehalt in der gesamten erfindungsgemäßen Benefit-Zusammensetzung, enthalten.

In einer weiteren bevorzugten Ausführungsform enthalten die erfindungsgemäßen Benefit-Zusammensetzungen mindestens ein DNA-Oligonucleotid oder mindestens ein RNA-Oligonucleotid.

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).

Ein erfindungsgemäß besonders bevorzugtes Oligonucleotid ist das Thymidin-Dinucleotid.

Erfindungsgemäß besonders bevorzugte Benefit-Zusammensetzungen sind dadurch gekennzeichnet, dass sie mindestens ein DNA-Oligonucleotid und/oder ein RNA-Oligonucleotid in Gesamtmengen von 0,000001 - 5 Gew.-%, bevorzugt 0,0001 - 0,5 Gew.-% und besonders bevorzugt 0,001 - 0,05 Gew.-%, bezogen auf die gesamte Benefit-Zusammensetzung, enthalten.

In einer weiteren bevorzugten Ausführungsform enthalten die erfindungsgemäßen Benefit-Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins). Erfindungsgemäß besonders bevorzugte Benefit-Zusammensetzungen sind dadurch gekennzeichnet, dass sie mindestens eine natürliche Betainverbindung in einer Gesamtmenge von 0,05 - 5 Gew.-%, bevorzugt 0,1 bis 3 Gew.-%, besonders bevorzugt 0,5 bis 2 Gew.-%, jeweils bezogen auf die gesamte Benefit-Zusammensetzung, enthalten.

In einer weiteren bevorzugten Ausführungsform enthalten die erfindungsgemäßen Benefit-Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Benefit-Zusammensetzungen sind dadurch gekennzeichnet, dass sie mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Benefit-Zusammensetzung, enthalten.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Benefit-Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Benefit-Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfndungsgemäßen Benefit-Zusammensetzungen bevorzugt in Mengen von 0,0005 bis 0,1 - 1 Gew.-%, bezogen auf die gesamte erfindungsgemäße Benefit-Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt

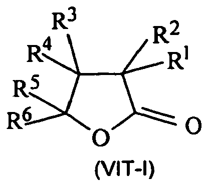

Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₁-C₄ - Kohlenwasserstoffrest oder einen gesättigten oder ein- oder zweifach ungesättigten, verzweigten oder linearen Mono-, Di- oder Tri- amino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure. Erfindungsgemäß besonders bevorzugte Benefit-Zusammensetzungen sind dadurch gekennzeichnet, dass sie mindestens eine der genannten Verbindungen des Vitamin B₅-Typs sowie der 2-Furanon-derivate in einer Gesamtmenge von 0,05 - 5 Gew.-%, bevorzugt 0,1 - 3 Gew.-%, besonders bevorzugt 0,5 bis 2 Gew.-%, jeweils bezogen auf die gesamte Benefit-Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Benefit-Zusammensetzungen sind dadurch gekennzeichnet, dass sie mindestens eine Vitamin B₆-Komponente in einer Gesamtmenge von 0,0001 - 1,0 Gew.-%, insbesondere in Mengen von 0,001 - 0,01 Gew.-%, bezogen auf die Benefit-Zusammensetzung, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Benefit-Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, bezogen auf die Benefit-Zusammensetzung, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammeibegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Benefit-Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Benefit-Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Benefit-Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Benefit-Zusammensetzung, enthalten.

In einer weiteren bevorzugten Ausführungsform enthalten die erfindungsgemäßen Benefit-Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und/oder Salzen und aus Pantolacton. Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und
- sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Benefit-Zusammensetzungen sind dadurch gekennzeichnet, dass sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Benefit-Zusammensetzung, enthalten. Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat,
- oleat, Tocophereth-5, Tocophereth-10, Tocophereth-12, Tocophereth-18, Tocophereth-50 und Tocophersolan. Erfindungsgemäß besonders bevorzugte Benefit-Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Benefit-Zusammensetzung, enthalten.

Vitamin H ist eine andere Bezeichnung für Biotin oder Vitamin B₇ (siehe oben).

Zu den fettlöslichen Vitaminen der Vitamin K-Gruppe, denen das Grundgerüst des 2-Methyl-1.4-naphthochinons zugrunde liegt, gehören Phyllochinon (Vitamin K₁), Farnochinon oder Menachinon-7 (Vitamin K2) und Menadion (Vitamin K₃). Erfindungsgemäß besonders bevorzugte Benefit-Zusammensetzungen sind dadurch gekennzeichnet, dass sie mindestens ein Vitamin K in einer Gesamtmenge von 0001 bis 1,0 Gew.-%, bevorzugt 0,05 bis 0,01 Gew.-%, besonders bevorzugt 0,1 bis 0,5 Gew.-%, jeweils bezogen auf die gesamte Benefit-Zusammensetzung, enthalten.

Vitamin A-palmitat (Retinylpalmitat), Pantolacton, Nicotinsäureamid, Pyridoxin, Pyridoxamin, Pyridoxal, Biotin, Folsäure, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Benefit-Zusammensetzungen mindestens ein Flavonoid und/oder mindestens einen Flavonoid-reichen Pflanzenextrakt.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß besonders bevorzugte Benefit-Zusammensetzungen sind dadurch gekennzeichnet, dass sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten Benefit-Zusammensetzung, enthalten.

In einer weiteren bevorzugten Ausführungsform enthalten die erfindungsgemäßen Benefit-Zusammensetzungen mindestens ein Isoflavonoid und/oder mindestens einen Isoflavonoid-reichen Pflanzenextrakt. Zu den Isoflavonoiden werden an dieser Stelle die Isoflavone und die Isoflavon-Glycoside gezählt.

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Benefit-Zusammensetzungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse. Erfindungsgemäß besonders bevorzugte Benefit-Zusammensetzungen sind dadurch gekennzeichnet, dass sie mindestens ein Isoflavonoid in einer Gesamtmenge von 0,00001 - 1 Gew.-%, bevorzugt 0,0005 - 0,5 Gew.-% und besonders bevorzugt 0,001 - 0,1 Gew.-%, jeweils bezogen auf die Isoflavonoidaktivsubstanz in der gesamten Benefit-Zusammensetzung, enthalten.

In einer weiteren bevorzugten Ausführungsform enthalten die erfindungsgemäßen Benefit-Zusammensetzungen mindestens ein Polyphenol oder einen Polyphenol-reichen Pflanzenextrakt.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.

Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Benefit-Zusammensetzung, eingesetzt.

In einer weiteren bevorzugten Ausführungsform enthalten die erfindungsgemäßen Benefit-Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 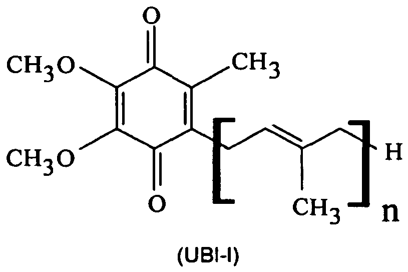 mit n = 6, 7, 8, 9 oder 10.

Besonders bevorzugt ist das Ubichinon der Formel (UBI-I) mit n = 10, auch bekannt als Coenzym Q10.

Erfindungsgemäß besonders bevorzugte Benefit-Zusammensetzungen sind dadurch gekennzeichnet, dass sie mindestens ein Ubichinon, Ubichinol oder ein Derivat hiervon in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Benefit-Zusammensetzung, enthalten.

In einer weiteren bevorzugten Ausführungsform enthalten die erfindungsgemäßen Benefit-Zusammensetzungen Silymarin. Silymarin stellt erfingdungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß besonders bevorzugte Benefit-Zusammensetzungen sind dadurch gekennzeichnet, dass sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Benefit-Zusammensetzung, enthalten.

In einer weiteren bevorzugten Ausführungsform enthalten die erfindungsgemäßen Benefit-Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Benefit-Zusammensetzung, enthalten.

In einer weiteren bevorzugten Ausführungsform enthalten die erfindungsgemäßen Benefit-Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Benefit-Zusammensetzung, enthalten.

In einer weiteren bevorzugten Ausführungsform enthalten die erfindungsgemäßen Benefit-Zusammensetzungen Kreatin. Kreatin ist der Trivialname für N-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Benefit-Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Erfindungsgemäß besonders bevorzugte Benefit-Zusammensetzungen sind dadurch gekennzeichnet, dass sie mindestens einen Olivenblattextrakt in einer Gesamtmenge von 0,01 bis 5 Gew.-%, bevorzugt 0,1 bis 3 Gew.-% und besonders bevorzugt 0,5 bis 1 - 2 Gew.-%, jeweils bezogen auf die Benefit-Zusammensetzung, enthalten.

Olivenblattextrakte können einen hohen Gehalt an Oleanolsäure und/oder Oleanol aufweisen. In einer weiteren bevorzugten Ausführungsform enthalten die erfindungsgemäßen Benefit-Zusammensetzungen Oleanolsäure und/oder Oleanol. Erfindungsgemäß besonders bevorzugte Benefit-Zusammensetzungen sind dadurch gekennzeichnet, dass sie Oleanolsäure und/oder Oleanol in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Benefit-Zusammensetzung, enthalten.

In einer weiteren bevorzugten Ausführungsform enthalten die erfindungsgemäßen Benefit-Zusammensetzungen Ursolsäure. Erfindungsgemäß besonders bevorzugte Benefit-Zusammensetzungen sind dadurch gekennzeichnet, dass sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Benefit-Zusammensetzung, enthalten.

In einer weiteren bevorzugten Ausführungsform enthalten die erfindungsgemäßen Benefit-Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte Benefit-Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Benefit-Zusammensetzung.

In einer weiteren bevorzugten Ausführungsform enthalten die erfindungsgemäßen Benefit-Zusammensetzungen mindestens ein Derivat von methyliertem Silanol, vorzugsweise mindestens einen Ester von methyliertem Silanol. Bevorzugte Derivate von methyliertem Silanol sind ausgewählt aus:
- sodium mannuronate methylsilanol (Algisium, Exsymol)
- methylsilanol mannuronate (Algisium C®, Exsymol)
- methylsilanol mannuronate Nylon-12 (Algisium C powder®, Exsymol)
- ascorbylmethylsilanol (Ascorbosilane concentrate C®. Exsymol)
- ascorbylmethylsilanol pectinate (Ascorbosilane C®, Exsymol)
- dimethyl oxobenzodioxasilane (Pro-DSB® 10/palmitate octyle, Exsymol)
- dimethyl oxobenzodioxasilane Nylon-12 (DSBC powder®, Exsymol)
- sodium hyaluronate dimethylsilanol (DSH®, Exsymol)
- dimethylsilanol hyaluronate (DSHC®, Exsymol)
- methysilanol glycyrrhizinate (Glysinol®, Exsymol)
- methylsilanolhydroxyproline (Hydroxyprolisilane®, Exsymol)
- methylsilanolhydroxyproline aspartate (Hydroxyprolisilane C®, Exsymol)
- sodium lactate methylsilanol (Lasilium®, Exsymol)
- lactoylmethylsilanol elastinate (Lasilium C®, Exsymol)
- dioleyl tocopheryl methylsilanol (Liposiliol C®, Exsymol)
- methylsilanol acetylmethionate (Methiosilane®, Exsymol)
- acetylmethionylmethylsifanol elastinate (Methiosilane C®, Exsymol)
- methylsilanol PEG 7 glyceryl cocoate (Monosiliol®, Exsymol)
- methylsilanol tri PEG 7 glyceryl cocoate (Monosiliol C®, Exsymol)
- methylsilanol elastinate (Proteosilane C®, Exsymol)
- pyrollidone carboxylate caustic methylsilanol (Silhydrate®, Exsymol)
- pyrollidone carboxylate copper methylsilanol (Silhydrate C®, Exsymol)
- methylsilanolcarboxymethyl theophylline (Theophyllisilane®, Exsymol)
- methylsilancarboxymethyl theophylline alginate (Theophyllisilane C® Exsymol)
- methylsilanol acetyltyrosine (Tyrosilane®, Exsymol)
- copper acetyl tyrosinate methylsilanol (Tyrosilane C®, Exsymol).

Besonders bevorzugt sind Sodium Hyaluronate Dimethylsilanol, Dimethylsilanol Hyaluronate, Methylsilanol Mannuronate, Methylsilanol Hydroxyproline und Methylsilanol Hydroxyproline Aspartate. In einer weiteren bevorzugten Ausführungsform enthalten die erfindungsgemäßen Benefit-Zusammensetzungen mindestens ein Derivat von methyliertem Silanol in Gesamtmengen von 0,001 - 5 Gew.-%, bevorzugt 0,005 - 1 Gew.-% und besonders bevorzugt 0,01 - 0,5 Gew.-%, jeweils bezogen auf die Aktivsubstanz in der gesamten erfindungsgemäßen Benefit-Zusammensetzung.

In einer weiteren bevorzugten Ausführungsform enthalten die erfindungsgemäßen Benefit-Zusammensetzungen Phytinsäure. Erfindungsgemäß besonders bevorzugte kosmetische oder dermatologische Zusammensetzungen sind dadurch gekennzeichnet, dass sie Phytinsäure in einer Gesamtmenge von 0,001 - 1 Gew.-%, bevorzugt 0,01 - 0,5 Gew.-% und besonders bevorzugt 0,05 - 0,1 Gew.-% enthalten, jeweils bezogen auf die gesamte Benefit-Zusammensetzung.

In einer weiteren bevorzugten Ausführungsform enthalten die erfindungsgemäßen Benefit-Zusammensetzungen mindestens einen Wirkstoff, der die beta-Endorphinsynthese in Keratinozyten stimuliert.

Erfindungsgemäß besonders bevorzugte Stimulatoren der beta-Endorphin-Synthese sind ausgewählt aus Mischungen aus mindestens einem Extrakt aus den Blättern der Mentha piperita und mindestens einem Extrakt aus Kakaobohnen, wobei wässrige, glycolische oder wässrig-glycolische Zubereitungen dieser Extraktmischungen, die unter den Handelsbezeichnungen Caomint, Caophenol, Caobromine, Caospice und Caoorange von der Firma Solabia erhältlich sind, besonders bevorzugt sind. Ein weiterer besonders bevorzugter Stimulator der beta-Endorphin-Synthese ist das Dipeptidderivat N-Acetyl-Tyr-Arg-hexyldecylester mit der INCI-Bezeichnung Acetyl Dipeptide-1 Cetyl Ester, das z. B. als wässrige Zubereitung unter der Handelsbezeichnung Calmosensine von der Firma Sederma erhältlich ist.

Weitere bevorzugte Stimulatoren der beta-Endorphin-Synthese sind Extrakte aus Helichrysum italicum, z.B. erhältlich unter der Handelsbezeichnung Areaumat Perpetua von der Firma Codif, Extrakte aus Crithmum Maritimum, z. B. erhältlich unter den Handelsbezeichnungen Areaumat Samphira und Aroleat Samphira von der Firma Codif, Extrakte aus Lavendula stoechas, z. B. erhältlich unter der Handelsbezeichnung Areaumat Lavanda von der Firma Codif, Extrakte aus Mentha piperita, wie sie z. B. unter den Handelsbezeichnungen Authenticals of Peppermint (Solabia) und Calmiskin (Silab) erhältlich sind, Glutamylamidoethyl Indole, z. B. erhältlich unter der Handelsbezeichnung Glistin von der Firma Exsymol, ein durch mikrobielle Fermentation gewonnenes verzweigtes Polysaccharid mit Rhamnose-, Galactose- und Glucuronsäure-Einheiten mit der INCl-Bezeichnung Biosaccharide Gum-2, z. B. erhältlich unter der Handelsbezeichnung Rhamnosoft von der Firma Solabia, Extrakte aus den Samen von Tephrosia Purpurea mit der INCI-Bezeichnung Tephrosia Purpurea Seed Extract, z. B. erhältlich unter der Handelsbezeichnung Tephroline von der Firma Vincience, Mischungen aus dem Öl von Mentha arvensis-Blättern, Limonenschalenöl, Zypressenöl, Lavendelöl und Cistus Ladaniferus-Öl mit der INCI-Bezeichnung Mentha Arvensis Leaf Oil and Citrus Medica Limonum (Lemon) Peel Oil and Cupressus Sempervirens Oil and Lavandula Hybrida Oil and Cistus Ladaniferus Oil, z. B. erhältlich unter der Handelsbezeichnung V-Tonic (Gattefossé), und Hexasaccharide gemäß FR 2842201 sowie beliebige Mischungen dieser Wirkstoffe. Erfindungsgemäß besonders bevorzugte Benefit-Zusammensetzungen sind dadurch gekennzeichnet, dass sie mindestens einen Wirkstoff zur Stimulation der beta-Endorphin-Synthese in Gesamtmengen von 0,01 - 10 Gew.-%, bevorzugt 0,1 - 5 Gew.-% und besonders bevorzugt 1 - 3 Gew.-%, jeweils bezogen auf das Handelsprodukt, das den Wirkstoff enthält, in der gesamten erfindungsgemäßen Benefit-Zusammensetzung, enthalten. Erfindungsgemäß besonders bevorzugte Benefit-Zusammensetzungen sind dadurch gekennzeichnet, dass sie mindestens einen Wirkstoff zur Stimulation der beta-Endorphin-Synthese in Gesamtmengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten erfindungsgemäßen Benefit-Zusammensetzung, enthalten.

In einer weiteren bevorzugten Ausführungsform enthalten die erfindungsgemäßen Benefit-Zusammensetzungen mindestens einen selbstbräunenden Wirkstoff. Erfindungsgemäß bevorzugte selbstbräunende Wirkstoffe sind ausgewählt aus Dihydroxyaceton, Tyrosin, Tyrosinderivaten und Erythrulose. Erfindungsgemäß besonders bevorzugte Benefit-Zusammensetzungen sind dadurch gekennzeichnet, dass sie mindestens einen selbstbräunenden Wirkstoff in einer Gesamtmenge von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Benefit-Zusammensetzung, enthalten.

In einer weiteren bevorzugten Ausführungsform enthalten die erfindungsgemäßen Benefit-Zusammensetzungen mindestens einen hautaufhellenden Wirkstoff. Erfindungsgemäß bevorzugte hautaufhellende Wirkstoffe sind ausgewählt aus Ascorbinsäure, den Estern der Ascorbinsäure mit Phosphorsäure und/oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.

Erfindungsgemäß besonders bevorzugte Benefit-Zusammensetzungen sind dadurch gekennzeichnet, dass sie mindestens einen hautaufhellenden Wirkstoff in einer Gesamtmenge von 0,05 bis 5 Gew.-%, bevorzugt von 0,1 - 2 Gew-%, jeweils bezogen auf die gesamte Benefit-Zusammensetzung, enthalten.

In einer weiteren bevorzugten Ausführungsform enthalten die erfindungsgemäßen Benefit-Zusammensetzungen mindestens einen Wirkstoff, der die Prostaglandinsynthese und/oder die Leukotrien-Synthese inhibiert.

Bevorzugte Wirkstoffe, die die Prostaglandinsynthese inhibieren, sind ausgewählt aus Wirkstoffen, die das Enzym Cyclooxygenase inhibieren und Wirkstoffen, die die Ausschüttung von Interleukinen, insbesondere von Interleukin-1-alpha, inhibieren. Im Sinne der vorliegenden Erfindung kann unter der Inhibierung der Cyclooxygenase sowohl eine Senkung der Menge dieses Enzyms als auch eine Minderung seiner Aktivität sowie beides hiervon verstanden werden.

Bevorzugte Wirkstoffe, die die Leukotrien-Synthese inhibieren, sind ausgewählt aus Wirkstoffen, die das Enzym 5-Lipoxygenase inhibieren. Im Sinne der vorliegenden Erfindung kann unter der Inhibierung der 5-Lipoxygenase sowohl eine Senkung der Menge dieses Enzyms als auch eine Minderung seiner Aktivität sowie beides hiervon verstanden werden.

Erfindungsgemäß bevorzugte Inhibitoren der Prostaglandin-Synthese, insbesondere Inhibitoren der Cyclooxygenase und/oder der Interleukin-Ausschüttung, sind ausgewählt aus Silymarin, das besonders bevorzugt in liposomenverkapselter Form eingesetzt wird (erhältlich z. B. unter der Handelsbezeichnung Silymarin Phytosome (INCI: Silybum Marianum Extract and Phospholipids) von der Firma Indena SpA. Silymarin stellt ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Weitere erfindungsgemäß bevorzugte Inhibitoren der Prostagladin-Synthese, insbesondere Inhibitoren der Cyclooxygenase und/oder der Interleukin-Ausschüttung, sind ausgewählt aus Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter der Handelsbezeichnung Calmsphere von Soliance erhältlich ist, Mischungen aus Getreidewachsen, Extrakten aus Schibutter und Argania Spinosa-Öl mit der INCI-Bezeichnung "Spent grain wax and butyrospermum parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Extrakten aus Olivenblättern (INCI: Olea Europaea (Olive) Leaf Extract), wie sie insbesondere unter der Handelsbezeichnung Oleanoline DPG von der Firma Vincience erhältlich sind, weiterhin Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter der Handelsbezeichnung Rooibos Herbasec MPE von der Firma Cosmetochem erhältlich sind, den physiologisch verträglichen Salzen von Sterolsulfaten, wie sie z. B. unter der Handelsbezeichnung Phytocohesine (INCl: Sodium Beta-Sitosterylsulfate) von der Firma Vincience erhältlich sind, sowie beliebigen Mischungen dieser Wirkstoffe.

Erfindungsgemäß besonders bevorzugte Benefit-Zusammensetzungen sind dadurch gekennzeichnet, dass sie mindestens einen Inhibitor der Prostaglandin-Synthese in einer Gesamtmenge von 0,0001 - 10,0 Gew.-%, bevorzugt 0,001 - 2,0 Gew.-%, besonders bevorzugt 0,05 - 1,0 Gew.-% und außerordentlich bevorzugt 0,1 - 0,5 Gew.-%, jeweils bezogen auf die gesamte Benefit-Zusammensetzung, enthalten. Erfindungsgemäß bevorzugte Inhibitoren der Leukotrien-Synthese, insbesondere Inhibitoren der 5-Lipoxygenase, sind ausgewählt aus Alginhydrolysaten, Aminodicarbonsäuren mit einer C-Kettenlänge von 3 - 6 Kohlenstoffatomen sowie deren physiologisch verträglichen Salzen, N-alkylierten C₂-C₁₁-Aminosäuren mit C₁-C₂₂-Alkylresten sowie deren physiologisch verträglichen Salzen, N-acylierten C₂-C₁₁-Aminosäuren mit C₂-C₂₂-Acylresten sowie deren physiologisch verträglichen Salzen, Hefeextrakten, α-Bisabolol, α-Liponsäure, Allantoin sowie beliebigen Mischungen dieser Wirkstoffe.

In einer bevorzugten Ausführungsform sind erfindungsgemäß besonders bevorzugte Alginhydrolysate ausgewählt aus den Produkten, die z. B. unter der Handelsbezeichnung Phycosaccharide, insbesondere Phycosaccharide Al, von der Firma Codif erhältlich sind.

In einer weiteren bevorzugten Ausführungsform sind die erfindungsgemäß bevorzugten Aminodicarbonsäuren mit einer C-Kettenlänge von 3 - 6 Kohlenstoffatomen ausgewählt aus Aminomalonsäure, Aminobernsteinsäure (= Asparaginsäure), Aminoglutarsäure und Aminoadipinsäure sowie deren physiologisch verträglichen Salzen. Besonders bevorzugt sind Asparaginsäure und ihre physiologisch verträglichen Salze, insbesondere Kaliumaspartat und Magnesiumaspartat.

Erfindungsgemäß bevorzugt werden die Aminodicarbonsäuren mit einer C-Kettenlänge von 3 - 6 Kohlenstoffatomen sowie deren Salze in Mengen von 0,01 bis 5 Gew.-%, bevorzugt 0,1 bis 2 Gew.-% und besonders bevorzugt 0,5 bis 1 Gew.-%, jeweils bezogen auf die gesamte erfindungsgemäße Benefit-Zusammensetzung, eingesetzt.

In einer weiteren bevorzugten Ausführungsform sind die erfindungsgemäß bevorzugten N-alkylierten C₂-C₁₁-Aminosäuren mit einem C₁-C₂₂-Alkylrest ausgewählt aus Alanin, Glutaminsäure, Pyroglutaminsäure, Lysin, Arginin, Histidin, Valin, Leucin, Isoleucin, Prolin, Tryptophan, Phenylalanin, Methionin, Glycin, Serin, Tyrosin, Threonin, Cystein, Asparagin und Glutamin sowie deren physiologisch verträglichen Salzen, die am Stickstoffatom der Aminogruppe einen C₁-C₂₂-Alkylrest, ausgewählt aus einer Gruppe Methyl, Ethyl, Propyl, Butyl, Pentyl, Hexyl, Heptyl, Octyl, Nonyl, Decyl, Undecyl, Dodecyl (Lauryl), Tridecyl, Tetradecyl (Myristyl), Pentadecyl, Hexadecyl (Palmityl, Cetyl), Heptadecyl, Octadecyl (Stearyl), Nonadecyl, Eicosanyl (Arachidyl) und Behenyl, aufweisen. Besonders bevorzugt ist N-Methylglycin (= Sarcosin).

Erfindungsgemäß bevorzugt werden die N-alkylierten C₂-C₁₁-Aminosäuren mit einem C₁-C₂₂-Alkylrest sowie deren physiologisch verträglichen Salze in Mengen von 0,01 - 10 Gew.-%, bevorzugt 0,1 - 5 Gew.-% und besonders bevorzugt 0,5 bis 2 Gew.-%, jeweils bezogen auf die gesamte erfindungsgemäße Benefit-Zusammensetzung, eingesetzt.

In einer weiteren bevorzugten Ausführungsform sind die erfindungsgemäß bevorzugten N-acylierten C₂-C₁₁-Aminosäuren mit einem C₂-C₂₂-Acylrest ausgewählt aus Glutaminsäure, Pyroglutaminsäure, Lysin, Arginin, Histidin, Valin, Leucin, Isoleucin, Prolin, Tryptophan, Phenylalanin, Methionin, Glycin, Serin, Tyrosin, Threonin, Cystein, Asparagin und Glutamin sowie deren physiologisch verträglichen Salzen. Die Aminosäuren können einzeln oder im Gemisch eingesetzt werden. Erfindungsgemäß geeignet sind insbesondere Aminosäuren-Gemische, die aus Pflanzen, insbesondere Getreidepflanzen, gewonnen wurden. Der C₂ - C₂₂-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Besonders bevorzugt sind Natriumcocoylaminosäuren, Natriumoctanoylglutamat, Natriumdecanoylglutamat, Natriumlauroylglutamat, Natriummyristoylglutamat, Natriumcetoylglutamat und Natriumstearoylglutamat und die Lauroyl-Derivate von aus Getreidepflanzen gewonnenen Aminosäuren.

Die Getreidepflanzen, aus denen die erfindungsgemäß bevorzugten Aminosäuren gewonnen werden, unterliegen keiner Einschränkung. Geeignet sind beispielsweise Hafer, Weizen, Gerste und Roggen; besonders geeignet ist Hafer.

Ein besonders bevorzugter 5-Lipoxygenase-Inhibitor ist das Handelsprodukt Seppicalm von der Firma Seppic mit der INCI-Bezeichnung "Sodium Cocoyl Aminoacids, Sarcosine, Potassium Aspartate, Magnesium Aspartate".

In einer weiteren bevorzugten Ausführungsform werden die erfindungsgemäß als 5-Lipoxygenase-Inhibitoren bevorzugten Hefeextrakte in Mengen von 0,001 bis 5 Gew.-%, bevorzugt 0,01 bis 2 Gew.-% und besonders bevorzugt 0,1 bis 1 Gew.-%, jeweils bezogen auf den Extrakt tel quel in der gesamten erfindungsgemäßen Benefit-Zusammensetzung, eingesetzt. Ein besonders bevorzugt verwendetes Handelsprodukt ist Drieline (INCI-Bezeichnung "Sorbitol, Yeast Extract"), erhältlich von der Firma Lanatech.

In einer weiteren bevorzugten Ausführungsform wird der erfindungsgemäß bevorzugte 5-Lipoxygenase-Inhibitor α-Bisabolol in Mengen von 0,001 bis 5 Gew.-%, bevorzugt 0,01 bis 2 Gew.-% und besonders bevorzugt 0,1 bis 1 Gew.-%, jeweils bezogen auf die gesamte Benefit-Zusammensetzung, eingesetzt.

In einer weiteren bevorzugten Ausführungsform wird der erfindungsgemäß bevorzugte 5-Lipoxygenase-Inhibitor α-Liponsäure in Mengen von 0,001 bis 5 Gew.-%, bevorzugt 0,01 bis 2 Gew.-% und besonders bevorzugt 0,1 bis 1 Gew.-%, jeweils bezogen auf die gesamte Benefit-Zusammensetzung, eingesetzt.

In einer weiteren bevorzugten Ausführungsform wird der erfindungsgemäß bevorzugte 5-Lipoxygenase-Inhibitor Allantoin in Mengen von 0,001 bis 5 Gew.-%, bevorzugt 0,01 bis 2 Gew.-% und besonders bevorzugt 0,1 bis 1 Gew.-%, jeweils bezogen auf die gesamte Benefit-Zusammensetzung, eingesetzt.

In einer weiteren bevorzugten Ausführungsform sind die erfindungsgemäß als 5-Lipoxygenase-Inhibitoren bevorzugten physiologisch verträglichen Salze der Sterolsulfate ausgewählt aus den Salzen von β-Sitosterolsulfat, Ergosterolsulfat, Stigmasterolsulfat, Cholesterolsulfat und Lanosterolsulfat. Besonders bevorzugt sind die Salze von β-Sitosterolsulfat. Die Sterolsulfatsalze werden in Mengen von 0,001 bis 5 Gew.-%, bevorzugt 0,01 bis 2 Gew.-% und besonders bevorzugt 0,1 bis 1 Gew.-%, jeweils bezogen auf die gesamte Benefit-Zusammensetzung, eingesetzt. Die Sterolsulfatsalze können dabei sowohl einzeln als auch in beliebigen Mischungen eingesetzt werden. Ein besonders bevorzugt eingesetztes Handelsprodukt ist Phytocohesine (INCl-Bezeichnung "Sodium Beta-Sitosteryl Sulfate"), erhältlich von der Firma Vincience.

Die physiologisch verträglichen Salze der vorgenannten 5-Lipoxygenase-Inhibitoren sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Erfindungsgemäß besonders bevorzugte kosmetische oder dermatologische Benefit-Zusammensetzungen sind dadurch gekennzeichnet, dass sie mindestens einen Inhibitor der Leukotrien-Synthese in einer Gesamtmenge von 0,0001 - 10,0 Gew.-%, bevorzugt 0,001 - 2,0 Gew.-%, besonders bevorzugt 0,05 - 1,0 Gew.-% und außerordentlich bevorzugt 0,1 - 0,5 Gew.-%, jeweils bezogen auf die gesamte Benefit-Zusammensetzung, enthalten.

In einer weiteren bevorzugten Ausführungsform enthalten die erfindungsgemäßen Benefit-Zusammensetzungen mindestens einen sebumregulierenden Wirkstoff. Erfindungsgemäß bevorzugte sebumregulierende Wirkstoffe sind ausgewählt aus 10-Hydroxydecansäure, Sebacinsäure, Azelainsäure und den Estern der Azelainsäure, insbesondere Kaliumazeloyldiglycinat, 1,10-Decandiol und mindestens einem Extrakt aus Spiraea Ulmaria sowie Mischungen der vorgenannten Substanzen. Bevorzugte Mischungen sind beispielsweise erhältlich als Handelsprodukt Acnacidol PG (Propylene Glycol, 10-Hydroxydecanoic acid, Sebacic acid, 1,10-Decandiol) von Vincience erhältlich. Ein bevorzugter Extrakt aus Spiraea Ulmaria ist z. B. im Produkt Seboregul 2 der Firma Silab enthalten. Kaliumazeloyldiglycinat ist z. B. in dem Produkt Azeloglicina der Firma Sinerga enthalten. Erfindungsgemäß besonders bevorzugte Benefit-Zusammensetzungen sind dadurch gekennzeichnet, dass sie mindestens einen sebumregulierenden Wirkstoff in Gesamtmengen von 0,00001 bis 10 Gew.-%, bevorzugt 0,01 bis 5 Gew.-% und besonders bevorzugt 0,1 bis 1 bis 2 Gew.-%, jeweils bezogen auf Aktivsubstanz in der gesamten erfindungsgemäßen Benefit-Zusammensetzung, enthalten.

In einer weiteren bevorzugten Ausführungsform enthalten die erfindungsgemäßen Benefit-Zusammensetzungen mindestens eine den Haarwuchs inhibierende Substanz. Geeignete Substanzen, die den Haarwuchs inhibieren, sind insbesondere ausgewählt aus Eflornithin, Wirkstoffkombinationen aus Sojaproteinhydrolysat, Harnstoff, Menthol, Salicylsäure und Extrakten aus Hypericum Perforatum, Hamamelis Virginiana, Amica Montana und der Rinde von Salix Alba, wie sie beispielsweise und bevorzugt in dem Rohstoff Pilinhib^{®} Veg LS 9109 von Laboratoires Sérobiologiques mit der INCI-Deklaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid" enthalten ist, weiterhin Wirkstoffkombinationen aus Extrakten aus Epilobium Angustifolium, den Samen von Cucurbita pepo (Kürbis) und den Früchten von Serenoa serrulata, wie sie beispielsweise und bevorzugt in dem Rohstoff ARP 100 von Greentech S.A./ Rahn mit der INCI-Deklaration "Water, Alcohol, Serenoa Serrulata Fruit Extract, Epilobium Angustifolium Extract, Cucurbita Pepo (Pumpkin) Seed Extract" enthalten sind, weiterhin Wirkstoffkombinationen aus Xylitol und Extrakten von Citrus Medica Limonum (Lemon) Fruit, Carica Papaya (Papaya) und Olivenblättern, wie sie beispielsweise und bevorzugt in dem Rohstoff Xyleine von Impag / Seporga mit der INCI-Deklaration "Xylitol and Citrus Medica Limonum (Lemon) Fruit Extract and Carica Papaya (Papaya) Fruit Extract and Olea europaea (olive) leaf extract" enthalten sind, weiterhin Wirkstoffkombinationen aus Humulus Lupulus, Viscum Album, Salvia Officinalis, Carica Papaya und Thuya Occidentalis, wie sie beispielsweise und bevorzugt in dem Rohstoff Plantafluid Komplex AH der Firma Plantapharm mit der INCI-Deklaration "Aqua, Propylene Glycol, Humulus Lupulus, Viscum Album, Salvia Officinalis, Carica Papaya, Thuya Occidentalis" enthalten sind, sowie Extrakten aus Larrea divaricata, wie sie beispielsweise in dem Rohstoff Capislow von Sederma, der Lecithinvesikel mit einem hydroglycolischen Extrakt aus Larrea divaricata enthält, enthalten sind.

Weitere bevorzugte Haarwuchs-inhibierende Wirkstoffe sind ausgewählt aus den Substanzen, die die Protein-Tyrosinkinase hemmen, insbesondere aus Lavendustin-A, Erbstatin, Tyrphosfin, Piceatannol, 4-Hydroxybenxylidenmalononitril, 3,5-Di-tert-butyl-4-hydroxybenzylidenmalononitril, α-Cyano-(3,4-dihydroxy)-cinnamonitril, α-Cyano-(3,4,5-trihydroxy)cinnamonitril, α-Cyano-(3,4-dihydroxy)cinnamid, α-Cyano-(3,4-dihydroxy)thiocinnamid, 2- Amino-4-(4'-hydroxyphenyl)-1,1,3-tricyanobuta-1,3-dien, 2-Amino-4-(3,4,5'- trihydroxyphenyl)-1,1,3-tricyanobuta-1,3-dien, 2-Amino-4-(1HA-indol-5-yl)-1,1,3-tricyanotuta-1,3-dien, 4-Hydroxy-3-methoxy-5-(benzothiazolylthiomethyl)benzylidencyanoacetamid, 4-Amino-N-(2,5-dihydroxybenzyl)methylbenzoat, α-Cyano-(3,4-dihydroxy)-cinnamonitril, 4-(3-Chloranilino)-6,7-dimethoxy-chinazolin, α-Cyano-(3,4-dihydroxy)-N-benzylcinnamid, (-)-R-N-(a-Methylbenzyl)-3,4-dihydroxybenzylidencyanoacetamid, α-Cyano-(3,4-dihydroxy)-N-(3- phenylpropyl)-cinnamid, α-Cyano-(3,4-dihydroxy)-N-phenylcinnamid, α-Cyano-(+)-(S)-N-(alpha-phenethyl)-(3,4-dihydroxy)cinnamid,α-Cyano-(3,4-dihydroxy)-N-(phenylbutyl)cinnamid, Herbimycin A, Thiazolidindion, Phenazocin, 2,3-Dihydro-2-thioxo-1H-indol-3-Alkansäuren, 2,2'-Dithiobis-(1H-indol-3-Alkansäuren), ein Sulfonylbenzoylnitrostyrol, Methylcaffeat, HNMPA(AM)₃ (hydroxy-2-naphthalenylmethylphosphonsäure-tris-acetoxymethylester) und N-Acetyl-Asp-Tyr-(2-malonyl)-Val-Pro-Met-Leu-NH₂.

Weitere bevorzugte Haarwuchs-inhibierende Wirkstoffe sind ausgewählt aus den Substanzen, die in WO 2006/130330 A2 offenbart sind, nämlich Agonisten des Farnesoid X-Rezeptors, bevorzugt ausgewählt aus Gallensäuren, wie insbesondere Lithocholsäure, Cholsäure, Deoxycholsäure, Chenodeoxycholsäure, Ursodeoxycholsäure und 6-alpha-Ethylchenodeoxycholsäure, weiterhin aus Farnesoiden, insbesondere Farnesol (3,7,11-Trimethyl-2,6,10-dodecatrien-1-ol), Farnesal, Farnesylacetat, 3,7,11-Trimethyl-2,6,10-dodecatrien-1-carbonsäure, Methylfarnesylether, Methylfarnesoat, Ethylfarnesylether, Ethylfarnesoat, weiterhin aus 7-Methyl-9-(3,3-dimethyloxivanyl)-3-methyl-2,6-nonadiensäuremethylester (Juvenilhormon III), 7-Methyl-9-(3,3-dimethyloxivanyl)-3-methyl-2,6-nonadiensäureethylester, 3-alpha,7-alpha-Dihydroxy-6-alpha-ethyl-5p-cholan-24-säure, 7-alpha-Dihydroxy-6-alpha-propyl-5p-cholan-24-säure, 7-alpha-Dihydroxy-6-alpha-allyl-5p-cholan-24-säure, N-(2,2,2-trifluoroethyl)-N-[4-[2,2,2-trifluoro-1-hydroxy-1-(trifluoromethyl)-ethyl]phenyl]-benzolsulfonanilid, 3-[2-[2-Chloro-4-[3-(2,6-dichlorophenyl)-5-(1-methyl-ethyl)-4-isoxazolyl]methoxyl-phenylethenyll-benzoesäure, [3,5-bis(1,1-dimethylethyl)-4-hydroxyphenyl]-ethenyliden]bisphosphonsäuretetraethylester, [2-[3,5-bis(1,1-dimethylethyl)-4-hydroxyphenyl]ethyliden]-bisphosphonsäuretetrakis(1-methylethyl)ester, [2-[3,5-bis(1,1-dimethylethyl)-4-hydroxyphenyl]-ethyliden]-bisphosphonsäuretetraethylester und [3,5-bis(1,1-dimethylethyl-4-hydroxyphenyl]ethenyliden]bisphosphonsäuretetrakis(1-methylethyl)ester.

Bevorzugte erfindungsgemäße Benefit-Zusammensetzungen enthalten mindestens eine den Haarwuchs inhibierende Substanz vorzugsweise in einer Menge von 0,1 - 10 Gew.-%, besonders bevorzugt 0,5 - 5 Gew.-% und außerordentlich bevorzugt 1 - 4 Gew.-%, jeweils bezogen auf das Gewicht des Rohstoffs tel quel und das Gesamtgewicht der erfindungsgemäßen Benefit-Zusammensetzung.

In einer weiteren bevorzugten Ausführungsform enthalten die erfindungsgemäßen Benefit-Zusammensetzungen mindestens einen Desoxyzucker oder ein mindestens einen Desoxyzucker-Baustein enthaltendes Polysaccharid.

Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid trägt die INCI-Bezeichnung Biosaccharide Gum-1 und ist beispielsweise in dem Handelsprodukt Fucogel^{®} von Solabia enthalten. Ein weiteres erfindungsgemäß besonders bevorzugtes, Desoxyzucker-Bausteine enthaltendes Polysaccharid trägt die INCI-Bezeichnung Biosaccharide Gum-2 und ist beispielsweise in dem Handelsprodukt Rhamnosoft^{®} von Solabia enthalten. Ein weiteres erfindungsgemäß besonders bevorzugtes, Desoxyzucker-Bausteine enthaltendes Polysaccharid trägt die INCI-Bezeichnung Biosaccharide Gum-3 und ist beispielsweise in dem Handelsprodukt Fucogenol^{®} von Solabia enthalten. Ein weiteres erfindungsgemäß besonders bevorzugtes, Desoxyzucker-Bausteine enthaltendes Polysaccharid trägt die INCI-Bezeichnung Biosaccharide Gum-4 und ist beispielsweise in dem Handelsprodukt Glycofilm^{®} von Solabia enthalten. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia. Erfindungsgemäß besonders bevorzugte Benefit-Zusammensetzungen sind dadurch gekennzeichnet, dass sie mindestens einen Desoxyzucker und/oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Benefit-Zusammensetzung, enthalten.

Weitere erfindungsgemäß bevorzugte Benefit-Zusammensetzungen sind dadurch gekennzeichnet, dass sie mindestens einen Antischuppenwirkstoff enthalten. Besonders bevorzugte Antischuppenwirkstoffe sind ausgewählt aus Piroctone Olamine, Zink Omadine und Climbazol. Erfindungsgemäß besonders bevorzugte Benefit-Zusammensetzungen sind dadurch gekennzeichnet, dass sie mindestens einen Antischuppenwirkstoff in einer Gesamtmenge von 0,01 - 5 Gew.-%, bevorzugt 0,1 - 2 Gew.-%, bezogen auf die Benefit-Zusammensetzung, enthalten.

Bevorzugte erfindungsgemäße kosmetische Produkte sind vorzugsweise
(a) kosmetische Produkte zur Hautpflege, insbesondere Badepräparate, Hautwasch- u. -reinigungsmittel, Hautpflegemittel, Augenkosmetika, Lippenpflegemittel, Nagelpflegemittel, Intimpflegemittel, Fußpflegemittel, Hautbleichmittel,
(b) kosmetische Produkte mit spezieller Pflegewirkung, insbesondere Lichtschutzmittel, Hautbräunungsmittel, Depigmentierungsmittel, Desodorantien, Antihidrotika, Haarentfemungsmittel, Rasiermittel, Duftmittel, Fingernagelbleichmittel,
(c) kosmetische Produkte zur Zahnpflege, insbesondere Zahn- und Mundpflegemittel, Gebisspflegemittel, Zahnprothesenreinigungsmittel, Zahnprothesenhaftmittel, Zahnbleichmittel, Zahnprothesenbleichmittel
(d) kosmetische Produkte zur Haarpflege, insbesondere Haarwaschmittel, Haarpflegemittel, Haarverfestigungsmittel, Haarverformungsmittel, Haarfärbemittel, Haarbleichmittel.

Bevorzugte Inhaltsstoffe der erfindungsgemäßen Mittel, insbesondere der kosmetischen Mittel, können über ihre Funktion definiert werden. Natürlich können manche Inhaltsstoffe auch multifunktionell sein. Bevorzugte Inhaltsstoffe der erfindungsgemäßen Mittel, vorzugsweise Kosmetikprodukte, können sein:
a) Absorptionsmittel
   Diese haben vorzugsweise die Aufgabe, wasser- und/oder öllösliche aufgelöste oder feinverteilte Substanzen aufzunehmen.
b) Antimikrobielle Stoffe
   Diese können den Produkten zugesetzt werden, um ganz allgemein die Aktivitäten von Mikroorganismen, z.B. auf der Haut und in der Mundhöhle, zu verringern.
c) Antioxidantien
   Diese sollen dazu dienen, durch Sauerstoff ausgelöste Reaktionen wie Oxidation zu verhindern und somit die Haltbarkeit der Produkte verlängern, d.h. die Qualität der Produkte erhalten.
d) Antiperspirantien
   Diese werden vorzugsweise in Kosmetika eingesetzt und verringern die Schweißabgabe.
e) Antischaummittel
   Diese können zugesetzt werden, z.B. um Schaum während der Herstellung zu beseitigen oder um
   die Neigung von Fertigprodukten zur überschießenden Schaumbildung zu verringern.
f) Antischuppenwirkstoffe
   Diese werden in erster Linie in Haarpflegeprodukten eingesetzt, da sie der Schuppenbildung entgegenwirken können.
g) Antistatika
   Das sind z.B. Kämmbarkeitshilfen in Haarpflegeprodukten. Sie verringern allgemein die elektrostatische Aufladung von Objekten, beispielsweise der Haaroberfläche. Haare lassen sich so deutlich
   leichter kämmen.
h) Bindemittel
   Sie gewährleisten z.B. den Zusammenhalt pulver- und puderhaltiger Produkte, wie z.B. kosmetischer Zubereitungen.
i) Bleichmittel
   Diese können z.B. dazu dienen, den Farbton des Haares oder der Haut aufzuhellen.
j) Chelatbildner
   Diese werden z.B. kosmetischen Mitteln zugesetzt, damit sie Komplexe mit Metallionen bilden, um so beispielsweise die Stabilität und/oder das Aussehen der Mittel zu manipulieren
k) Desodorierungsmittel/Antitranspirantien
   Diese können dazu beitragen, dass das Entstehen unangenehmer Körpergerüche verhindert oder verringert wird. Sie können solche Gerüche überdecken und ggf. die Schweißbildung reduzieren
l) Emollients
   Diese haben z.B. im kosmetischen Bereich die Aufgabe, die Haut geschmeidig zu machen und zu glätten.
m) Emulsionsstabilisatoren
   Diese können den Prozeß der Emulgierung (vgl. Emulgatoren) noch weiter unterstützen und auf diese Weise die Stabilität und Haltbarkeit des Produktes weiter verbessern.
n) Enthaarungsmittel
   Diese dienen der vorzugsweise selektiven Entfernung von Körperbehaarung.
o) Feuchtigkeitsspender
   Diese können einen Beitrag zur Erhaltung oder Wiederherstellung der Hautfeuchtigkeit leisten und dem Austrocknen der Haut entgegenwirken.
p) Filmbildner
   Diese sind dazu im Stande, beispielsweise in kosmetischen Mitteln einen schützenden, stabilisierenden Film auf Oberflächen. Vorzugsweise Haut, Haar oder Nägeln zu erzeugen
q) Farbstoff(vorprodukt)e
   Diese werden z.B. auch kosmetischen Produkten zugesetzt, um eine Produktfärbung zu erzeugen
   oder aber auch um eine mittelbare Objektfärbung zu bewirken, z.B. Haarfärbung
r) Konservierungsstoffe
   Diese werden z.B. kosmetischen Mitteln zugesetzt, um sie vor der schadhaften Einwirkung von
   Mikroorganismen (Bakterien, Pilze, Hefen) zu bewahren und damit ihren Verderb zu vermeiden.
s) Korrosionsschutzmittel
   Diese können z.B. dazu dienen, die Korrosion von Teilen, die mit dem efindungsgemäßen Produkt
   behandelt werden, zu verhindern.
t) Lösungsmittel (wasserfrei)
   Sie können z.B. die Grundlage für beispielsweise flüssige kosmetische Zubereitungen, aber auch
   Bestandteil fester Produkte sein.
u) Mundpflegestoffe
   Diese können der Pflege von Zähnen und Zahnfleisch dienen.
v) Oxidationsmittel
   Sie können dazu dienen, die chemische Beschaffenheit anderer Substanz durch Oxidation zu ändern.
w) pH-Wert-Regler/Puffersubstanzen
   Diese können z.B. in Kosmetika dazu dienen, um einen gewünschten pH-Wert einzustellen bzw. zu stabilisieren.
x) Reduktionsmittel
   Diese sind im Stande, die chemische Beschaffenheit einer anderen Substanz durch Redoxprozesse zu ändern.
y) Schleifmittel
   Diese können dazu dienen, um Materialien von verschiedenen (Körper-)Oberflächen zu entfernen, beispielsweise um die mechanische Zahnreinigung zu unterstützen oder um den Zahnglanz zu verbessern.

Das erfindungsgemäße Produkt kann vorzugsweise einen oder mehrere antimikrobielle Wirkstoffe bzw. Konservierungsmittel in einer Menge von üblicherweise 0,0001 bis 3 Gew.-%, vorzugsweise 0,0001 bis 2 Gew.-%, insbesondere 0,0002 bis 1 Gew.-%, besonders bevorzugt 0,0002 bis 0,2 Gew.-%, äußerst bevorzugt 0,0003 bis 0,1 Gew.-%, jeweils bezogen auf die gesamte Benefit-Zusammensetzung, enthalten.

Antimikrobielle Wirkstoffe bzw. Konservierungsmittel unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung. Geeignete antimikrobielle Wirkstoffe sind vorzugsweise ausgewählt aus den Gruppen der Alkohole, Amine, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-di-cyanobutan, lodo-2-propyl-butyl-carbamat, Iod, lodophore, Peroxoverbindungen, Halogenverbindungen sowie beliebigen Gemischen der vorstehend genannten Substanzen.

Der antimikrobielle Wirkstoff ist dabei bevorzugt ausgewählt aus Phenoxyethanol, Undecylensäure, Benzoesäure, Salicylsäure, Dihydracetsäure, o-Phenylphenol, N-Methylmorpholin-aceto-nitril (MMA), 2 Benzyl-4-chlorphenol, 2,2'-Methylen-bis-(6-brom-4-chlorphenol), 4,4'-Di-chlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-hamstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschließlich den Bi- und Polyguani-dinen, wie beispielsweise 1,6-Bis-(2-ethylhexyl-biguanido-hexan)-dihydrochlorid, 1,6-Di-(N₁,N₁'-phenyldiguanido-N₅,N₅')-hexan-tetrahydochlorid, 1,6-Di-(N₁,N₁'-phenyl-N₁,N₁-methyldiguanido-N₅,N₅')-hexan-dihydro-chlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N_{1,}N₁'-beta-(p-methoxyphenyl) diguanido-N₅,N₅']-hexan-dihydrochlorid, 1,6-Di-(N₁,N₁'-alpha-methyl-.beta.-phenyldiguanido-N₅,N₅')-hexan-dihydro-chlorid, 1,6-Di-(N₁,N₁'-p-nitrophenyldiguanido-N₅,N₅')hexan-dihydrochlorid, omega:omega-Di-(N₁,N₁'-phenyldiguanido-N₅,N₅')-di-n-propylether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4-dichlorophenyldiguanido-N₅,N₅')hexan-tetrahydrochlorid, 1,6-Di-(N₁,N₁'-p-methylphenyldiguanido-N₅,N₅')hexan-dihydrochlorid, 1,6-Di-(N₁,N₁'-2,4,5-trichlorophenyldiguanido-N₅,N₅')hexantetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophe-nyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido-N₅,N₅')-decan-tetrahydrochlorid, 1,12-Di-(N₁,N₁'-phenyldiguanido- N₅,N₅') dodecan-tetrahydrochlorid, 1,6-Di-(N₁,N₁'-o-chlorophenyldi-guanido-N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbi-guanid), Ethylen-bis-(N-butylphenylbi-guanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Bu- tyl-ethylen-bis-(phenylbiguanid), Trimethylen-bis-(o-tolylbiguanid), N-Butyl-trimethylenbis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetacresol oder p-Chlormetaxylol, sowie natürliche antimikrobielle Wirkstoffe pflanzlicher Herkunft (z.B. aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/ oder mindestens ein natürlicher antimikrobieller Wirkstoff tierischer Herkunft aus der Gruppe, umfassend Enzyme wie Eiweiß aus Milch, Lysozym und Lactoperoxidase, und/ oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden. Vorzugsweise finden Glycin, Glycinderivate, Formaldehyd, Verbindungen, die leicht Formaldehyd abspalten, Ameisensäure und Peroxide Verwendung.

Die als antimikrobielle Wirkstoffe bevorzugten quaternären Ammoniumverbindungen (QAV) sind oben schon beschrieben worden. Besonders bevorzugt ist beispielsweise Benzalkoniumchlorid. Benzalkoniumhalogenide und/ oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche bevorzugte antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Das erfindungsgemäße Produkt kann nach einer weiteren bevorzugten Ausführungsform mindestens einen Riechstoff enthalten. Dabei kann der Riechstoff in der Benefit-Zusammensetzung enthalten sein, er kann aber auch direkt auf das Substrat aufgetragen sein. Die Erwärmung der Benefit-Zusammensetzung beim Kontakt mit Wasser sorgt für eine schnelle Freisetzung der Riechstoffe und damit für ein besonders intensives Dufterlebnis des Verbrauchers. Erfindungsgemäß besonders bevorzugt enthalten sind insbesondere Riechstoffe mit
(a) mandelartigem Geruch, wie vorzugsweise Benzaldehyd, Pentanal, Heptenal, 5-Methylfurfural, Methylbutanal, Furfural und/oder Acetophenon oder
(b) apfelartigem Geruch, wie vorzugsweise (S)-(+)-Ethyl-2-methylbutanoat, Diethylmalonat, Ethylbutyrat, Geranylbutyrat, Geranylisopentanoat, Isobutylacetat, Linalylisopentanoat, (E)-β-Damascone, Heptyl-2-methylbutyrat, Methyl-3-methylbutanoat, 2-Hexenal-pentyl-methylbutyrat, Ethylmethylbutyrate und/oder Methyl-2-Methylbutanoat oder
(c) apfelschalenartigem Geruch, wie vorzugsweise Ethylhexanoat, Hexylbutanoat und/oder Hexylhexanoat oder
(d) aprikosenartigem Geruch wie vorzugsweise γ-Undecalacton oder
(e) bananenartigem Geruch, wie vorzugsweise Isobutylacetat, Isoamylacetat, Hexenylacetat und/oder Pentylbutanoat oder
(f) bittermandelartigem Geruch wie vorzugsweise 4-Acetyltoluol oder
(g) schwarze Johannisbeere-artigem Geruch wie vorzugsweise Mercaptomethylpentanon und/oder Methoxymethylbutanethiol oder
(h) zitrusartigem Geruch wie vorzugsweise Linalylpentanoat, Heptanal, Linalylisopentanoat, Dodecanal, Linalylformiat, α-p-Dimethylstyrol, p-Cymenol, Nonanal, β-Cubebene, (Z)-Limonenoxid, cis-6-Ethenyltetrahydro-2,2,6-trimethylpyran-3-ol, cis-Pyranoidlinalooloxid, Dihydrolinalool, 6(10)-Dihydromyrcenol, Dihydromyrcenol, β-Farnesen, (Z)-β-Farnesen, (Z)-Ocimen, (E)-Limonenoxid, Dihydroterpinylacetat, (+)-Limonen, (Epoxymethylbutyl)-methylfuran und/oder p-Cymen oder
(i) kakaoartigem Geruch wie vorzugsweise Dimethylpyrazin, Butylmethylbutyrat und/oder Methylbutanal oder
(j) kokusnußartigem Geruch, wie vorzugsweise γ-Octalacton, γ-Nonalacton, Methyllaurat, Tetradecanol, Methylnonanoat, (3S,3aS,7aR)-3a,4,5,7a-tetrahydro-3,6-dimethylbenzofuran-2(3H)-on, 5-Butyl-dihydro-4-methyl- 2(3H)-Furanon, Ethylundecanoat und/oder δ-Decalacton oder
(k) sahneartigem Geruch wie vorzugsweise Diethylacetal, 3-Hydroxy-2-butanon, 2,3-Pentadion und/oder 4-Heptenal oder
(l) blumenartigem Geruch wie vorzugsweise Benzylalcohol, Phenylessigsäure, Tridecanal, p-Anisylalcohol, Hexanol, (E,E)-Farnesylaceton, Methylgeranat, trans-Crotonaldehyd, Tetradecylaldehyd, Methylanthranilat, Linalooloxid, Epoxylinalool, Phytol, 10-epi-γ-Eudesmol, Neroloxid, Ethyldihydrocinnamat, γ-Dodecalacton, Hexadecanol, 4-Mercapto-4-methyl-2-pentanol, (Z)-Ocimene, Cetylalcohol, Nerolidol, Ethyl-(E)-cinnamat, Elemicin, Pinocarveol, α-Bisabolol, (2R,4R)-Tetrahydro-4-methyl-2-(2-methyl-1-propenyl)-2H-pyran, (E)-Isoelemicin, Methyl-2- methylpropanoat, Trimethylphenylbutenon, 2-Methylanisol, β-Farnesol, (E)-Isoeugenol, Nitro-phenylethan, Ethylvanillat, 6-Methoxyeugenol, Linalool, β-Ionon, Trimethylphenylbutenon, Ethylbenzoat, Phenylethylbenzoat; Isoeugenol und/oder Acetophenone oder
(m) Frische-Geruch wie vorzugsweise Methylhexanoat, Undecanon, (Z)-Limonenoxid, Benzylacetat, Ethylhydroxyhexanoat, Isopropylhexanoat, Pentadecanal, β-Elemene, α-Zingiberene, (E)-Limonenoxid, (E)-p-Mentha-2,8-dien-1-ol, Menthon, Piperiton, (E)-3-Hexenol und/oder Carveol oder
(n) Frucht-Geruch wie verzugsweise Ethylphenylacetat, Geranylvalerat, γ-Heptalacton, Ethylpropionat, Diethylacetal, Geranylbutyrat, Ethylheptylat, Ethyloctanoat, Methylhexanoat, Dimethylheptenal, Pentanon, Ethyl-3-methylbutanoat, Geranylisovalerat, lobutylacetat, Ethoxypropanol, Mthyl-2-butenal, Methylnonanedion, Linalylacetat, Mthylgeranat, Limonenoxid, Hydrozimtalkohol, Diethylsuccinat, Ehylhexanoat, Ehylmethylpyrazin, Neryletat, Citronellylbutyrat, Heylacetat, Nonylacetat, Butylmethylbutyrat, Pentenal, Isopentyldimethylpyrazin, p-menth-1-en-9-ol, Hexadecanon, Octylacetat, γ-Dodecalacton, Epoxy-β-ionon, Ethyloctenoat, Ethylisohexanoat, Isobornylpropionat, Cedrenol, p-menth-1-en-9-yl acetat, Cadinadien, (Z)-3-Hexenylhexanoat, Ethylcyclohexanoat, 4-Methylthio-2-butanon, 3,5-Octadienon, Methylcyclohexancarboxylat, 2-pentylthiophen, α-Ocimene, Butandiol, Ethylvalerat, Pentanol, Isopiperiton, Butyloctanoat, Ethylvanillat, Methylbutanoat, 2-Methylbutylacetat, Propylhexanoat, Butylhexanoat, Isopropylbutanoat, Spathulenol, Butanol, δ-Dodecalacton, Methylquinoxalin, Sesquiphellandren, 2-Hexenol, Ethylbenzoate,lsopropylbenzoat, Ethyllactat und/oder Citronellylisobutyrat oder
(o) Geranium-artigen Geruch, wie vorzugsweise Geraniol, (E,Z)-2,4-Nonadienal, Octadienon und/oder o-Xylen oder
(p) weintraubenartigem Geruch wie vorzugsweise Ethyldecanoat und/oder Hexanon oder
(q) grapefruitartigem Geruch wie vorzugsweise (+)-5,6-Dimethyl-8-isopropenylbicyclo[4.4.0]dec-1-en-3-on und/oder p-Menthenethiol oder
(r) grasartigem Geruch wie vorzugsweise 2-Ethylpyridin, 2,6-Dimethylnaphthalen, Hexanal und/oder (Z)-3-Hexenol oder
(s) grüner Note, vorzugsweise 2-Ethylhexanol, 6-Decenal, Dimethylheptenal, Hexanol, Heptanol, Methyl-2-butenal, Hexyloctanoate, Nonansäure, Undecanon, Methylgeranat, Isobornylformiate, Butanal, Octanal, Nonanal, Epoxy-2-decenal, cis-Linalool, Pyranoxid, Nonanol, alpha, γ-dimethylallylalcohol, (Z)-2-penten-1-ol, (Z)-3-hexenylbutanoat, Isobutylthiazol, (E)-2-nonenal, 2-dodecenal, (Z)-4-decenal, 2-octenal, 2-hepten-1-al, Bicyclogermacrene, 2-Octenal, α-Thujene, (Z)-β-Farnesene, (-)-γ-Elemene, 2,4-Octadienal, Fucoserraten, Hexenylacetat, Geranylaceton, Valencene, β-Eudesmol, 1-Hexenol, (E)-2-Undecenal, Artemisia keton, Viridiflorol, 2,6-Nonadienal, Trimethylphenylbutenon, 2,4-Nonadienal, Butylisothiocyanat, 2-Pentanol, Elemol, 2-Hexenal, 3-Hexenal, (+)-(E)- Limonenoxid, cis-Isocitral, Dimethyloctadienal, Bornylformiat, Bornylisovalerat, Isobutyraldehyd, 2,4-Hexadienal, Trimethylphenylbutenon, Nonanon, (E)-2-Hexenal, (+)-cis-Rosenoxide, Menthone, Coumarin, (Epoxymethylbutyl)-methylfuran, 2-Hexenol, (E)-2-hexenol und/oder Carvylacetat oder
(t) Grüner Tee-artigem Geruch, vorzugsweise (-)-Cubenol oder
(u) kräuterartigem Geruch, vorzugsweise Octanon, Hexyloctanoat, Caryophyllenoxide, Methylbutenol, Safranal, Benzylbenzoat, Bornylbutyrat, Hexylacetat, β-Bisabolol, Piperitol, β-Selinene, α-Cubebene, p-Menth-l-en-9-ol, 1,5,9,9-Tetramethyl-12-oxabicyclododeca-4,7-dien, T-muurolol, (-)-Cubenol, Levomenol, Ocimene, α-Thujene, p-Menth-1-en-9-yl acetat, Dehydrocarveol, Artemisia alcohol, γ-Muurolene, Hydroxypentanon, (Z)-Ocimene, β-Elemene, δ-Cadinol, (E)-β-Ocimene, (Z)-Dihydrocarvone, α-Cadinol, Calamenen, (Z)-Piperitol, Lavandulol, β-Bourbonene, (Z)-3-Hexenyl-2-methylbutanoat, 4-(1-Methylethyl)-benzenemethanol, Artemisia keton, Methyl-2-butenol, Heptanol, (E)-Dihydrocarvon, p-2-Menthen-1-ol, α-Curcumene, Spathulenol, Sesquiphellandren, Citronellylvalerat, Bornylisovalerat, 1,5-Octadien-3-ol, Methylbenzoat, 2,3,4,5-Tetrahydroanisol und/oder Hydroxycalamenen oder
(v) honigartigem Geruch, vorzugsweise Ethylcinnamate, β-Phenethylacetat, Phenylessigsäure, Phenylethanal, Methylanthranilat, Zimtsäure, β-Damascenone, Ethyl-(E)-cinnamat, 2-Phenylethylalkohol, Citronellylvalerate, Phenylethylbenzoate und/oder Eugenol oder
(w) Hyazinthen-artigem Geruch, vorzugsweise Hotrienol oder
(x) jasminartigem Geruch, vorzugsweise Methyljasmonate, Methyldihydroepijasmonat und/oder Methylepijasmonat oder
(y) lavendelartigem Geruch, vorzugsweise Linalylvalerate und/oder Linalool oder
(z) zitronenartigem Geruch, vorzugsweise Neral, Octanal, δ-3-Carene, Limonen, Geranial, 4-Mercapto-4-methyl-2-pentanol, Citral, 2,3-Dehydro-1,8-cineol und/oder α-Terpinen oder
(aa) lilienartigem Geruch, vorzugsweise Dodecanal oder
(bb) magnolienartigem Geruch, vorzugsweise Geranylaceton oder
(cc) mandarinenartigem Geruch, vorzugsweise Undecanol oder
(dd) melonenartigem Geruch, vorzugsweise Dimethylheptenal oder
(ee) Minze-artigem Geruch, vorzugsweise Menthone, Ethylsalicylat, p-Anisaldehyd, 2,4,5,7a-tetrahydro-3,6-dimethyl-benzofuran, Epoxy-p-menthene, Geranial, (Methylbutenyl)-methylfuran, Dihydrocarvylacetat, β-Cyclocitral, 1,8-Cineol, β-Phellandrene, Methylpentanon, (+)-Limonen, Dihydrocarveol (-)-Carvon, (E)-p-Mentha-2,8-dien-l-ol, Isopulegylacetat, Piperiton, 2,3-Dehydro-1,8-cineol, α-Terpineol, DL-carvon und/oder α-Phellandrene oder
(ff) nußartigem Geruch, vorzugsweise 5-methyl-(E)-2-hepten-4-on, γ-Heptalacton, 2-Acetylpyrrol, 3-Octen-2-on, Dihydromethylcyclopentapyrazin, Acetylthiazol, 2-Octenal, 2,4-Heptadienal, 3-Octenon, Hydroxypentanon, Octanol, Dimethylpyrazin, Methylquinoxalin und/oder Acetylpyrrolin oder
(gg) orangenartigem Geruch, vorzugsweise Methyloctanoat, Undecanon, Decylalcohol, Limonen
   und/oder 2-Decenal oder
(hh) Orangenschalen-artigem Geruch, vorzugsweise Decanal und/oder β-Carene oder
(ii) pfirsichartigem Geruch, vorzugsweise γ-Nonalacton, (Z)-6-Dodecene-y-lacton, δ-Decalacton, R-δ-Decenolacton, Hexylhexanoat, 5-Octanolid, γ-Decalacton und/oder δ-Undecalacton oder
(jj) Pfeffertninze-artigem Geruch, vorzugsweise Methylsalicylat und/oder I-Menthol oder
(kk) Kiefer-artigem Geruch, vorzugsweise α-p-Dimethylstyrol, β-Pinene, Bornylbenzoat, δ-Terpinen,
   Dihydroterpinylacetat und/oder α-Pinen oder
(ll) ananasartigem Geruch, vorzugsweise Propylbutyrat, Propylpropanoat und/oder Ethylacetat oder
(mm)pflaumenartigem Geruch, vorzugsweise Benzylbutanoat, oder
(nn) himbeerartigem Geruch, vorzugsweise β-Ionone oder
(oo) Rosen-artigem Geruch, vorzugsweise β-Phenethylacetat, 2-Ethylhexanol, Geranylvalerat, Geranylacetat, Citronellol, Geraniol, Geranylbutyrat, Geranylisovalerat, Citronellylbutyrat, Citronellylacetat, Isogeraniol, Tetrahydro-4-methyl-2-(2-methyl-1-propenyl)-2,5-cis-2H-pyran, Isogeraniol, 2-Phenylethylalcohol, Citronellylvalerat und/oder Citronellylisobutyrat, oder
(pp) Grüne Minze-artigem Geruch, vorzugsweise Carvylacetate und/oder Carveol, oder
(qq) erdbeerenartigem Geruch, vorzugsweise Hexylmethylbutyrat, Methylcinnamat, Pentenal, Methylcinnamate oder
(rr) süßlichem Geruch, vorzugsweise Benzylalcohol, Ethylphenylacetat, Tridecanal, Nerol, Methylhexanoat, Linalylisovalerat, Undecanaldehyd, Caryophyllenoxid, Linalylacetat, Safranal, Uncineol, Phenylethanal, p-Anisaldehyd, Eudesmol, Ethylmethylpyrazin, Citronellylbutyrat, 4-Methyl-3-penten-2-on, Nonylacetat, 10-Epi-γ-eudesmol, β-Bisabolol, (Z)-6-Dodecen-y-lacton, β-Famesene, 2-Dodecenal, γ-Dodecalacton, Epoxy-β-ionon, 2-Undecenal, Styrenglycol, Methylfuraneol, (-)-cis-Rosenoxid, (E)-β-Ocimene, Dimethylmethoxyfuranon, 1,8-Cineole, Ethylbenzaldehyd, 2-Pentylthiophen, o-Farnesene, Methionol, 7-Methoxycoumarin, (Z)-3-Hexenyl-2-methylbutanoat, o-Aminoacetophenon, Viridiflorol, Isopiperitone, β-Sinensal, Ethylvanillat, Methylbutanoat, p-Methoxystyrol, 6-Methoxyeugenol, 4-Hexanolid, δ-Dodecalacton, Sesquiphellandren, Diethylmalat, Linalylbutyrat, Guaiacol, Coumarin, Methylbenzoat, Isopropylbenzoat, Safrole, Durene, γ-Butyrolacton, Ethylisobutvrat und/oder Furfural oder
(ss) Vanille-artigem Geruch, vorzugsweise Vanillin, Methylvanillat, Acetovanillon und/oder Ethylvanillat oder
(tt) wassermelonenartigem Geruch, vorzugsweise 2,4-Nonadienal oder
(uu) holzartigem Geruch, vorzugsweise α-Muurolene, Cadina-1,4-dien-3-ol, Isocaryophyllene, Eudesmol, α-Ionon, Bornylbutyrat, (E)-α-Bergamoten, Linalooloxid, Ethylpyrazin, 10-epi-γ-Eudesmol, Germacrene B, trans-Sabinenhydrat, Dihydrolinalool, Isodihydrocarveol, β-Farnesene, β-Sesquiphellandren, δ-Elemene, α-Calacorene, Epoxy-β-ionon, Germacrene D, Bicyclogermacrene, Alloaromadendrene, α-Thujene, oxo-β-Ionon, (-)-γ-Elemene, γ-Muurolene, Sabinene, α-Guaiene, α-Copaene, γ-Cadinene, Nerolidol, β-Eudesmol, α-Cadinol, δ-Cadinene, 4,5-Dimethoxy-6-(2-propenyl)-1,3-benzodioxol, [1ar-(1aalpha,4aalpha,7alpha, 7abeta,7balpha)]-decahydro-1,1,7-trimethyl-4-methylene-1H-cycloprop[e]azulen, α-Gurjunen, Guaiol, α-Farnesene, γ-Selinene, 4-(1-Methylethyl)-benzenemethanol, Perillen, Elemol, α-Humulene, β-Caryophyllene und/oder β-Guaiene oder
(vv) Mischungen aus vorgenannten.

In einer weiteren bevorzugten erfindungsgemäßen Ausführungsform sind die Riechstoffe in derivatisierter Form enthalten, aus der sie bei der Anwendung mit Wasser zeitverzögert freigesetzt werden. Für diese Zwecke besonders bevorzugte Riechstoff-Derivate sind Siliciumverbindungen, wie sie in US 3215719, GB 2041964 A und GB 2319527 A offenbart sind und insbesondere Kieselsäureester, wie sie in US 20040072704 A1, DE 10 2004 028018 A1 und in DE 102005059935 A1 offenbart sind. Aber auch andere Riechstoffderivate, die durch Hydrolyse oder andere Reaktionsmechanismen Riechstoffmoleküle freisetzen, sind erfindungsgemäß bevorzugt.

In einer weiteren bevorzugten erfindungsgemäßen Ausführungsform sind die Riechstoffe in verkapselter Form enthalten, aus der sie bei der Anwendung mit Wasser und/oder bei der mechanischen Anwendung des erfindungsgemäßen Produktes zeitverzögert freigesetzt werden.

Es kann erfindungsgemäß besonders bevorzugt sein, mindestens einen unverkapselten und nicht-derivatisierten Riechstoff in Kombination mit mindestens einem verkapselten und/oder mindestens einem derivatisierten Riechstoff einzusetzen. Dies ermöglicht eine zeitlich gestaffelte Freisetzung verschiedener Riechstoffe und ein außergewöhnlichea Dufterlebnis für den Verbraucher.

In einer bevorzugten Ausführungsform enthält die erfindungsgemäße Benefit-Zusammensetzung bestimmte Minimalwerte an Parfümöl, nämlich zumindest 0,00001 Gew.-%, vorteilhafterweise zumindest 0,0001 Gew.-%, in beträchtlich vorteilhafter Weise zumindest 0,001 Gew.-%, in vorteilhafterer Weise zumindest 0,01 Gew.-%, in weiter vorteilhafter Weise zumindest 0,1 Gew.-%, in noch weiter vorteilhafter Weise zumindest 0,2 Gew.-%, in sehr vorteilhafter Weise zumindest 0,3 Gew.-%, in besonders vorteilhafter Weise zumindest 0,4 Gew.-%, in ganz besonders vorteilhafter Weise zumindest 0,45 Gew.-%, in erheblich vorteilhafter Weise zumindest 0,5 Gew.-%, in ganz erheblich vorteilhafter Weise zumindest 0,55 Gew.-%, in äußerst vorteilhafter Weise zumindest 0,6 Gew.-%, in höchst vorteilhafterweise zumindest 0,65 Gew.-%, in überaus vorteilhafterweise zumindest 0,7 Gew.-%, in ausnehmend vorteilhafter Weise zumindest 0,75 Gew.-%, in außergewöhnlich vorteilhafter Weise zumindest 0,8 Gew.-%, in außerordentlich vorteilhafter Weise zumindest 0,85 Gew.-%, insbesondere zumindest 0,9 Gew.-% an Parfümöl, bezogen auf die Benefit-Zusammensetzung.

In einer weiteren bevorzugten Ausführungsform enthalten die Parfümöle weniger als 8, vorteilhafterweise weniger als 7, in vorteilhafterer Weise weniger als 6, in wiederum vorteilhafterer Weise weniger als 5, in weiter vorteilhafterweise weniger als 4, noch vorteilhafter weniger als 3, vorzugsweise weniger als 2, insbesondere keine Duftstoffe aus der Liste Amylcinnamal, Amylcinnamylalkohol, Benzylalkohol, Benzylsalicylat, Cinnamylalkohol, Cinnamal, Citral, Cumarin, Eugenol, Geraniol, Hydroxycitronellal, Hydroxymethylpentylcyclohexencarboxaldehyd, Isoeugenol, Anisylalkohol, Benzylbenzoat, Benzylcinnamat, Citronellol, Farnesol, Hexylcinnamaldehyd, Lilial, d-Limonen, Linalool, Methylheptincarbonat, 3-Methyl-4-(2,6,6-trimethyl-2-cyclohexen-1-yl)-3-buten-2-on, Eichenmoosextrakt, Baummoosextrakt.

In einer besonders bevorzugten Ausführungsform enthalten die erfindungsgemäßen Benefit-Zusammensetzungen das/die Parfümöl/e in geträgerter oder verkapselter Form. Als (pulverförmiger) Träger für Parfümöle sind insbesondere Talkum, Stärken, Cellulosen, Maltodextrin und Cyclodextrine bevorzugt. Nach einer weiteren bevorzugten Ausführungsform kann das erfindungsgemäße Produkt frei von Parfümöl sein. Dies ist insbesondere empfehlenswert für Verbraucher, die zu Allergien neigen.

UV-Schutz-Substanzen

In einer weiteren bevorzugten Ausführungsform enthalten die erfindungsgemäßen Benefit-Zusammensetzungen mindestens eine anorganische und/oder mindestens eine organische UV-Filtersubstanz.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäßen Benefit-Zusammensetzungen können die UV-Filtersubstanzen entweder als Produktschutz (zum Schutz lichtempfindlicher Substanzen) oder als kosmetische oder dermatologische Sonnen- oder Lichtschutzzusammensetzung für das Haut und die Haare enthalten.

Die erfindungsgemäß bevorzugten organischen UV-Filter sind ausgewählt aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestem und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino) benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone, Uvinul^{®} T 150), Dimethicodiethylbenzal malonate (CAS-Nr. 207574-74-1, Parsol^{®} SLX), Dioctyl Butamido Triazone (Uvasorb^{®} HEB), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A) und sowie beliebige Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusotex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß besonders bevorzugte Benefit-Zusammensetzungen sind dadurch gekennzeichnet, dass sie mindestens eine organische UV-Filtersubstanz in einer Gesamtmenge von 0,1 - 30 Gew.%, bevorzugt 0,5 - 20 Gew.%, besonders bevorzugt 1,0 - 15 Gew.-% und außerordentlich bevorzugt 3,0 - 10 Gew.-%, jeweils bezogen auf die gesamte Benefit-Zusammensetzung, enthalten.

Weitere erfindungsgemäß besonders bevorzugte Benefit-Zusammensetzungen sind dadurch gekennzeichnet, dass sie mindestens eine anorganische UV-Filtersubstanz in einer Gesamtmenge von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Benefit-Zusammensetzung, enthalten.

Bevorzugte erfindungsgemäße Produkte sind dadurch gekennzeichnet, dass in den Benefit-Zusammensetzungen mindestens ein unter Normalbedingungen fester, teilchenförmiger inerter Füllstoff enthalten ist. Über den Füllstoffgehalt lässt sich - in überraschender Weise - insbesondere die Wärmeentwicklung regulieren. Der Füllstoffgehalt kann darüber hinaus den Feuchtegehalt und die Qualität der Benefit-Zusammensetzung bei längerer Lagerung regulieren.

In einer außerordentlich bevorzugten Ausführungsform der Erfindung ist dieser Füllstoff ausgewählt aus gegebenenfalls modifizierten Stärken (z. B. aus Mais, Reis, Kartoffeln) und Stärkederivaten, die gewünschtenfalls vorverkleistert sind, insbesondere Stärkederivaten vom Typ DRY FLO^{®}, Cellulose und Cellulosederivaten, Siliciumdioxid, amorphen Kieselsäuren, insbesondere Fällungskieselsäuren, besonders bevorzugt pyrogenen Kieselsäuren, z. B. erhältlich unter der Handelsbezeichnung Aerosil^{®}, sphärischen Polyalkylsesquisiloxan-Partikeln (insbesondere Aerosil^{®} R972 und Aerosil^{®} 200V von Degussa), Kieselgelen, Talkum, Kaolin, Tonen, insbesondere Bentoniten, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol^{®} OE, Glaspulvern, organischen Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen.

Als Füllstoffe erfindungsgemäß bevorzugte Polymerpulver auf Basis eines Polymethacrylat-Copolymers sind z. B. als Handelsprodukt Polytrap^{®} 6603 (Dow Corning) erhältlich. Als Füllstoffe erfindungsgemäß bevorzugte Polymerpulver auf Basis von Polyamiden sind beispielsweise unter der Bezeichnung Orgasol^{®} 1002 (Polyamid-6) und Orgasol^{®} 2002 (Polyamid-12) von Elf Atochem erhältlich. Weitere als Füllstoffe erfindungsgemäß bevorzugte Polymerpulver sind vernetzte Polymethacrylate und Polymethylmethacrylate (von SEPPIC insbesondere Covabead LH 85), z. B. die Produkte der Serie Micropearl^{®} M, Micropearl^{®} 305 und Micropearl^{®} 310 von SEPPIC, Covabead von Cognis oder Plastic Powder A von NIKKOL), Styrol-Divinylbenzol-Copolymere (z. B. Plastic Powder FP von NIKKOL), Polyethylen- und Polypropylen-Pulver (z. B. ACCUREL^{®} EP 400 von AKZO) oder auch Siliconpolymere (z. B. Silicone Powder X2-1605 von Dow Corning).

Besonders bevorzugte Füllstoffe sind ausgewählt aus wasserunlöslichen Füllstoffen, insbesondere ausgewählt aus amorphen Kieselsäuren, insbesondere Fällungskieselsäuren, besonders bevorzugt pyrogenen Kieselsäuren, Talkum, Kaolin, Tonen, insbesondere Bentoniten, Magnesiumaluminiumsilikaten und organischen Polymerpulvern, außerordentlich bevorzugt sind amorphen Kieselsäuren, insbesondere Fällungskieselsäuren, besonders bevorzugt pyrogenen Kieselsäuren, Talkum, Kaolin, Tonen, insbesondere Bentoniten, wobei Talkum aufgrund seiner herausragenden Eigenschaften besonders hervorzuheben ist.

Besonders bevorzugte erfindungsgemäße Produkte sind dadurch gekennzeichnet, dass die Benefit-Zusammensetzungen mindestens einen festen teilchenförmigen inerten Füllstoff in einer Gesamtmenge von 0,01 bis 80 Gew-%, bevorzugt 1 - 75 Gew. %, besonders bevorzugt 5 - 70 Gew.-%, außerordentlich bevorzugt 10 - 65 Gew.-%, jeweils bezogen auf die Benefit-Zusammensetzung, enthalten.

Besonders bevorzugte erfindungsgemäße Produkte sind dadurch gekennzeichnet, dass die Benefit-Zusammensetzungen mindestens einen festen, wasserunlöslichen teilchenförmigen inerten Füllstoff in einer Gesamtmenge von 0,01 bis 80 Gew.-%, bevorzugt 1 - 75 Gew.-%, besonders bevorzugt 5 - 70 Gew.-%, außerordentlich bevorzugt 10 - 65 Gew.-%, jeweils bezogen auf die Benefit-Zusammensetzung, enthalten.

Weitere erfindungsgemäß bevorzugte Produkte sind dadurch gekennzeichnet, dass die Benefit-Zusammensetzung mindestens einen verkapselten Benefit-Wirkstoff enthält.

Unter dem Begriff "verkapselter Benefit-Wirkstoff" werden Aggregate verstanden, die mindestens einen festen oder flüssige Benefit-Wirkstoff-haltigen Kern enthalten, der von mindestens einer kontinuierlichen Hülle umschlossen ist. Besonders bevorzugt handelt es sich um mit filmbildenden Polymeren umhüllte feindisperse flüssige oder feste Benefit-haltige Phasen, bei deren Herstellung sich die Polymere nach Emulgierung und Koazervation oder Grenzflächenpolymerisation auf dem einzuhüllenden Material niederschlagen. Die Kapseln lassen sich wie Pulver trocknen. Neben einkernigen Kapseln sind auch mehrkernige Aggregate bekannt, die zwei oder mehr Kerne im kontinuierlichen Hüllmaterial verteilt enthalten. Ein- oder mehrkernige Kapseln können zudem von einer zusätzlichen zweiten, dritten etc. Hülle umschlossen sein. Bevorzugt sind einkernige Kapseln mit einer kontinuierlichen Hülle. Die Hülle kann aus natürlichen, halbsynthetischen oder synthetischen Materialien bestehen. Natürliche Hüllmaterialien sind beispielsweise Gummi arabicum, Agar Agar, Agarose, Maltodextrine, Alginsäure bzw. ihre Salze, z. B. Natrium- oder Calciumalginate, Fette und Fettsäuren, Cetylalkohol, Collagen, Chitosan, Phospholipide, insbesondere Lecithine, die gehärtet (hydriert) oder ungehärtet (nicht hydriert) sein können, Gelatine, Albumin, Schellack, Polysaccharide, wie Stärke oder Dextran, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Füllmaterialien sind beispielsweise Polymere wie Polyacrylate, Polyamide, Polyvinylalkohole oder Polyvinylpyrrolidon. Besonders bevorzugte erfindungsgemäße Produkte sind dadurch gekennzeichnet, dass die Benefit-Zusammensetzung Kapseln enthält, deren Hüllsubstanz ausgewählt ist aus der Gruppe Gummi arabicum, Agar, Agarose, Maltodextrine, Alginsäure, Alginate, Fette, Fettsäuren, Cetylalkohol, Collagen, Chitosan, Phospholipide, insbesondere Lecithine, die gehärtet (hydriert) oder ungehärtet (nicht hydriert) sein können, Gelatine, Albumin, Schellack, Polysaccharide, Cellulosen, Celluloseester, Celluloseether, Stärkeether, Stärkeester, Polyacrylate, Polyamide, Polyvinylalkohole und Polyvinylpyrrolidon.

Die Benefit-Wirkstoff-haltigen Kapseln können im herstellungsbedingten Rahmen eine beliebige Form aufweisen, sie sind jedoch bevorzugt näherungsweise kugelförmig. Ihr Durchmesser entlang ihrer größten räumlichen Ausdehnung kann je nach den in ihrem Inneren enthaltenen Substanzen und der Anwendung zwischen 10 nm (visuell nicht als Kapsel erkennbar) und 10 mm liegen. Bevorzugt sind sichtbare Mikrokapseln mit einem Durchmesser im Bereich von 0,1 mm bis 7 mm, insbesondere von 0,4 mm bis 5 mm. Nicht mehr mit bloßem Auge wahrnehmbare Kapseln haben vorzugsweise einen Durchmesser im Bereich von 20 bis 500 nm, vorzugsweise 50 bis 200 nm. Besonders bevorzugte erfindungsgemäße Produkte sind dadurch gekennzeichnet, dass die Benefit-Zusammensetzung Kapseln enthält, die einen Durchmesser entlang ihrer größten räumlichen Ausdehnung von 0,01 bis 10.000 µm aufweisen.

Die Kapseln sind nach im Stand der Technik bekannten Verfahren zugänglich, wobei der Koazervation und der Grenzflächenpolymerisation die größte Bedeutung zukommt. Als Kapseln lassen sich sämtliche auf dem Markt angebotenen tensidstabilen Kapseln einsetzen, beispielsweise die Handelsprodukte (in Klammern angegeben ist jeweils das Füllmaterial) Hallcrest Microcapsules (Gelatine, Gummi Arabicum), Coletica Thalaspheres (maritimes Collagen), Lipotec Millicapseln (Alginsäure, Agar-Agar), Induchem Unispheres (Lactose, mikrokristalline Cellulose, Hydroxypropylmethylcellulose), Unicerin C30 (Lactose, mikrokristalline Cellulose, Hydroxypropylmethylcellulose), Kobo Glycospheres (modifizierte Stärke, Fettsäureester, Phospholipide), Softspheres (modifiziertes Agar-Agar) und Kuhs Probiol Nanospheres (Phospholipide).

Die Freisetzung der Substanzen aus den Kapseln erfolgt üblicherweise während der Anwendung der sie enthaltenden Zubereitungen durch Zerstörung der Hülle infolge mechanischer, thermischer, chemischer oder enzymatischer Einwirkung. Vorzugsweise erfolgt die Freisetzung bei den erfindungsgemäßen Produkten durch mechanische Einwirkung, insbesondere durch mechanische Kräfte, denen die Kapseln beim Behandeln der Oberflächen mit dem erfindungsgemäßen Produkt ausgesetzt werden. Aber auch die thermische Einwirkung durch die im Kontakt mit Wasser freigesetzte Wärme kann bevorzugt zur Öffnung der Kapseln beitragen. In einer bevorzugten Ausführungsform der Erfindung enthalten die Produkte gleiche oder verschiedene Kapseln in einer Gesamtmenge von 0,1 bis 60 Gew.-%, insbesondere 0,2 bis 30 Gew.-%, äußerst bevorzugt 0,5 bis 20 Gew.-%, jeweils bezogen auf das Gewicht der Benefit-Zusammensetzung.

Weitere bevorzugte erfindungsgemäße Produkte sind dadurch gekennzeichnet, dass die Benefit-Zusammensetzungen mindestens ein Antioxidans enthalten.

Bevorzugte Antioxidantien sind ausgewählt aus der Gruppe, bestehend aus Imidazol und Imidazolderivate (z. B. Urocaninsäure), D,L-Carnosin, D-Camosin, L-Carnosin und deren Derivaten (z. B. Anserin), Carotinoiden, Carotinen (z. B. α-Carotin, β-Carotin, Lycopin) und deren Derivaten, Liponsäure und deren Derivaten (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), Gallensäure, Gallenextrakten, Gallussäureestern (z. B. Propyl-, Octyl- und Dodecylgallat), 6,7-disubstituierten 2,2-Dialkylchromanen oder -chromenen, insbesondere 4-Dihydro-7-methoxy-2,2-dimethyl-2H-1-benzopyran-6-ol, Flavonoiden, Catechinen, Bilirubin, Biliverdin, und deren Derivaten, Folsäure und deren Derivaten. Hydrochinon und dessen Derivaten (7 R Arbutin), Ubichinon und Ubichinol sowie deren Derivaten, Vitamin C und dessen Derivaten (z. B. Ascorbylpalmitat, -stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivaten, Tocopherolen und deren Derivaten (z. B. Tocopherylacetat, -linoleat, -oleat und -succinat, Tocophereth-5, Tocophereth-10, Tocophereth-12, Tocophereth-18, Tocophereth-50, Tocophersolan), Tocotrienolen und deren Derivaten, insbesondere den Tocotrienolestern, Vitamin A und Derivaten, insbesondere den Estern, z. B. Vitamin-A-Palmitat, dem Coniferylbenzoat des Benzoeharzes, Rutin, Rutinsäure und deren Derivaten, Dinatriumrutinyldisulfat, Kojisäure, Chitosanglycolat und -salicylat, Butylhydroxytoluol, Butylhydroxyanisol, Nordihydroguajakharzsäure, Nordihydroguajaretsäure und Trihydroxybutyrophenon.
Die Gesamtmenge des mindestens einen Antioxidans in den efindungsgemäßen Produkten beträgt bevorzugt 0,001 - 10 Gew.-%, besonders bevorzugt 0,05 - 5 Gew.-% und außerordentlich bevorzugt 0,1 - 2 Gew.-%, jeweils bezogen auf das Gesamtgewicht der Benefit-Zusammensetzung.

Weitere erfindungsgemäß bevorzugte Produkte sind dadurch gekennzeichnet, dass die Benefit-Zusammensetzung mindestens einen Blondier- oder Bleichwirkstoff enthält. Eine wichtige Blondier- oder Bleichkomponente ist Wasserstoffperoxid. Da die erfindungsgemäßen Benefit-Zusammensetzungen wasserfrei sein sollten, kann das Wasserstoffperoxid erfindungsgemäß nur in Form einer festen Anlagerungsverbindung von Wasserstoffperoxid an anorganische oder organische Verbindungen, wie beispielsweise Natriumperborattetrahydrat, Natriumperboratmonohydrat, Natriumpercarbonat, Magnesiumpercarbonat, Natriumpercarbamid, Polyvinylpyrrolidon · n H₂O₂ (n ist eine positive ganze Zahl größer 0), Harnstoffperoxid und Melaminperoxid, eingesetzt werden.

Das mindestens eine Wasserstoffperoxid-Anlagerungsprodukt ist bevorzugt in einer Gesamtmenge von 0,1 - 12 Gew.-%, besonders bevorzugt in 1 - 6 Gew.-%, jeweils bezogen auf das Gewicht der Benefit-Zusammensetzung, enthalten.

Derartige erfindungsgemäß bevorzugte Produkte können nach dem Anfeuchten mit Wasser zur Haarblondierung eingesetzt werden. Die Auftragung mit dem Substrat ermöglicht dabei vor allem das Blondieren einzelner Haarsträhnen.

In einer weiteren bevorzugten erfindungsgemäßen Ausführung als Blondierprodukt kann die Wirkung des Wasserstoffperoxid-Anlagerungsprodukts durch einen sogenannten Bleich-Booster weiter gesteigert werden. Dies sind in der Regel feste Peroxoverbindungen, die keine Anlagerungsprodukte von Wasserstoffperoxid an andere Komponenten darstellen. Die Auswahl der in den erfindungsgemäßen Mitteln enthaltenen Peroxoverbindungen unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat und Peroxide wie Magnesium- und Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Bleich-Booster-Peroxoverbindungen sind in den erfindungsgemäßen Blondierprodukten bevorzugt in Gesamtmengen von 1 - 40 Gew.-%, insbesondere 2-30 Gew.-%, bezogen auf das Gewicht der Benefit-Zusammensetzung, enthalten.

In einer weiteren bevorzugten Ausführungsform der erfindungsgemäßen Produkte Reinigungsmittel enthält die Benefit-Zusammensetzung mindestens ein Enzym. Das Enzym kann in verkapselter Form und/oder direkt in der Benefit-Zusammensetzung enthalten sein. Als Enzyme sind insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen, Hemicellulase, Cutinasen, β-Glucanasen, Oxidasen, Peroxidasen, Perhydrolasen und/oder Laccasen und Gemische der genannten Enzyme bevorzugt. Zur Bleiche bzw. zur Hemmung der Farbübertragung können auch Oxireduktasen eingesetzt werden. Als Oxidationsmittel in oxidativen Haarfärbemitteln sind bevorzugt Laccasen geeignet. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen, Iso-Amylasen, Pullulanasen und Pektinasen. Als Cellulasen werden vorzugsweise Cellobiohydrolasen, Endoglucanasen und beta-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert sein, um sie gegen vorzeitige Zersetzung zu schützen. Der Anteil der Enzyme in der Benefit-Zusammensetzung beträgt bevorzugt 0,01 - 5 Gew.-%, besonders bevorzugt 0,12 - 2,5 Gew.-%, bezogen auf die Benefit-Zusammensetzung.

Es kann, beispielsweise bei speziellen erfindungsgemäßen Produkten für Konsumenten mit Allergien, aber auch bevorzugt sein, dass das erfindungsgemäße Produkt keine Enzyme enthält.

In einer weiteren bevorzugten erfindungsgemäßen Ausführungsform enthält die Benefit-Zusammensetzung mindestens ein Farbstoffvorprodukt und/oder mindestens einen Farbstoff zur Färbung keratinischer Fasern, insbesondere zur Färbung menschlicher Haare. Als Farbstoff(vorprodukt)e können
- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate, sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin..

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind
- m-Aminophenol und dessen Derivate wie beispielsweise 5-Amino-2-methylphenol, 5-(3-Hydroxy-propylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diamino-phenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Volet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere alpha-Aminocarbonsäuren und omega-Aminocarbonsäuren. Unter den alpha-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die als Färbemittel verwendeten bevorzugten Benefit-Zusammensetzungen mindestens ein wasserfreies Alkalisierungsmittel, üblicherweise Alkali- oder Erdalkalihydroxide oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von omega-Aminosäuren wie omega-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder die Anlagerungsprodukte von Wasserstoffperoxid an Harnstoff, Melamin oder Natriumborat verwendet werden.

Besonders bevorzugt kann hierbei der Wassergehalt einer Wasserstoffperoxid-Lösung gleichzeitig zur Wärme freisetzenden Aktivierung der Farbstoff(vorprodukt)-haltigen Benefit-Zusammensetzung verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Färbemittel, das gegebenenfalls außer der Benefit-Zusammensetzung die Wasserstoffperoxid-Lösung und gegebenenfalls weiteres Wasser umfasst) Wasserstoffperoxid durch Peroxidasen zu verstärken.

Zweckmäßigerweise wird die Zubereitung des Oxidationsmittels unmittelbar vor dem Färben der Haare mit der Benefit-Zusammenssetzung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die durch die freigesetzte Hydratationswärme erhöhte Anwendungstemperatur bewirkt hervorragende Färbeergebnisse. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn eine stark tensidhaltige Benefit-Zusammensetzung verwendet wurde. Insbesondere bei schwer färbbarem Haar kann die Benefit-Zusammensetzung mit den Farbstoffvorprodukten ohne vorherige Vermischung mit der Oxidationskomponente auf das Haar aufgebracht werden. Falls die Benefit-Zusammensetzung mit den Farbstoffvorprodukten pulverförmig vorliegt, muss sie mit einem Lösemittel gebrauchsfertig angefeuchtet werden. Dies kann bevorzugt Wasser sein, wobei die so erwärmte Farbstoffvorproduktzusammensetzung hervorragend auf das Haar aufzieht. Nach einer Einwirkdauer von 20 bis 30 Minuten wird dann - gegebenenfalls nach einer Zwischenspülung - die Oxidationskomponente aufgebracht. Auch die Oxidationskomponente kann in Form eines weiteren erfindungsgemäßen Produktes aufgebracht werden, nachdem dieses zuvor mit Wasser befeuchtet wurde. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Zubereitungen als Oxidationsmittel bevorzugt sein.

Weiterhin kann die Ausbildung der Färbung dadurch unterstützt und gesteigert werden, dass der färbenden Benefit-Zusammensetzung bestimmte Metallionen zugesetzt werden. Solche Metallionen sind bevorzugt Zn²⁺, Cu²⁺, Fe²⁺, Fe³⁺, Mn²⁺, Mn⁴⁺, Li⁺, Mg²⁺, Ca²⁺ und Al³⁺. Besonders bevorzugt sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Besonders bevorzugte erfindungsgemäße Ausführungsformen sind dadurch gekennzeichnet, dass die Benefit-Zusammensetzung nicht gleichzeitig Oxidationsmittel und (davon verschiedene) Reduktionsmittel enthält.

In einer bevorzugten Ausführungsform enthält das erfindungsgemäße Reinigungs-, Pflege-, Wasch- oder Konditionierprodukt mindestens einen Komplexbildner.

Komplexbildner (INCl Chelating Agents), auch Sequestriermittel genannt, sind Inhaltsstoffe, die Metallionen zu komplexieren und inaktivieren vermögen, beispielsweise um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert die oxidative Zersetzung der fertigen Benefit-Zusammensetzung.

Geeignet sind beispielsweise die folgenden gemäß INCI bezeichneten Komplexbildner, die beispielsweise im International Cosmetic Ingredient Dictionary and Handbook näher beschrieben sind: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphos-phate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

Bevorzugte Komplexbildner sind tertiäre Amine, insbesondere tertiäre Alkanolamine (Aminoalkohole). Die Alkanolamine besitzen sowohl Amino- als auch Hydroxy- und/oder Ethergruppen als funktionelle Gruppen. Besonders bevorzugte tertiäre Alkanolamine sind Tri-ethanolamin und Tetra-2-hydroxypropylethylendiamin (N,N,N',N'-Tetrakis-(2-hydroxy-pro-pyl)ethylendiamin). Besonders bevorzugte Kombinationen tertiärer Amine mit Zinkricinoleat und einem oder mehreren ethoxylierten Fettalkoholen als nichtionische Lösungsvermittler sowie ggf. Lösungsmittel sind im Stand der Technik beschrieben.

Ein besonders bevorzugter Komplexbildner ist die Etidronsäure (1-Hydroxyethyliden-1,1-diphosphonsäure, 1-Hydroxyethyan-1,1-diphosphonsäure, HEDP, Acetophosphonsäure, INCI Etidronic Acid) einschließlich ihrer Salze. In einer bevorzugten Ausführungsform enthält die erfindungsgemäße Benefit-Zusammensetzung demgemäß als Komplexbildner Etidronsäure und/oder eines oder mehrere ihrer Salze.

In einer besonderen Ausführungsform enthält die erfindungsgemäße Benefit-Zusammensetzung eine Komplexbildnerkombination aus einem oder mehreren tertiären Aminen und einer oder mehreren weiteren Komblexbildnem, vorzugsweise einer oder mehreren Komplexbildnersäuren oder deren Salzen, insbesondere aus Triethanolamin und/oder Tetra-2-hydroxypropylethylendiamin und Etidronsäure und/oder einem oder mehrerer ihrer Salze.

Bevorzugte erfindungsgemäße Reinigungs-, Pflege-, Wasch- oder Konditionierprodukte enthalten mindestens einen Komplexbildner in einer Gesamtmenge von 0,1 - 20 Gew.-%, vorzugsweise 0,2 - 15 Gew.-%, insbesondere 0,5 - 10 Gew.-%, besonders bevorzugt 1 - 8 Gew.-%, äußerst bevorzugt 1,5 bis 6 Gew.-%, bezogen auf die gesamte Benefit-Zusammensetzung.

In einer weiteren bevorzugten Ausführungsform der Erfindung kann die Wirkung der erfindungsgemäßen Produkte durch Fettstoffe weiter optimiert werden. Bevorzugte Fettstoffe sind insbesondere:
- pflanzliche Öle, wie Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls,
- flüssige Paraffinöle, Isoparaffinöle und synthetische Kohlenwasserstoffe, z. B. 1,3-Di-(2-ethylhexyl)-cyclohexan (Cetiol^{®} S) oder Polydecene,
- Di-n-alkylether mit insgesamt 12 bis 36, insbesondere 12 bis 24 C-Atomen, z. B. Di-n-octylether (Cetiol^{®} OE), Di-n- n-Hexyl-n-octylether und n-Octyl-n-decylether,
- Fettsäuren, besonders lineare und/oder verzweigte, gesättigte und/oder ungesättigte C₈₋₃₀-Fettsäuren. Bevorzugt sind C₁₀₋₂₂-Fettsäuren. Beispiele sind die Isostearinsäuren und Isopalmitinsäuren wie die unter der Handelsbezeichnung Edenor^{®} vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt sind üblicherweise die Fettsäureschnitte, die aus Kokosöl oder Palmöl erhältlich sind; insbesondere bevorzugt ist der Einsatz von Stearinsäure.
- Fettalkohole, besonders gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z. B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, z. B. 2-Ethylhexanol, wobei diese Aufzählung beispielhaften und nicht limitierenden Charakter haben soll.
- Esteröle, das heißt, Ester von C₆₋₃₀-Fettsäuren mit C₂₋₃₀-Fettalkoholen. Bevorzugt sind die Monoester der Fettsäuren mit Alkoholen mit 2 bis 24 C-Atomen. Als Alkohol- und Säurekomponenten der Esteröle können die vorstehend genannten Substanzen verwendet werden. Erfindungsgemäß besonders bevorzugt sind Isopropylmyristat, Isononansäure-C₁₆₋₁₈-alkylester, 2-Ethylhexylpalmitat, Stearinsäure-2-ethylhexylester, Cetyloleat, Glycerintricaprylat, Kokosfettalkoholcaprinat/-caprylat, n-Butylstearat, Oleylerucat, Isopropylpalmitat, Oleyloleat, Hexyllaurat, Di-n-butyladipat, Myristylmyristat und Ölsäuredecylester.
- Hydroxycarbonsäurealkylester, wobei die Vollester der Glycolsäure, Milchsäure, Äpfelsäure, Weinsäure oder Citronensäure bevorzugt sind, aber auch Ester der β-Hydroxypropionsäure, der Tartronsäure, der D-Gluconsäure, Zuckersäure, Schleimsäure oder Glucuronsäure geeignet sind und besonders bevorzugt die Ester von C₁₂-C₁₅-Fettalkoholen, z. B. die Handelsprodukte Cosmacol^{®} der EniChem, Augusta Industriale, sind,
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglycoldioleat, Ethylenglycol-di-isotridecanoat, Propylenglycoldi(2-ethylhexanoat), Propylenglycol-di-isostearat, Propylenglycol-di-pelargonat, Butandiol-di-isostearat, Neopentylglycoldicaprylat,
- symmetrische, unsymmetrische oder cyclische Ester der Kohlensäure mit Fettalkoholen, z. B.
   Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, z. B. Monomuls^{®} 90-018, Monomuls^{®} 90-L12 oder Cutina^{®} MD,
- Wachse, insbesondere Insektenwachse wie Bienenwachs und Hummelwachs, Pflanzenwachse wie Candelillawachs und Carnaubawachs, Fruchtwachse, Ozokerit, Mikrowachs, Ceresin, Paraffin, Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, synthetische Vollester aus Fettsäuren und Glycolen (z. B. Syncrowachs^{®}) oder Polyolen mit 2 - 6 C-Atomen, Ester von gegebenenfalls hydroxylierten C₂₋₄-Carbonsäuren mit Lanolinalkoholen und C₁₂₋₁₈-Fettalkoholen, Cholesterol- oder Lanosterolester von C₁₀₋₃₀-Fettsäuren und ethoxylierten C₁₂₋₂₀-Fettsäureglycolestern, Fettsäuremonoalkanolamiden mit einem C₁₂₋₂₂-Acylrest und einem C₂₋₄-Alkanolrest, synthetischen Fettsäure-Fettalkoholestern, z. B. Stearylstearat oder Cetylpalmitat, Esterwachse aus natürlichen Fettsäuren und synthetischen C₂₀₋₄₀-Fettalkoholen (INCl-Bezeichnung C20-40 Alkyl Stearate), Ozokerit und Paraffin,
- Siliconverbindungen, bevorzugt ausgewählt aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane, bevorzugt die Substanzen mit der INCI-Bezeichnung Dimethicone Copolyol, sowie Polydialkylsiloxane, die Amin- und/oder Hydroxy-Gruppen enthalten.

Bevorzugte erfindungsgemäße Produkte sind dadurch gekennzeichnet, dass die Benefit-Zusammensetzung mindestens eine Siliconverbindung in einer Gesamtmenge von 0,05 bis 5 Gew.-%, vorzugsweise von 0,2 bis 3 Gew.-%, jeweils bezogen auf die Benefit-Zusammensetzung, enthält.

Insbesondere bevorzugt werden die Siliconverbindungen ausgewählt aus mindestens einem Vertreter aus der Liste, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter;
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silicon polymeren mit nicht Siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Silicon polymeren mit Polysiloxan-Grundgerüst, auf das nicht-Siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Besonders bevorzugte erfindungsgemäße Produkte sind dadurch gekennzeichnet, dass die Benefit-Zusammensetzungen mindestens ein Silicon der Formel (Si-1)

(CH₃)₃Si-[O-Si(CH₃)₂]ₓ-O-Si(CH₃)₃ (Si-1),

enthalten, in der x für eine Zahl von 0 bis 100, vorzugsweise von 1 bis 50, weiter bevorzugt von 2 bis 20 und insbesondere 3 bis 10, steht.

Die Silicone der Formel (Si-1) werden nach der INCI-Nomenklatur als Dimethicone bezeichnet. Es werden im Rahmen der vorliegenden Erfindung als Silicon der Formel (Si-1) vorzugsweise die Verbindungen:

(CH₃)₃Si-O-Si(CH₃)₃

(CH₃)₃Si-O-(CH₃)₂Si-O-Si(CH₃)₃

(CH₃)₃Si-[O-(CH₃)₂Si]₂-O-Si(CH₃)₃

(CH₃)₃Si-[O-(CH₃)₂Si]₃-O-Si(CH₃)₃

(CH₃)₃Si-[O-(CH₃)₂Si]₄-O-Si(CH₃)₃

(CH₃)₃Si-[O-(CH₃)₂Si]₅-O-Si(CH₃)₃

(CH₃)₃Si-[O-(CH₃)₂Si]₆-O-Si(CH₃)₃

(CH₃)₃Si-[O-(CH₃)₂Si]₇-O-Si(CH₃)₃

(CH₃)₃Si-[O-(CH₃)₂Si]₈-O-Si(CH₃)₃

(CH₃)₃Si-[O-(CH₃)₂Si]₉-O-Si(CH₃)₃

(CH₃)₃Si-[O-(CH₃)₂Si]₁₀-O-Si(CH₃)₃

(CH₃)₃Si-[O-(CH₃)₂Si]₁₁-O-Si(CH₃)₃

(CH₃)₃Si-[O-(CH₃)₂Si]₁₂-O-Si(CH₃)₃

(CH₃)₃Si-[O-(CH₃)₂Si]₁₃-O-Si(CH₃)₃

(CH₃)₃Si-[O-(CH₃)₂Si]₁₄-O-Si(CH₃)₃

(CH₃)₃Si-[O-(CH₃)₂Si]₁₅-O-Si(CH₃)₃

(CH₃)₃Si-[O-(CH₃)₂Si]₁₆-O-Si(CH₃)₃

(CH₃)₃Si-[O-(CH₃)₂Si]₁₇-O-Si(CH₃)₃

(CH₃)₃Si-[O-(CH₃)₂Si]₁₈-O-Si(CH₃)₃

(CH₃)₃Si-[O-(CH₃)₂Si]₁₉-O-Si(CH₃)₃

(CH₃)₃Si-[O-(CH₃)₂Si]₂₀-O-Si(CH₃)₃

eingesetzt, wobei (CH₃)₃Si-O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silicone in den bevorzugten erfindungsgemäßen Benefit-Zusammensetzungen enthalten sein.

Bevorzugte erfindungsgemäß einsetzbare Silicone weisen bei 20°C Viskositäten von 0,2 bis 2 mm²s⁻¹ auf, wobei Silicone mit Viskositäten von 0,5 bis 1 mm²s⁻¹ besonders bevorzugt sind. Besonders bevorzugte erfindungsgemäße Produkte enthalten ein oder mehrere aminofunktionelle Silicone. Solche Silicone können z.B. durch die Formel (Si-2)

M(RₐQ_{b}SiO_{(4-a-b)/2})ₓ(R_{c}SiO_{(4-c)/2})_{y}M (Si-2)

beschrieben werden, wobei in der obigen Formel
- R: ein Kohlenwasserstoff oder ein Kohlenwasserstoffrest mit 1 bis 6 Kohlenstoffatomen ist,
- Q: ein polarer Rest der allgemeinen Formel -R¹HZ ist, worin
R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
Z ein organischer, aminofunktioneller Rest ist, der mindestens eine aminofunktionelle Gruppe enthält;
a Werte im Bereich von etwa 0 bis etwa 2 annimmt,
b Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und

- M: eine geeignete Silicon-Endgruppe ist, wie sie im Stande der Technik bekannt ist, vorzugsweise Trimethylsiloxy.

Nicht einschränkende Beispiele der in Formel (Si-2) durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄- und -(CH₂)₃C(O)SCH₂CH₂- ein.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)_{z}(CH₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH₂NH₂-Rest. Eine andere mögliche Formel für besagtes Z ist -N(CH₂)_{z}CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel -CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Das molare Verhältnis der RₐQ_{b} SiO_{(4-a-b)/2}-Einheiten zu den R_{c}SiO_{(4-c)/2}-Einheiten in Formel (Si-2) liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel (Si-2) eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Benefit-Zusammensetzungen enthalten ein aminofunktionelles Silicon der Formel (Si-3)

R'ₐG₃₋ₐ-Si(OSiG₂)ₙ-(OSiG_{b}R'_{2-b)m}-O-SiG₃₋ₐ-R'ₐ (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, CH(CH₃)CH₂CH₃, -O-C(CH₃)₃, -C(CH₃)₃;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus -Q-N(R")-CH₂-CH₂-N(R")₂
-Q-N(R")₂
-Q-N⁺(R")₃A⁻
-Q-N⁺H(R")₂ A⁻
-Q-N⁺H₂(R")A⁻
-Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht, R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Erfindungsgemäß ebenfalls bevorzugt sind kationische Siliconöle wie beispielsweise ein hydroxylaminomodifiziertes Silicon, das als Amodimethicone bezeichnet wird, DC 2-2078 (Hersteller Dow Corning, INCI-Bezeichung: Aminopropyl Phenyl Trimethicone), DC 5-7113 (Hersteller Dow Corning, INCI-Bezeichung:
Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).
Besonders bevorzugte erfindungsgemäße Benefit-Zusammensetzungen sind dadurch gekennzeichnet,
dass sie mindestens ein aminofunktionelles Silicon der Formel (Si3-a) 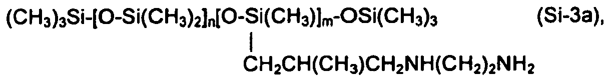
enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt. Diese Silicone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Benefit-Zusammensetzungen, die mindestens ein aminofunktionelles Silicon der Formel (Si-3b) 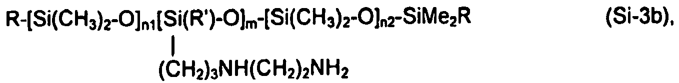 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet.

Unabhängig davon, welche aminofunktionellen Silicone eingesetzt werden, sind erfindungsgemäß solche Benefit-Zusammensetzungen bevorzugt, die ein aminofunktionelles Silicon enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden. Erfindungsgemäß bevorzugte Benefit-Zusammensetzungen sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silicon(e) enthalten.

Auch die nach INCI als Cyclomethicone bezeichneten cyclischen Dimethicone sind erfindungsgemäß mit Vorzug einsetzbar. Hier sind erfindungsgemäße Benefit-Zusammensetzungen bevorzugt, die mindestens ein Silicon der Formel (Si-4) 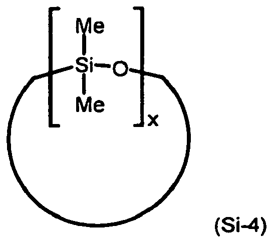 enthalten, in der x für eine Zahl von 4 bis 200, vorzugsweise von 5 bis 10, weiter bevorzugt von 6 bis 9 und insbesondere 7 oder 8, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich. Zusammenfassend umfasst dieses Herstellungsverfahren die emulgierende Mischung von Komponenten, deren eine mindestens ein Polysiloxane enthält, deren andere mindestens ein Organo-Siliconmaterial enthält, das mit dem Polysiloxane in einer Kettenverlängerungsreaktion reagiert, wobei mindestens ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)p-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, dass durchschnittlich ein bis zwei reaktive Gruppen pro Polymer vorliegen, n ist eine ganze Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, die Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), bzw. sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Si-haltige Gruppen, O-haltige Gruppen usw.. Vorzugsweise sind mindestens 80% der Reste R Alkylreste, besonders bevorzugt Methylgruppen.

Das Organosiliconmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosiliconmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosiliconmaterial ein Kettenverlängerungs-Agens umfasst, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfasst, welches mindestens eine Si-OH Gruppe aufweist, ketteverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem OrganoSiliconmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das OrganoSiliconmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist.

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosiliconmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosiliconsystemen und der geringen Farbveränderung bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosiliconmaterial zur Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinndilaurat, Dibutylzinn-diacetat, Dimethylzinn-dineodecanoat, Dibutylzinn-dimethoxid, Isobutylzinntriceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitanat, Tetraphenyltitanat, Tetraoctadecyltitanat, Titannaphthanat, Ethyltriethanolamin-Titanat, Titan-diisopropyl-diethyl-acetoacetat, Titan-diisopropoxy-diacetyl-acetonat und Titantetra-Alkoxide, bei denen das Alkoxid Butoxy oder Propoxy ist.

Erfindungsgemäß ebenfalls bevorzugte Benefit-Zusammensetzungen sind dadurch gekennzeichnet, dass sie mindestens ein Silicon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silicone wasserlöslich. Erfindungsgemäß bevorzugte Mittel der Ausführungsform mit einem Silicon sind dadurch gekennzeichnet, dass das Silicon wasserlöslich ist.

Entsprechende hydrophile Silicone werden beispielsweise aus den Verbindungen der Formeln (Si-6) und/oder (Si-7) ausgewählt. Insbesondere bevorzugte wasserlösliche Tenside auf Siliconbasis sind ausgewählt aus der Gruppe der Dimethiconecopolyole die bevorzugt alkoxyliert, insbesondere polyethoxyliert oder polypropoxyliert sind.

Unter Dimethiconecopolyolen werden erfindungsgemäß bevorzugt Polyoxyalkylen-modifizierte Dimethylpolysiloxane der allgemeinen Formeln (Si-6) oder (Si-7) verstanden: 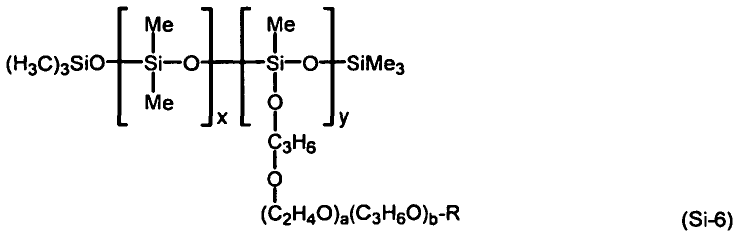 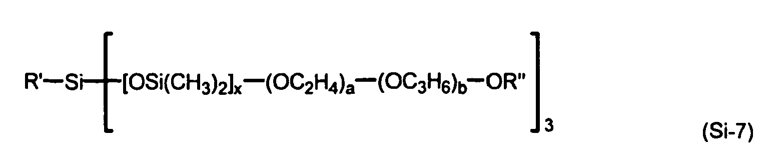 worin
- der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation), Abil (Goldschmidt) und DOW CORNING (Dow) vertriebenen Produkte.

Erfindungsgemäß besonders bevorzugte Dimethiconcopolyole sind Dow Corning 190, Dow Corning 193 (Dow), Abil EM 97 und Abil EM 90.

Bevorzugte erfindungsgemäße Produkte sind dadurch gekennzeichnet, dass die Benefit-Zusammensetzung mindestens einen Fettstoff in einer Gesamtmenge von 0,1-50 Gew.%, bevorzugt 0,5 - 20 Gew.% und besonders bevorzugt 1,0 - 10 Gew.%, jeweils bezogen auf die Benefit-Zusammensetzung, enthält.

Antimikrobielle Wirkstoffe bzw. Konservierungsmittel unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat.

Erfindungsgemäß bevorzugte geruchskomplexierende Substanzen sind ausgewählt aus Silicaten, Zinkricinoleat, Cyclodextrinen, Aluminiumoxid sowie Chlorophyll. Zu den erfindungsgemäß besonders bevorzugten Silicaten zählen vor allem Schichtsilicate und unter diesen insbesondere Montmorillonit, Kaolinit, Illit, Beidellit, Nontronit, Saponit, Hectorit, Bentonit und Smectit. Bevorzugte erfindungsgemäße Produkte sind dadurch gekennzeichnet, dass die Benefit-Zusammensetzung mindestens eine geruchskomplexierende Substanz in einer Menge von 0,1 - 10 Gew.%, vorzugsweise 0,5 - 7 Gew.% und insbesondere 1-5 Gew.%, jeweils bezogen auf das Gewicht der Benefit-Zusammensetzung, enthält. Weitere erfindungsgemäß bevorzugte Produkte sind dadurch gekennzeichnet, dass die Benefit-Zusammensetzung zusätzlich zu den mindestens 1 Gew.-% mindestens einer unter Normalbedingungen festen Substanz, die sich in Wasser mit negativer Lösungsenthalpie löst oder in Kontakt mit Wasser Hydratationswärme freisetzt, und dem mindestens einen Wirkstoff, ausgewählt aus Tensiden, kosmetischen Wirkstoffen und dermatologischen Wirkstoffen, antibakteriellen Stoffen, Antioxidantien, Blondier- oder Bleichwirkstoffen, Bügelhilfsmitteln, Enzymen, Farbstoff(vorprodukt)en, Fettstoffen, Fungiziden, Germiziden, geruchskomplexierenden Substanzen, Hydrophobiermitteln, Riechstoffen, UV-Schutz-Substanzen und/oder Weichmachenden Komponenten, weiterhin mindestens einen Hilfsstoff enthält, der ausgewählt ist aus Acidifizierungsmitteln, Alkalisierungsmitteln, Antiredepositionsmitteln, Antistatika, Bleichaktivatoren, Bleich-Boostern, Bleichkatalysatoren, Buildersubstanzen, Cobuildern, Dispergiermitteln, Einlaufverhinderern, Farbschutzstoffen, Klarspülern, Knitterschutzmitteln, Komplexbildnern, Konservierungsmitteln, Korrosionsinhibitoren, optischen Aufhellern, Parfümträgern, Quell- und Schiebefestmitteln, Schauminhibitoren, schmutzabweisenden Stoffen, Silberschutzmitteln, Verdickungsmitteln, Verfärbungsinhibitoren und/oder Vergrauungsinhibitoren.

Da textile Flächengebilde, insbesondere aus Rayon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können weitere bevorzugte erfindungsgemäße Produkte, die als Textilpflegemittel ausgebildet sind, mindestens ein synthetisches Knitterschutzmittel enthalten. Hierzu zählen bevorzugt synthetische Produkte auf der Basis von Fettsäuren, Fettsäureestern, Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

In einer weiteren bevorzugten Ausführungsform der Erfindung enthält die erfindungsgemäße Benefit-Zusammensetzung als Hilfsstoff mindestens ein Verdickungsmittel, besonders bevorzugt mindestens ein organisches synthetisches polymeres Verdickungsmittel.

In einer bevorzugten Ausführungsform der erfindungsgemäßen Lehre kann die Wirkung mit Polymeren weiter gesteigert werden. Unter Polymeren sind sowohl natürliche als auch synthetische Polymere, welche anionisch, kationisch, amphoter geladen oder nichtionisch sein können, zu verstehen.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (POL-III), 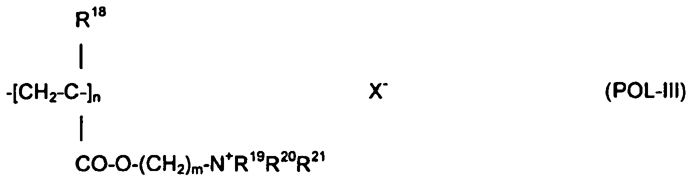 in der R¹⁸ = -H oder -CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (POL-111) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxy-ethyltrimethylammoniumchlorid) mit der INCl-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglycols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß US 5773595,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quatemierter Polyvinylalkohol,
- sowie die unter den Bezeichnungen
   - Polyquaternium 2,
   - Polyquaternium 17,
   - Polyquaternium 18 und
   - Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß bevorzugt sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Besonders bevorzugte Chitosane weisen einen Deacetylierungsgrad von wenigstens 80 %, bevorzugt wenigstens 90 %, und eine relative Molmasse von 5 10⁵ bis 5 10⁶ (g/mol) auf.

Zur Herstellung erfindungsgemäßer Zubereitungen muß das Chitosan in die Salzform überführt werden. Dies kann durch Auflösen in verdünnten wässrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet. Bei den anionischen Polymeren, welche die Wirkung des erfindungsgemäßen Wirkstoffes unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder CoMonomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Weiterhin hat sich überraschend gezeigt, dass die Stabilitätseigenschaften und/oder die sensorischen Eigenschaften der erfindungsgemäßen Zusammensetzungen weiter verbessert werden konnten durch den Zusatz mindestens eines polymeren Verdickers in Form eines verzweigten oder vernetzten Polyelektrolyten, wobei der Polyelektrolyt ausgewählt ist aus einem Copolymer aus mindestens zwei Monomer-Typen, wobei mindestens ein Monomer eine starke Säurefunktion enthält, die teilweise oder vollständig neutralisiert ist, und mindestens ein Monomer entweder neutral ist oder eine schwache Säurefunktion enthält. Eine weitere bevorzugte Ausführungsform der Erfindung ist dadurch gekennzeichnet, dass mindestens ein polymerer Verdicker in Form eines verzweigten oder vernetzten Polyelektrolyten enthalten ist, wobei der Polyelektrolyt ausgewählt ist aus einem Copolymer aus mindestens zwei Monomer-Typen, wobei mindestens ein Monomer eine starke Säurefunktion enthält, die teilweise oder vollständig neutralisiert ist, und mindestens ein Monomer entweder neutral ist oder eine schwache Säurefunktion enthält.

In einer bevorzugten Ausführungsform der Erfindung sind die Polyelektrolytmonomeren, die eine schwache Säurefunktion enthalten, ausgewählt aus den Monomeren, die eine Carboxylgruppe -COOH enthalten, die teilweise oder vollständig neutralisiert ist. Besonders bevorzugte Beispiele für solche schwach sauren Polyelektrolytmonomeren sind Acrylsäure, Methacrylsäure, Itaconsäure und Maleinsäure.

In einer weiteren bevorzugten Ausführungsform der vorliegenden Erfindung sind die Polyelektrolytmonomere mit einer starken Säuregruppe ausgewählt aus Monomeren, die mit einer Sulfonsäuregruppe -SO₃H oder einer Phosphonsäuregruppe funktionalisiert sind. Ein besonders bevorzugtes Polyelektrolytmonomer mit starker Säurefunktion ist 2-Methyl-2[(1-oxo-2-propenyl)amino]-1-propansulfonsäure, die teilweise oder vollständig neutralisiert ist.

Weitere erfindungsgemäß besonders bevorzugte verdickende Polymere sind teilweise oder vollstandig neutralisierte, vernetzte Copolymere aus 2-Methyl-2[(1-oxo-2-propenyl)amino]-1-propansulfonsäure (AMPS) und Hydroxyethylacrylat. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Hydroxyethylacrylat.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Acrylsäure. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Acrylsäure.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Acrylamid. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Acrylamid.

Eine weitere bevorzugte Ausführungsform der Erfindung ist dadurch gekennzeichnet, dass die Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Satz vorliegen. Besonders bevorzugt sind die Natriumsalze und die Ammoniumsalze.

Eine weitere bevorzugte Ausführungsform der Erfindung ist dadurch gekennzeichnet, dass das neutrale Polyelektrolytmonomer ausgewählt ist aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der vorstehend genannten Ester mit einem Molekulargewicht zwischen 400 und 1000 g/mol, Acrylamid sowie Vinylpyrrolidon. Erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Vinylpyrrolidon. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Vinylpyrrolidon, die in Form des Ammoniumsalzes kommerziell erhältlich sind, z. B. unter dem Handelsnamen Aristoflex^{®} AVC von der Firma Clariant.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vemetzungsagentien sein. Solche Verbindungen sind beispielsweise unter dem Warenzeichen Carbopol^{®} im Handel erhältlich. Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere zur Steigerung der Wirkung der erfindungsgemäßen Lehre amphotere Polymere als Bestandteil eingesetzt. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefasst, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus
(a) Monomeren mit quartären Ammoniumgruppen der allgemeinen Formel (IV),

   R²²-CH=CR²³-CO-Z-(CₙH₂ₙ)-N⁽⁺⁾R²⁴R²⁵R²⁶ A⁽⁻⁾ (IV)

   in der R²² und R²³ unabhängig voneinander stehen für Wasserstoff oder eine Methylgruppe und R²⁴, R²⁵ und R²⁶ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (V),

   R²⁷-CH=CR²⁸-COOH (V)

   in denen R²⁷ und R²⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R²⁴, R²⁵ und R²⁶ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Produkte können in einer weiteren bevorzugten Variante weiterhin mindestens ein nichtionogenes Polymer als Hilfsstoff, insbesondere als Verdickungsmittel, aber auch als Vergrauungsinhibitor, Dispergiermittel oder Cobuilder, enthalten.

Bevorzugte nichtionogene Polymere sind beispielsweise:
VinylpyrrolidonNinylester-Copolymere, wie sie beispielsweise unter dem Warenzeichen Luviskol^{®} (BASF) vertrieben werden. Luviskol^{®} VA 64 und Luviskol^{®} VA 73, jeweils VinylpyrrolidonNinylacetat-Copolymere,
sind ebenfalls bevorzugte nichtionische Polymere; Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden; Schellack; Polyvinylpyrrolidone, wie sie beispielsweise unter der Bezeichnung Luviskol^{®} (BASF) vertrieben werden.

Es kann erfindungsgemäß besonders bevorzugt sein, dass die erfindungsgemäßen Benefit-Zusammensetzungen mehrere, insbesondere zwei verschiedene Polymere gleicher Ladung und/oder jeweils ein ionisches und ein amphoteres und/oder nicht ionisches Polymer enthalten.

In einer weiteren erfindungsgemäß bevorzugten Ausführungsform wird die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an Tensid in einem Gewichtsverhältnis zueinander von 0,1 - 100, bevorzugt 0,2 - 50, besonders bevorzugt 0,5 - 20 und außerordentlich bevorzugt 1-10, ebenfalls außerordentlich bevorzugt 1,5-3 eingesetzt, um eine optimale Wärmeentwicklung in Verbindung mit einer optimalen Benefit-Leistung, insbesondere einer optimalen Wasch- und Reinigungsleistung und gegebenenfalls einer optimalen Benefit-Leistung weiterer enthaltener Wirkstoffe, der erfindungsgemäßen Produkte zu gewährleisten.

In einer weiteren erfindungsgemäß bevorzugten Ausführungsform wird die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an kosmetischen oder dermatologischen Wirkstoffen in einem Gewichtsverhältnis zueinander von 1 - 1000, bevorzugt 2 - 500, besonders bevorzugt 5-100 und außerordentlich bevorzugt 10-50, ebenfalls außerordentlich bevorzugt 15 - 30 eingesetzt, um eine optimale Wärmeentwicklung in Verbindung mit einer optimalen Benefit-Leistung, insbesondere einer optimalen Antifalten-, Antiageing-, Antioxidans-, Hautbefeuchtungs-, Hautbräunungs-, Hautaufhellungs-, Hautberuhigungs-, entzündungshemmenden, sebumregulierenden, Haarwuchs inhibierenden und/oder Antischuppen-Leistung und gegebenenfalls einer optimalen Benefit-Leistung weiterer enthaltener Wirkstoffe, der erfindungsgemäßen Produkte zu gewährleisten.

In einer weiteren erfindungsgemäß bevorzugten Ausführungsform wird die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an festen, teilchenförmigen inerten Füllstoffen in einem Gewichtsverhältnis zueinander von 0,01 - 50, bevorzugt 0,1 - 20, besonders bevorzugt 0,2-10 und außerordentlich bevorzugt 0,3 - 5, ebenfalls außerordentlich bevorzugt 0,5 - 1 eingesetzt, um eine optimale Wärmeentwicklung in Verbindung mit einer optimalen Benefit-Leistung, insbesondere einer optimalen Reinigungs-, Konditionier-, Bleich-, Desinfektions-, Haarfärbe- oder Hautpflegeleistung, der erfindungsgemäßen Produkte zu gewährleisten. Substrat

Erfindungsgemäß bevorzugte Produkte sind dadurch gekennzeichnet, dass das Trägersubstrat ausgewählt ist aus einem Beutel, einem Pad, einem Schwamm, einem porenhaltigen Schwamm, einem maschenhaltigen Schwamm, einem Tuch, einem Pflaster oder einem Bausch.

Erfindungsgemäß bevorzugte Produkte, deren Substrat als "Kissen", "pouch" oder "Beutel" ausgebildet sein können, umfassen einen Beutel, der für Luft und Wasser durchlässig ist. Dieser Beutel kann aus an sich wasserundurchlässigen Materialien gefertigt werden, die durch geeignete Fertigung wasserpermeabel gemacht wurden. So können beispielsweise Folien mit Öffnungen versehen werden. Im Hinblick auf die bevorzugte kosmetische Ausführungsform, bei der der Konsument das erfindungsgemäße Kissen über seine Haut reiben soll, sind allerdings Gewebe, Gewirke oder Vliese aus natürlichen oder synthetischen Fasern als Beutelmaterialien bevorzugt. Besonders bevorzugte Materialien für diese Gewebe, Gewirke oder Vliese sind als natürliche Fasern auf Cellulosebasis Baumwolle, Kapok, Flachs, Leinen, Hanf, Jute, Ramie; als natürliche Fasern auf Eiweißbasis Wolle und andere Tierhaare wie Angora, Alpaka, Kamelhaar, Kaschmir, Lama, Mohair, Cashoora und Wikunja, Seide; und als synthetische Fasern Kunstfasern auf Cellulosebasis wie Viskose, Cupro, Modal, Lyocell, Acetat, Triacetat sowie als vollsynthetische Fasern Perlon oder Nylon bzw. andere Polyamide, Polyacryl, Polyester, Polypropylen, Polyurethan, Modacryl, Elastan.

Erfindungsgemäß besonders bevorzugt können solche wasserdurchlässigen Beutel sein, deren Beutelmaterial strukturiert oder genoppt ist, um bei der Reinigung gleichzeitig einen mechanisch-abrasiven Effekt zu erzielen. Die Struktur kann durch Poren, Vertiefungen, Prägungen und Erhöhungen der Oberfläche des Beutelmaterials erzielt werden. Werden geprägte Materialien, insbesondere Vliese, verwendet, so erleichtern große Kavitäten die Aufnahme von Schmutz und Verunreinigungen beim Behandeln der Oberfläche (Haut, Haar, hard surfaces) mit dem erfindungsgemäßen Produkt Die Reinigungswirkung kann gegenüber ungeprägten Substraten um ein Vielfaches gesteigert werden. Bei Substraten in Pad- oder Schwammform aus porösem Material erfolgt eine verbesserte Schmutzaufnahme analog durch größere Poren an der Oberfläche des Substrates.

Geeignete Noppen werden bevorzugt durch das (beispielsweise) punktförmige (bzw. kleinflächige) Aufbringen von kleinen Mengen eines geschmolzenen Kunststoffs, der zu abrasiv wirkenden Strukturen erstarrt, auf die Oberfläche des Beutelmaterials erhalten. Hierzu wird ausdrücklich Bezug genommen auf die Offenbarung in der Research Disclosure 1996-382014 A, publiziert am 20.01.1996, sowie auf EP 1283019 A1.

Geeignete Noppen werden auch durch das Aufbringen, insbesondere das Aufkleben, von (beispielsweise mineralischen) Abrasivpartikeln, auf die Oberfläche des Beutelmaterials erhalten.

Entsprechende Strukturierungen und Noppen sind nicht nur für die Substratform "Beutel", sondern auch für Pads, Tücher und Bäusche möglich und, je nach Anwendungsgebiet des erfindungsgemäßen Produkts, erfindungsgemäß bevorzugt.

Weitere erfindungsgemäß bevorzugte Produkte sind dadurch gekennzeichnet, dass der Beutel oder das Tuch aus Vlies besteht. Im Rahmen der vorliegenden Erfindung kennzeichnet der Begriff "Vlies" zusammenhängende Fasermassen, die aus vielen einzelnen Stapeln unterschiedlicher Länge und Feinheit bestehen.

Erfindungsgemäß bevorzugte Trägersubstrate, insbesondere Tücher und Beutel, können sowohl aus wasserlöslichen (wie z. B. Hygiene- und Toilettenpapier) als auch aus wasserunlöslichen Materialien (z.B. einem Vlies) bestehen. Wasserunlösliche Materialien sind erfindungsgemäß bevorzugt. Erfindungsgemäß bevorzugt werden in Kombination mit den erfindungsgemäßen Benefit-Zusammensetzungen "trockene" Substrate eingesetzt, die bevorzugt aus Papier, Zellstoff, Watte, Schaumstoff oder Vlies bestehen, insbesondere aus wasserstrahlverfestigtem ("hydroentangled, spun-lace") und/oder wasserstrahlgeprägtem ("hydroembossed") Vlies. Derartige Vliese können Makroprägungen jeden gewünschten Musters aufweisen. Die zu treffende Auswahl richtet sich zum einen nach der aufzubringenden Benefit-Zusammensetzung und zum anderen nach dem Einsatzgebiet des erfindungsgemäßen Produktes.

Die Substrate können bevorzugt auch als Bausch, gelochtes Vlies oder Netz ausgeführt sein.

Falls das erfindungsgemäße Produkt als Substrat ein geprägtes Vlies umfasst, ist es erfindungsgemäß bevorzugt, wenn, bezogen auf das ungeprägte Vlies, die Dicke des Vlieses mit den durch die Prägung erzeugten Erhebungen ungefähr doppelt so dick ist. In weiteren bevorzugten erfindungsgemäßen Ausführungsformen ist das geprägte Vlies zwischen 5 % und 50 %, ganz besonders bevorzugt zwischen 10 % und 25 % dicker als das ungeprägte Tuch.

Es hat sich weiterhin als erfindungsgemäß bevorzugt herausgestellt, wenn das Tuch-Substrat ein Flächengewicht von 20 bis 120 g/m², bevorzugt 30 bis 80 g/m², besonders bevorzugt 40 bis 60 g/m², außerordentlich bevorzugt 45 - 55 g / m², aufweist (gemessen bei 20 °C ± 2 °C und bei einer Feuchtigkeit der Raumluft von 65 % ± 5 % für 24 Stunden).

Die bevorzugte Dicke erfindungsgemäß bevorzugter Vlies- oder Papiermaterialien für Tuch- oder Beutelsubstrate beträgt 0,2 mm bis 2 mm, insbesondere 0,4 mm bis 1,5 mm, ganz besonders bevorzugt 0,6 mm bis 0,9 mm.

Falls das erfindungsgemäße Produkt als Substrat ein gelochtes Substrat umfasst, ist es erfindungsgemäß bevorzugt, wenn die Anzahl der Löcher pro Quadratzentimeter Substrat mindestens 10, bevorzugt mindestens 15 beträgt. Bevorzugte Lochzahlen sind 15-80 pro cm², besonders bevorzugt sind 24 - 60 Löcher pro cm², außerordentlich bevorzugt sind 30 - 49 Löcher pro cm².

Falls das erfindungsgemäße Produkt als Substrat ein gelochtes Vlies umfasst, ist es erfindungsgemäß bevorzugt, wenn die Anzahl der Löcher pro Quadratzentimeter Vlies mindestens 10, bevorzugt mindestens 15 beträgt. Bevorzugte Lochzahlen sind 15-80 pro cm², besonders bevorzugt sind 24 - 60 Löcher pro cm², außerordentlich bevorzugt sind 30 - 49 Löcher pro cm².

Gelochte Vliese werden bevorzugt durch Wasserstrahlprägung, die bevorzugt gleichzeitig mit der Wasserstrahlverfilzung erfolgt, hergestellt. Ebenfalls bevorzugt kann es sein, dass die - bevorzugt wasserstrahlverfilzten - Vliese nachträglich gestanzt werden.

Bevorzugte Substrat-Vliese sind einlagig. Eine mehrlagige Ausführungsform ist aber ebenfalls möglich. Als Ausgangsmaterialien für den Vliesstoff des Tuches oder Beutels können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstofffasern angeführt. Die vorliegende Erfindung ist aber nicht auf die genannten Materialien beschränkt, sondern es können eine Vielzahl weiterer Fasern zur Vliesherstellung eingesetzt werden. Es ist erfindungsgemäß besonders bevorzugt, wenn die eingesetzten Fasern nicht wasserlöslich sind.

In einer bevorzugten Ausführungsform des Vlieses oder Beutels bestehen die Fasern aus einer Mischung aus 60 % bis 80 % Viskose und 40% bis 20 % PET. Besonders bevorzugt ist eine Mischung aus 70 % Viskose und 30 % PET.

Besonders bevorzugt sind auch Fasern aus hochfesten Polymeren wie Polyamid, Polyester und/oder hochgerecktem Polyethylen. Darüber hinaus können die Fasern auch eingefärbt sein, um die optische Attraktivität des Substrates betonen und/oder erhöhen zu können. Die Fasern können zusätzlich UV-Stabilisatoren und/oder Konservierungsmittel enthalten.

Die zur Bildung erfindungsgemäß bevorzugter Substrate eingesetzten Fasern weisen vorzugsweise eine Wasseraufnahmerate von mehr als 60 mm/10 min (gemessen mit dem EDANA Test 10.1-72), insbesondere mehr als 80 mm/10 min auf. Ferner weisen die zur Bildung erfindungsgemäß bevorzugter Substrate eingesetzten Fasern vorzugsweise ein Wasseraufnahmevermögen von mehr als 5 g Wasser/g Fasern (gemessen mit dem EDANA Test 10.1-72), insbesondere mehr als 8 g/g, besonders bevorzugt von 8,5-10 g/g, auf.

Weitere besonders bevorzugte Substrate im Sinne der vorliegenden Erfindung weisen eine Reißkraft auf von mindestens 60 Newton/50 mm, bevorzugt mindestens 80 Newton/50 mm (trocken, in Maschinenrichtung) und/oder mindestens 20 Newton/50 mm, bevorzugt mindestens 30 Newton/ 50 mm (trocken, in Querrichtung) und/oder mindestens 4 Newton/50 mm, bevorzugt mindestens 60 Newton/50 mm (in feucht-beaufschlagtem Zustand, in Maschinenrichtung) und/oder mindestens 10 Newton/50 mm, bevorzugt mindestens 20 Newton/50 mm (in feucht-beaufschlagtem Zustand, in Querrichtung).

Weitere besonders bevorzugte Substrate im Sinne der vorliegenden Erfindung weisen eine Dehnfähigkeit auf von 15 - 100%, bevorzugt 20 - 50 % (trocken, in Maschinenrichtung) und/oder 40 - 120 %, bevorzugt 50 - 85 % (trocken, in Querrichtung) und/oder 15 - 100%, bevorzugt 20 - 40 % (in feucht-beaufschlagtem Zustand, in Maschinenrichtung) und/oder 40-120 %, bevorzugt 50 - 85 % (in feucht-beaufschlagtem Zustand, in Querrichtung).

Erfindungsgemäß bevorzugte Verfahren zur Herstellung der erfindungsgemäßen Produkte sind dadurch gekennzeichnet, dass folgende Verfahren Anwendung finden können:
Herstellen der Benefit-Zusammensetzung

Die nicht-flüchtigen und mechanisch stabilen Bestandteile der Benefit-Zusammensetzung werden in eine Trockenmühle oder ähnliche Mischapparatur gegeben und vermischt, bis sich eine gleichförmig verteilte Zusammensetzung ergibt. Anschließend werden flüchtige oder mechanisch empfindliche Benefit-Wirkstoffe, beispielsweise Duftstoffe oder verkapselte Wirkstoffe, als eine Phase B in die trockene Mühle unter gleichzeitigem Bewegen (nicht Mahlen) der gesamten Zusammensetzung gesprüht.

In einer anderen bevorzugten Herstellvariante wird das Parfüm nicht aufgesprüht, sondern als geträgertes Parfüm zugemischt.

2. Beaufschlagung des Trägersubstrates mit der Benefit-Zusammensetzung
a) Beschichtung durch Abstreifung. Einer Abstreifvorrichtung (Abstreifbalken, gegenläufiges Rollensystem o.a.) wird kontinuierlich eine fließfähige Imprägnierung zugeführt. Diese wird durch den Kontakt auf ein vorbeilaufendes Substrat übertragen. Anschließend kann eine Trocknung erfolgen.
b) Beschichtung durch Aufsprühung. Die sprühfähige Imprägnierung wird auf das vorbeilaufende Substrat aufgesprüht. Anschließend kann eine Trocknung erfolgen.
c) Beschichtung durch Tauchen. Das Substratmaterial, insbesondere in Tuchform, durchläuft ein mit der flüssigen Imprägnierung gefülltes Bad, oder eine komplette Tuchrolle wird in die Imprägnierung eingetaucht. Anschließend kann eine Trocknung erfolgen.
d) Beschichtung mit einem Pulver. Es kann eine Einarbeitung durch anschließendes Erhitzen oder mechanische Einarbeitung nachgeschaltet sein. Das Pulver kann auch mit einer Klebesubstanz (Polymer, geschmolzenes und wiedererstarrtes Lipid etc.) am Substrat fixiert werden.
e) Verpackung der pulverförmigen Benefit-Zusammensetzung in einem Beutel. Hierzu werden bevorzugt Rollen von ersten und zweiten Substratfolien der verschiedenen Seiten von einer Beschickungsposition abgewickelt. Die Benefit-Zusammensetzung wird in einen Trichter, der über der Beschickungsposition angeordnet ist, gegeben und zwischen die Substratfolien gespeist. Eine für ein erfindungsgemäßes Pouch geeignete Dosis an Benefit-Zusammensetzung wird direkt zwischen den Folien freigesetzt und in einem teilweise gebildeten Beutel oder Säckchen eingefangen. An diesem Punkt werden alle Kanten, die den Beutel definieren, im Schiebewerk verschlossen unter Einschließen der Benefit-Zusammensetzung darin. Schneidwerke trennen dann einen verschlossenen Abschnitt von einem weiteren, wodurch die erfindungsgemäßen Produkte gebildet werden. Eines oder mehrere der erfindungsgemäßen Produkte werden dann in eine Feuchtigkeits-undurchlässige äußere Verpackung, beispielsweise einen laminierten Folienbeutel oder eine verschließbare, insbesondere eine wiederverschließbare, Dose, zur Verhinderung einer Aktivierung des Wärme erzeugenden Systems während der Lagerung, verpackt.

Ultraschallschweißen kann als eine Alternative zum Wärmeverschließen der ersten und zweiten Substrate miteinander angewendet werden. Fadenvernähen, Kleberauftrag oder andere Verschlussmechanismen können ebenfalls angewendet werden.

Erfindungsgemäß bevorzugt ist die Verwendung der erfindungsgemäßen Produkte als Reinigungs-, Hygiene- oder Erfrischungstücher, -pads,- schwämme, -beutel, -bausch oder -pflaster.

Erfindungsgemäß bevorzugt ist auch die Verwendung der erfindungsgemäßen Produkte zur Reinigung und Pflege der Haut sowie die Verwendung von erfindungsgemäßen Produkten als Gesichtstücher, Babytücher, Intimpflegetücher, Deotücher, Sonnenschutztücher (oder analog als Pad, Schwamm, Beutel, Bausch oder Pflaster) und/oder zur Pflege und/oder Reinigung sensibler Haut. Darüber hinaus werden die erfindungsgemäßen Produkte bevorzugt zur Reinigung, Politur und Pflege von Bedarfsgegenständen des täglichen Lebens (z.B. Geschirr, Tisch- und Schrankflächen, Autos, Fußböden, Kacheln, sanitäre Einrichtungen, Fenster, Glas) verwendet.

Ein weiteres bevorzugtes erfindungsgemäßes Produkt ist dadurch gekennzeichnet, dass die Benefit-Zusammensetzung eine Reinigungszusammensetzung, bevorzugt zur Haar-, Gesichts- oder Körperreinigung oder zum Entfernen von Make-up, darstellt.

Ein weiteres bevorzugtes erfindungsgemäßes Produkt ist dadurch gekennzeichnet, dass die Benefit-Zusammensetzung eine konditionierende Zusammensetzung darstellt.

Ein weiteres bevorzugtes erfindungsgemäßes Produkt ist dadurch gekennzeichnet, dass die konditionierende Zusammensetzung eine Zusammensetzung für die persönliche Pflege ist, ausgewählt aus:
- 2-in1-Zusammensetzungen zur Reinigung und gleichzeitigen Pflege der Gesichts- und/oder Körperhaut und/oder der Haare,
- Sonnenschutzzusammensetzungen,
- Anti-Akne-Zusammensetzungen,
- Desinfektionszusammensetzungen,
- Schmink- oder Abdeckzusammensetzungen,
- Zusammensetzungen zur Veränderung der Haarfarbe.

Ein weiteres bevorzugtes erfindungsgemäßes Produkt ist dadurch gekennzeichnet, dass das Trägersubstrat erste und zweite in Wasser unlösliche Substrate umfasst, wobei mindestens eines für Wasser permeabel ist, wobei die ersten und zweiten wasserunlöslichen Substrate dazwischen einen Bereich bilden, der die Benefit-Zusammensetzung aufnimmt, wobei das erste Substrat ein Spun-Lace- oder ein kardierteslchemisch gebundenes Vliesmaterial umfasst, und das zweite Substrat ein High-Loft-Tuch, das an eine Vliesfolie gebunden ist, die ausgewählt ist aus der Gruppe, bestehend aus schmelzgeblasenen, spunbonded und schmelzgeblasenen/spunbonded Vliesen, umfasst.

Eine in Wasser unlösliche High-Loft-Folie bildet mindestens einen zweiten Teil des zweiten Substrats mit einer High-Loft-Oberfläche an der Auswärtsseite des zweiten Substrats. Das erste Substrat ist von einem anderen Aufbau als von dem zweiten. Insbesondere ist das erste Substrat ein Spun-Lace (wasservernadeltes, "hydroentangled") Vliestextil oder ein kardiertes/chemisch gebundenes Vlies. Schmelzgeblasene oder spunbonded Materialien weisen den Vorteil auf, dass sie in ihrer Verschließbarkeit weniger negativ beeinflusst werden, wenn verunreinigende Teilchen der Benefit-Zusammensetzung unabsichtlich während des Beutelherstellungsverfahrens in die Maschinerie gelangen. Darüber hinaus erlauben schmelzgeblasene und spun-bonded Vliese bessere Verschließbarkeit, wenn sie sandwichartig zwischen dem ersten Substrat und jedem High-Loft-Folien-bildenden Teil des zweiten Substrates angeordnet sind. Die High-Loft-Folie verleiht dem zweiten Substrat erhöhte Belüftung und verbesserte Schäumbarkeitseigenschaften. In den Beuteln, wo eine High-Loft-Seite bevorzugt ist, ist es notwendig, das schmelzgeblasene und/oder spun-bonded Substrat für den zusätzlichen Zweck zu verteilen, um Pulververluste oder Verluste der Benefit-Zusammensetzung durch die High-Loft-Folie hindurch zu verhindern.

Bevorzugte erfindungsgemäße Produkte, die einen Beutel, einen Schwamm, ein Tuch, ein Pad oder einen Bausch als Trägersubstrat umfassen, sind dadurch gekennzeichnet dass sie eine ovale Form mit einer Größe aufweisen, die sich der menschlichen Hand anpasst. Bevorzugte Querschnittsdurchmesser des Substrats liegen im Bereich von 2 - 15 cm, besonders bevorzugt 5-10 cm.

Bevorzugte erfindungsgemäße Produkte, die einen Beutel und eine pulverförmige Benefit-Zusammensetzung umfassen, sind dadurch gekennzeichnet, dass der Beutel mindestens eine Folie aufweist, die aus mindestens einer Schicht eines schmelzgeblasenen oder spun-bonded Vliesmaterials besteht. Besonders bevorzugte Folien für das Beutelmaterial umfassen mindestens zwei Schichten, mindestens drei oder sogar mindestens vier Schichten von Vliesmaterialien, die schmelzgeblasen oder spun-bonded sind. Dabei kann die Folie eine einzige schmelzgeblasene Schicht sein, jedoch stellt sie vorzugsweise eine Kombination von verschiedenen Schichten dar. Das schmelzgeblasene Vliesmaterial kann mit anderen tragenden Vliesmaterialien kombiniert oder laminiert werden, wie spun-bonded Vliesmaterialien, um dem Trägersubstrat beispielsweise Festigkeit zu verleihen. Spun-bonded Vliesmaterialien zeichnen sich durch ein hohes Festigkeits-/ Gewichtsverhältnis, isotrope Festigkeit, hohe Porosität und gute Abriebbeständigkeit aus. Eine bevorzugte Ausführungsform weist ein dreischichtiges Material spun-bonded/schmelzgeblasen/ spun-bonded (SMS) auf. Eine weitere bevorzugte Ausführungsform weist ein vierschichtiges Material spun-bonded/schmelzgeblasen/schmelzgeblasen/spun-bonded (SMMS) auf. In diesen Systemen sind die schmelzgeblasenen Fasern sehr kompakt und wirken als eine Sperre gegen den Verlust an Benefit-Zusammensetzung vor Ingebrauchnahme. Die spun-bonded Schichten sind dabei wegen ihrer Festigkeit und Weichheit bevorzugt.

Das schmelzgeblasene Vliesmaterial kann jedes schmelzgeblasene Vliesmaterial sein, das aus einem thermoplastischen Polymer mit einem Schmelzpunkt größer als etwa 50°C hergestellt ist. Ein außerordentlich bevorzugtes Polymer ist Polypropylen. Andere bevorzugte thermoplastische Polymere sind ausgewählt aus Poly(butylenterephthalat), Polycaprolactam, Poly(ethylenterephthalat) und Polyethylen. Spunbonding erfordert Extrudieren einer Vielzahl von kontinuierlichen thermoplastischen Polymersträngen durch eine Vielzahl von Düsenöffnungen in einer Abwärtsrichtung auf eine bewegte Oberfläche, wo die extrudierten Stränge statistisch verteilt gesammelt werden. Die statistisch abgeschiedenen Stränge werden dann miteinander in einem erhitzten Walzspalt verbunden, um ausreichende Integrität für das erhaltene Vliesmaterial von kontinuierlichen Fasern bereitzustellen.

Schmelzblasen unterscheidet sich von Spunbonding dahin gehend, dass die extrudierten Polymerstränge aufgebrochen werden und durch einen Druckluftstrom in einzelne Fasern verteilt werden, bevor sie auf der gesammelten Oberfläche abgeschieden werden. Zusätzlich werden die Fasern im Wesentlichen durch die Luft gekühlt, so dass sie nicht wesentlich kristallisieren und/ oder aneinander binden. Das Binden des Flächengebildes, um Integrität und Festigkeit beizubehalten, findet als ein getrennter Stromabwärtsvorgang statt.

Bevorzugte schmelzgeblasene Vliesmaterialien sind durch ein Flächengewicht im Bereich von 20 bis 300 g/m², bevorzugt 80 bis 250 und besonders bevorzugt 100 bis 200 g/m² gekennzeichnet.

Ein besonders bevorzugtes Vliesmaterial ist ein schmelzgeblaseneslspun-bonded Vliessubstrat, das von Polyprop Corporation erhältlich ist.

Ein besonders bevorzugtes Vliesmaterial ist ein Spun-Lace- oder ein kardiertes/chemisch gebundenes Vlies aus in Wasser unlöslichem Material. Besonders bevorzugt ist ein Tuch NC008 (Image Spun Lace), erhältlich von der PGI Corporation.

Falls verwendet, ist die High-Loft-Folie schrittweise fluidpermeabel. Erfindungsgemäß bedeutet "High-Loft", dass die Folie eine Dichte von 0,00005 g/cm³ bis 0,1 g/cm³, vorzugsweise 0,001 g/m³ bis 0,09 g/cm³ und eine Dicke von 1-5 mm aufweist.

Weitere erfindungsgemäß bevorzugte Trägersubstrate sind ausgewählt aus geformten Filmen, Schwämmen, Schäumen, leichten Baumwollstoffen usw. Wenn das Trägersubstrat Fasern umfasst, können die Fasern entweder statistisch angeordnet sein oder sie können kardiert sein (das heißt gekämmt, um vorwiegend in einer Richtung orientiert zu sein).

Für die erfindungsgemäßen Zwecke ist das am meisten bevorzugte High-Loft-Material ein vernadelter Verbundwerkstoff, der von der Union Wadding Corporation vertrieben wird.

Es ist wichtig, dass die Trägersubstratmaterialien beim Reiben des Gegenstands vom Verbraucher nicht leicht zerrissen werden. Bei der erfindungsgemäßen Ausführungsform als Säckchen oder Sachet sollten die erfindungsgemäßen Beutel nicht aufreißen. Stattdessen ist es erfindungsgemäß bevorzugt, dass alle Zusammensetzungskomponenten durch Auflösung durch die permeablen Wände des Beutels nach außen gelangen.

Hautoberflächen, für die die erfindungsgemäßen Gegenstände verwendbar sind, schließen Gesicht, Körper, Kopfhaut, Achseln und auch Beine/Füße ein. Wenn der Gegenstand ein Fußreinigungsprodukt ist, ist es für das Trägersubstrat vorteilhaft, auf einer seiner Seiten grob zu sein, während die zweite Seite des Substrats weich und mild sein kann.

Ein weiterer Gegenstand der vorliegenden Anmeldung ist ein nicht-therapeutisches oder therapeutisches Verfahren zur persönlichen Reinigung und/oder zur persönlichen Pflege, gekennzeichnet durch folgende Verfahrensschritte:
(i) Anfeuchten eines Produkts gemäß einem der Patentansprüche 1-25 mit Wasser und
(ii) Auftragen des angefeuchteten Produkts auf die zu reinigende und/oder pflegende Haut- und/oder Haaroberfläche,
(iii) gegebenenfalls Abspülen und/oder Abwischen und/oder Aussspülen der Benefit-Zusammensetzung.

Bei dem Verfahren ist es erfindungsgemäß bevorzugt, dass Schritt (i) maximal 10 Minuten, bevorzugt maximal 5 Minuten, besonders bevorzugt maximal 1 Minute, vor Schritt (ii) erfolgt.

Ein weiterer Gegenstand der vorliegenden Anmeldung ist die Verwendung eines Produkts gemäß einem der Ansprüche 1 - 10 zur nicht-therapeutischen oder therapeutischen persönlichen Reinigung und/oder zur nicht-therapeutischen oder therapeutischen persönlichen Pflege, wobei das Produkt vor dem Behandlungsschritt mit Wasser angefeuchtet wird.

Bei den Verwendungen ist es erfindungsgemäß bevorzugt, dass das Produkt maximal 10 Minuten, besonders bevorzugt maximal 5 Minuten, außerordentlich bevorzugt maximal 1 Minute vor dem Behandlungsschritt angefeuchtet wird.

### Ausführungsbeispiele

Die nachstehenden Beispiele dienen dazu, die erfindungsgemäßen Ausführungsformen näher zu erläutern, ohne sie hierauf zu beschränken.

Alle Teil-, Prozent- und Verhältnisangaben, auf die hierin und in den Patentansprüchen Bezug genommen wird, sind, sofern nicht anders ausgewiesen, auf das Gewicht der Benefit-Zusammensetzung bezogen.

### BEISPIEL 1

Eine Benefit-Zusammensetzung wurde gemäß der Formulierung in Tabelle I hergestellt. Phase A wurde in einem Hochgeschwindigkeitsschermischer trocken vermischt. Das Parfüm wurde dann auf das erhaltene Pulver als eine Phase B gesprüht. 3 g von dem erhaltenen Pulver wurden dann in ein Päckchen von einem 5 cm × 8 cm-Beutel gegeben. Die Wände des Beutels werden durch Heißversiegelung am Rand des Spun-Lace-Vlieses zu einem Vlies-Säckchen versiegelt.

**Tabelle I: Reinigungsprodukt**

| | Bestandteil | Menge (Gew.-%), bezogen auf die Benefit-Zusammensetzung |
|---|---|---|
| Phase A: | Talkum | 65,00 |
| | Dinatriumlaurylsulfosuccinat | 19,00 |
| | Magnesiumsulfat (wasserfrei) | 15,00 |
| Phase B | Parfüm | 1,00 |

### BEISPIEL 2

Eine weitere Benefit-Zusammensetzung wurde mit den in Tabelle II genannten Bestandteilen hergestellt. Die Zusammensetzung wurde dann in einem wie in Beispiel 1 beschriebenen Beutel verschlossen. Im Unterschied zu Beispiel 1 wurde das Parfüm nicht aufgesprüht, sondern als geträgertes Parfümpulver eingesetzt

**Tabelle II**

| | Bestandteil | Menge (Gew.-%), bezogen auf die Benefit-Zusammensetzung |
|---|---|---|
| Phase A: | Talkum | 73,00 |
| | Elfan AT 84 G | 5,00 |
| | Tego Betain CK D | 8,00 |
| | Magnesiumsulfat (wasserfrei) | 13,00 |
| | Parfümpulver | 1,00 |

### BEISPIELE 3 und 4

Weitere Benefit-Zusammensetzungen wurden gemäß den in Tabelle III und IV genannten Bestandteilen hergestellt. Die Zusammensetzungen wurden dann in einem wie in Beispiel 1 beschriebenen Beutel verschlossen.

**Tabelle III**

| | Bestandteil | Menge (Gew.-%), bezogen auf die Benefit-Zusammensetzung |
|---|---|---|
| Phase A: | Talkum | 72,50 |
| | Elfan AT 84 G | 5,00 |
| | Tego Betain CK D | 8,00 |
| | Magnesiumsulfat (wasserfrei) | 13,00 |
| | Parfümpulver | 1,00 |
| | Herbasec White Tea COS-249/560-C | 0,50 |

**Tabelle IV**

| | Bestandteil | Menge (Gew.-%), bezogen auf die Benefit-Zusammensetzung |
|---|---|---|
| Phase A: | Talkum | 70,00 |
| | REWOPOL SB F 12 P | 5,00 |
| | Tego Betain CK D | 8,00 |
| | Magnesiumsulfat (wasserfrei) | 13,00 |
| | Parfümpulver | 1,00 |
| | Gluadin AGP | 3,00 |

Das erfindungsgemäße Produkt aus Beispiel 1 ist ein bevorzugtes Beispiel für ein Reinigungsprodukt für die Haut. Das Produkt wird mit etwas Wasser angefeuchtet und durchgewalkt, bis sich ein reinigender Schaum entwickelt. Anschließend reibt man mit dem Produkt über die zu behandelnden Oberflächen (Gesichtshaut, Körperhaut). Es ist auch möglich, das Produkt als Maske einen Moment lang (je nach Bedarf eine halbe bis 10, bevorzugt bis 5 Minuten) auf der Haut zu belassen, um den Wärmeeffekt zu genießen. Am Schluss des Reinigungsprozesses wird die Benefit-Zusammensetzung mit Wasser abgespült.

Das erfindungsgemäße Produkt aus Beispiel 2 ist ebenfalls ein bevorzugtes Beispiel für ein Reinigungsprodukt für die Haut, enthaltend eine besonders hautmilde Tensidkombination. Das Produkt wird mit etwas Wasser angefeuchtet und durchgewalkt, bis sich ein reinigender Schaum entwickelt. Anschließend reibt man mit dem Produkt über die zu behandelnden Oberflächen (Gesichtshaut, Korpernaut). Es ist auch möglich, das Produkt als Maske einen Moment lang (je nach Bedarf eine halbe bis 10, bevorzugt 5 Minuten) auf der Haut liegen zu lassen, um den Wärmeeffekt zu genießen. Am Schluss des Reinigungsprozesses wird die Benefit-Zusammensetzung mit Wasser abgespült.

Das erfindungsgemäße Produkt aus Beispiel 3 ist ebenfalls ein bevorzugtes Beispiel für ein Reinigungsprodukt für die Haut, enthaltend eine besonders hautmilde Tensidkombination und zusätzlich einen Wirkstoff gegen die Hautalterung. Das Produkt wird mit etwas Wasser angefeuchtet und durchgewalkt, bis sich ein reinigender Schaum entwickelt. Anschließend reibt man mit dem Produkt über die zu behandelnden Oberflächen (Gesichtshaut, Körperhaut). Es ist auch möglich, das Produkt als Maske einen Moment lang (je nach Bedarf eine halbe bis 10, bevorzugt 5 Minuten) auf der Haut zu belassen, um den Wärmeeffekt zu genießen. Am Schluss des Reinigungsprozesses wird die Benefit-Zusammensetzung mit Wasser abgespült.

Das erfindungsgemäße Produkt aus Beispiel 3 ist ebenfalls ein bevorzugtes Beispiel für ein Reinigungsprodukt für die Haut und das Haar, enthaltend eine besonders hautmilde Tensidkombination und zusätzlich einen konditionierenden, feuchtigkeitsspendenden Wirkstoff. Das Produkt wird mit etwas Wasser angefeuchtet und durchgewalkt, bis sich ein reinigender Schaum entwickelt. Anschließend reibt man mit dem Produkt über die zu behandelnden Oberflächen (Gesichtshaut, Körperhaut, Haupthaar). Es ist auch möglich, das Produkt als Maske einen Moment lang (je nach Bedarf eine halbe bis 5 Minuten) auf der Haut liegen zu lassen, um den Wärmeeffekt zu genießen. Am Schluss des Reinigungsprozesses wird die Benefit-Zusammensetzung mit Wasser abgespült.

Alle Beispielprodukte können selbstverständlich auch für die Reinigung des Haars eingesetzt werden. Alle Beispielprodukte können selbstverständlich auch für die Reinigung harter Oberflächen eingesetzt werden.

### Liste der eingesetzten Rohstoffe

| Rohstoffname | lNCl | Hersteller/Lieferant |
|---|---|---|
| Elfan AT 84 G | SODIUM COCOYL ISETHIONATE, Aktivsubstanz 82 - 86 Gew.%, Wassergehalt max. 2 Gew.-% | Akzo Nobel |
| Tego Betain CK D | COCAMIDOPROPYL BETAINE, Aktivsubstanz min. 82 Gew.%, Wassergehalt max. 3 Gew.-% | Degussa |
| REWOPOL SB F 12 P | Disodium lauryl sulfosuccinate, mind. 95 Gew.-% Feststoffgehalt | Degussa |
| Herbasec White Tea COS-249/560-C | MALTODEXTRIN, CAMELLIA SINENSIS LEAF EXTRACT (pulverförmig) | Cosmetochem |
| Gluadin AGP | HYDROLYZED WHEAT PROTEIN, Trockenrückstand mind. 94 Gew.-% | Cognis |

## Patentansprüche

1. Produkt zur persönlichen Reinigung und/oder zur persönlichen Pflege, umfassend:
a) eine Benefit-Zusammensetzung, die bei Kontakt mit Wasser Hydratationswärme freisetzt und die bei Kontakt mit Wasser kein Kohlendioxid freisetzt, enthaltend
i) mindestens 1 Gew.-% mindestens einer unter Normalbedingungen festen Substanz, die sich in Wasser mit negativer Lösungsenthalpie löst oder in Kontakt mit Wasser Hydratationswärme freisetzt, ausgewählt aus
A. anorganischen Salzen,
B. organischen Salzen,
C. sowie Mischungen hiervon,
ii) und mindestens einen Wirkstoff, ausgewählt aus Tensiden, kosmetischen Wirkstoffen und dermatologischen Wirkstoffen, antibakteriellen Stoffen, Antioxidantien, Blondier- oder Bleichwirkstoffen, Enzymen, Farbstoff(vorprodukt)en, Fettstoffen, Fungiziden, Germiziden, geruchskomplexierenden Substanzen, Hydrophobiermitteln, Riechstoffen, UV-Schutz-Substanzen und/oder weichmachenden Komponenten,
iii) 0-10 Gew.-%, bevorzugt 0,5-7 Gew.%, besonders bevorzugt 1-5 Gew.-% und außerordentlich bevorzugt 2-3 Gew.-% Wasser, jeweils bezogen auf das Gesamtgewicht der Benefit-Zusammensetzung,
iv) mindestens einen unter Normalbedingungen festen, teilchenförmigen inerten Füllstoff, wobei in der Benefit-Zusammensetzung die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an festen, teilchenförmigen inerten Füllstoffen in einem Gewichtsverhältnis zueinander von 0,1-20 enthalten sind; und
b) ein Trägersubstrat,
**dadurch gekennzeichnet, dass** die Benefit-Zusammensetzung pulverförmig und/oder agglomeriert und/oder granuliert vorliegt und weiterhin **dadurch gekennzeichnet, dass** die Benefit-Zusammensetzung nicht gleichzeitig eine oder mehrere Säuren und ein Carbonat- und/oder Hydrogencarbonat-haltiges Material umfasst, und weiterhin **dadurch gekennzeichnet, dass** die Benefit-Zusammensetzung keine Zeolithe enthält.

2. Produkt gemäß einem der vorstehenden Ansprüche, **dadurch gekennzeichnet, dass** die Benefit-Zusammensetzung 0,01 bis 95 Gew.-%, bevorzugt 0,5 - 70 Gew.%, besonders bevorzugt 3-50 Gew.-% und außerordentlich bevorzugt 10-30 Gew.%, jeweils bezogen auf das Gewicht der Benefit-Zusammensetzung, mindestens eines Tensids enthält.

3. Produkt gemäß einem der vorstehenden Ansprüche, **dadurch gekennzeichnet, dass** die Benefit-Zusammensetzung mindestens einen unter Normalbedingungen festen, teilchenförmigen inerten Füllstoff enthält, **dadurch gekennzeichnet, dass** in der Benefit-Zusammensetzung die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an festen, teilchenförmigen inerten Füllstoffen in einem Gewichtsverhältnis zueinander von 0,2 - 10, bevorzugt 0,3 - 5, besonders bevorzugt 0,5-1, enthalten sind.

4. Produkt gemäß einem der vorstehenden Ansprüche, **dadurch gekennzeichnet, dass** die Benefit-Zusammensetzung mindestens einen verkapselten Benefit-Wirkstoff enthält.

5. Produkt gemäß einem der vorstehenden Ansprüche, **dadurch gekennzeichnet, dass** in der Benefit-Zusammensetzung die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an Tensid in einem Gewichtsverhältnis zueinander von 0,1 - 100, bevorzugt 0,2 - 50, besonders bevorzugt 0,5 - 20 und außerordentlich bevorzugt 1-10, ebenfalls außerordentlich bevorzugt 1,5-3, enthalten sind.

6. Produkt gemäß einem der vorstehenden Ansprüche, **dadurch gekennzeichnet, dass** in der Benefit-Zusammensetzung die Gesamtmenge an unter Normalbedingungen festen Substanzen, die sich in Wasser mit negativer Lösungsenthalpie lösen oder in Kontakt mit Wasser Hydratationswärme freisetzen, und die Gesamtmenge an kosmetischen oder dermatologischen Wirkstoffen in einem Gewichtsverhältnis zueinander von 1 - 1000, bevorzugt 2 - 500, besonders bevorzugt 5-100 und außerordentlich bevorzugt 10-50, ebenfalls außerordentlich bevorzugt 15-30, enthalten sind.

7. Produkt gemäß einem der vorstehenden Ansprüche, **dadurch gekennzeichnet, dass** die Benefit-Zusammensetzung eine Reinigungszusammensetzung, bevorzugt zur Haar-, Gesichts- oder Körperreinigung und/oder zum Entfernen von Make-up, darstellt.

8. Produkt gemäß einem der vorstehenden Ansprüche, **dadurch gekennzeichnet, dass** die Benefit-Zusammensetzung eine konditionierende Zusammensetzung darstellt.

9. Produkt gemäß einem der vorstehenden Ansprüche, **dadurch gekennzeichnet, dass** das Trägersubstrat ausgewählt ist aus einem Beutel, der für Wasser und Luft durchlässig ist, einem Pad, einem Schwamm, einem maschenhaltigen Schwamm, einem Tuch, einem Pflaster, einer Maske oder einem Bausch, oder **dadurch gekennzeichnet, dass** das Trägersubstrat erste und zweite in Wasser unlösliche Substrate umfasst, wobei mindestens eines für Wasser permeabel ist, wobei die ersten und zweiten Substrate dazwischen einen Bereich bilden, der die Reinigungszusammensetzung aufnimmt, wobei das erste Substrat ein Spun-Lace- oder ein kardiertes/chemisch gebundenes Vliesmaterial umfasst, und das zweite Substrat ein High-Loft-Tuch, das an eine Vliesfolie gebunden ist, die ausgewählt ist aus der Gruppe, bestehend aus schmelzgeblasenen Vliesen, spun-bonded Vliesen und schmelzgeblasenenispun-bonded Vliesen, umfasst.

10. Produkt gemäß einem der vorstehenden Ansprüche, **dadurch gekennzeichnet, dass** die Benefit-Zusammensetzung mindestens eine unter Normalbedingungen feste Substanz, die sich in Wasser mit negativer Lösungsenthalpie löst oder in Kontakt mit Wasser Hydratationswärme freisetzt, in einer Gesamtmenge von 10-30 Gew.-% und bevorzugt 15-25 Gew.-%, enthält

11. Verfahren zur nicht-therapeutischen persönlichen Reinigung und/oder zur nicht-therapeutischen persönlichen Pflege, **gekennzeichnet durch** folgende Verfahrensschritte:
(i) Anfeuchten eines Produkts gemäß einem der Ansprüche 1 - 10 mit Wasser und
(ii) Auftragen des angefeuchteten Produkts auf die zu reinigende und/oder pflegende Haut- und/oder Haaroberfläche,
(iii) gegebenenfalls Abspülen und/oder Abwischen und/oder Ausspülen der Benefit-Zusammensetzung.

12. Verwendung eines Produkts gemäß einem der Ansprüche 1-10 zur nicht-therapeutischen persönlichen Reinigung und/oder zur nicht-therapeutischen persönlichen Pflege, wobei das Produkt vor dem Behandlungsschritt mit Wasser angefeuchtet wird.

## Claims

1. Product for personal cleansing and/or for personal care, containing:
a. a benefit-composition that releases heat of hydration on contact with water and does not release any carbon dioxide on contact with water, comprising
i) at least 1 wt % of at least one substance that is solid under normal conditions, which dissolves with negative enthalpy of solution in water or releases heat of hydration in contact with water, selected from
A. inorganic salts,
B. organic salts,
C. as well as mixtures thereof,
ii) and at least one active substance, selected from surfactants, cosmetic active substances and dermatological active substances, antibacterials, antioxidants, blonding or bleaching agents, enzymes, dyes or dye precursors, fats, fungicides, germicides, odour-complexing substances, water-repellent agents, fragrances, UV-protection substances and/or plasticising components.
iii) 0-10 wt %, preferably 0.5-7 wt %, particularly preferably 1-5 wt
% and extremely preferably 2-3 wt % water, each relative to the total weight of the benefit-composition,
iv) at least one particulate, inert filler that is solid under normal conditions, wherein the benefit-composition comprises the total amount of substances that are solid under normal conditions, which dissolve with negative enthalpy of solution in water or release heat of hydration in contact with water, and the total amount of solid, particulate inert fillers in a weight ratio to one another of 0.1-20, and
b) a carrier substrate,
**characterised in that** the benefit-composition is in the form of a powder and/or an agglomerate and/or a granulate and further **characterised in that** the benefit-composition does not simultaneously contain one or more acids and a carbonate-containing and/or hydrogen carbonate-containing material, and further **characterised in that** the benefit-composition does not comprise zeolites.

2. Product according to one of the previous claims, **characterised in that** the benefit-composition, comprises 0.01 to 95 wt %, preferably 0.5 to 70 wt %, particularly preferably 3 to 50 wt %, and extremely preferably 10 to 30 wt % of at least one surfactant, each with respect to the weight of the benefit-composition.

3. Product according to one of the previous claims, **characterised in that** the benefit-composition comprises at least one particulate, inert filler that is solid under normal conditions, **characterised in that** the benefit-composition comprises the total amount of substances that are solid under normal conditions, which dissolve with negative enthalpy of solution in water or release heat of hydration in contact with water, and the total amount of solid, particulate inert fillers in a weight ratio to one another of 0.2 - 10, preferably 0.3 - 5, particularly preferably 0.5 - 1.

4. Product according to one of the previous claims, **characterised in that** the benefit-composition comprises at least one encapsulated benefit-active substance.

5. Product according to one of the previous claims, **characterised in that** the benefit-composition comprises the total amount of substances that are solid under normal conditions, which dissolve with negative enthalpy of solution in water or release heat of hydration in contact with water, and the total amount of surfactant in a weight ratio to one another of 0 1 - 100, preferably 0.2 - 50, particularly preferably 0.5 - 20 and extremely preferably 1.5 - 3.

6. Product according to one of the previous claims, **characterised in that** the benefit-composition comprises the total amount of substances that are solid under normal conditions, which dissolve with negative enthalpy of solution in water or release heat of hydration in contact with water, and the total amount of cosmetic or dermatologically active substances in a weight ratio to one another of 1 - 1000, preferably 2 - 500, particularly preferably 5 - 100 and extremely preferably 15 - 30.

7. Product according to one of the previous claims, **characterised in that** the benefit-composition represents at least one cleaning composition, preferably for cleaning hair, the face or the body and/or for removing make-up.

8. Product according to one of the previous claims, **characterised in that** the benefit-composition represents a conditioning composition.

9. Product according to one of the previous claims, **characterised in that** the carrier substrate is selected from a pouch that is permeable to water and air, a pad, a sponge, a stitched sponge, a woven fabric, a patch, a mask or a pad, or **characterised in that** the carrier substrate contains first and second water-insoluble substrates, wherein at least one is permeable to water, wherein the first and second substrates form a region between them that holds the cleaning composition, wherein the first substrate contains a spun-lace non-woven material or a carded/chemically bonded non-woven material, and the second substrate contains a high-loft woven fabric that is bonded to a non-woven film that is selected from the group consisting of melt-blown non-wovens, spun bonded non-wovens and melt-blowntspun bonded non-wovens.

10. Product according to one of the previous claims, **characterised in that** the benefit-composition comprises at least one substance that is solid under normal conditions, which dissolves with negative enthalpy of solution in
water or releases heat of hydration in contact with water, in a total amount of 10-30 wt % and preferably 15 - 25 wt %.

11. Method for non-therapeutic personal cleansing and/or for non-therapeutic personal care, **characterised by** the following procedural steps:
(i) moistening a product according to one of the claims 1-10 with water and
(ii) applying the moistened product onto the surface of the skin and or hair to be cleaned and/or cared for,
(iii) optionally rinsing off and/or wiping off and/or rinsing out the benefit-composition.

12. Use of a product according to one of the claims 1- 10 for non-therapeutic personal cleansing and/or for non-therapeutic personal care, wherein the prod uct is moistened prior to the treatment step with water.

## Revendications

1. Produit pour l'hygiène personnelle et/ou pour les soins personnels, comprenant :
a) une composition bénéfique qui, en contact avec l'eau, libère de la chaleur d'hydratation et, en contact avec l'eau, ne libère aucun dioxyde de carbone, contenant
i) au moins 1 % en poids d'au moins une substance solide dans des conditions normales, qui se dissout dans l'eau avec une enthalpie de dissolution négative ou qui libère, en contact avec l'eau, de la chaleur d'hydratation, choisie parmi:
A. des sels inorganiques ;
B. des sels organiques ;
C. et des mélanges desdits sels ;
ii) et au moins une substance active choisie parmi des agents tensioactifs, des substances actives cosmétiques et des substances actives dermatologiques, des substances antibactériennes, des antioxydants, des substances actives de décoloration ou de blanchiment, des enzymes, des (précurseurs de) colorants, des graisses, des fongicides, des germicides, des substances complexant les odeurs, des agents rendant hydrophobe, des fragrances, des substances de protection contre le rayonnement ultraviolet et/ou des composants plastifiants ;
iii) de 0 à 10 % en poids, de préférence de 0,5 à 7 % en poids, de manière particulièrement préférée de 1 à 5 % en poids et de manière extraordinairement préférée de 2 à 3 % en poids d'eau, chaque fois rapportés au poids total de la composition bénéfique ;
iv) au moins une matière de charge inerte, solide dans des conditions normales, de forme particulaire, la quantité totale, dans la composition bénéfique, des substances solides dans des conditions normales, qui se dissolvent dans l'eau avec une enthalpie de dissolution négative ou qui libèrent en contact avec l'eau de la chaleur d'hydratation, et la quantité totale dans ladite composition bénéfique des matières de charge inertes, solides, de forme particulaire étant présentes dans un rapport pondéral réciproque de 0,1 à 20; et
b) un substrat faisant office de support ;
**caractérisé en ce que** la composition bénéfique est présente sous forme de poudre et/ou sous forme d'agglomérat et/ou sous forme de granulés et en outre, **caractérisé en ce que** la composition bénéfique ne comprend pas simultanément un ou plusieurs acides et une matière contenant du carbonates et/ou de l'hydrogénocarbonate, et en outre **caractérisé en ce que** la composition bénéfique ne contient aucune zéolithe.

2. Produit selon l'une quelconque des revendications précédentes, **caractérisé en ce que** la composition bénéfique contient de 0,01 à 95 % en poids, de préférence de 0,5 à 70 % en poids, de manière particulièrement préférée de 3 à 50 % en poids et de manière extraordinairement préférée de 10 à 30 % en poids, chaque fois rapportés au poids de la composition bénéfique, d'au moins un agent tensioactif.

3. Produit selon l'une quelconque des revendications précédentes, **caractérisé en ce que** la composition bénéfique contient au moins une
matière de charge inerte, solide dans des conditions normales, de forme particulaire, **caractérisé en ce que**, dans la composition bénéfique, la quantité totale des substances solides dans des conditions normales, qui se dissolvent dans l'eau avec une enthalpie de dissolution négative ou qui libèrent en contact avec l'eau de la chaleur d'hydratation, et la quantité totale des matières de charge inertes, solides, de forme particulaire sont présentes dans un rapport pondéral réciproque de 0,2 à 10, de préférence de 0,3 à 5, de manière particulièrement préférée de 0,5 à 1.

4. Produit selon l'une quelconque des revendications précédentes, **caractérisé en ce que** la composition bénéfique contient au moins une substance active bénéfique à l'état encapsulé.

5. Produit selon l'une quelconque des revendications précédentes, **caractérisé en ce que**, dans la composition bénéfique, la quantité totale des substances solides dans des conditions normales, qui se dissolvent dans l'eau avec une enthalpie de dissolution négative ou qui libèrent en contact avec l'eau de la chaleur d'hydratation, et la quantité totale de l'agent tensioactif sont présentes dans un rapport pondéral réciproque de 0,1 à 100, de préférence de 0,2 à 50, de manière particulièrement préférée de 0,5 à 20, et de manière extraordinairement préférée de 1 à 10, également de manière extraordinairement préférée de 1,5 à 3.

6. Produit selon l'une quelconque des revendications précédentes, **caractérisé en ce que**, dans la composition bénéfique, la quantité totale des substances solides dans des conditions normales, qui se dissolvent dans l'eau avec une enthalpie de dissolution négative ou qui libèrent en contact avec l'eau de la chaleur d'hydratation, et la quantité totale des substances actives cosmétiques ou dermatologiques sont présentes dans un rapport pondéral réciproque de 1 à 1000, de préférence de 2 à 500, de manière particulièrement préférée de 5 à 100, et de manière extraordinairement préférée de 10 à 50, également de manière extraordinairement préférée de 15 à 30.

7. Produit selon l'une quelconque des revendications précédentes, **caractérisé en ce que** la composition bénéfique représente une composition de nettoyage de préférence pour l'hygiène capillaire, faciale ou corporelle et/ou pour le démaquillage.

8. Produit selon l'une quelconque des revendications précédentes, **caractérisé en ce que** la composition bénéfique représente une composition revitalisante.

9. Produit selon l'une quelconque des revendications précédentes, **caractérisé en ce que** le substrat faisant office de support est choisi parmi un sachet qui est perméable à l'eau et à l'air, un tampon, une éponge, une éponge à mailles, un tissu, un emplâtre, un masque ou une boule de coton, ou **caractérisé en ce que** le substrat faisant office de support comprend des premier et deuxième substrats insolubles dans l'eau, au moins un étant perméable à l'eau, les premier et deuxième substrats formant entre eux une zone qui absorbe la composition de nettoyage, le premier substrat comprenant un non-tissé lacé par filage ou bien un non-tissé lié par cardage/par voie chimique, et le deuxième substrat comprenant un tissu à gonflant volumineux, qui est lié à un non-tissé qui est choisi parmi le groupe constitué par des non-tissés obtenus par un procédé de fusion-soufflage, des non-tissés de type filé-lié et des non-tissés obtenus par un procédé de fusion-soufflage/de type filé-lié.

10. Produit selon l'une quelconque des revendications précédentes, **caractérisé en ce que** la composition bénéfique contient au moins une substance solide dans des conditions normales, qui se dissout dans l'eau avec une enthalpie de dissolution négative ou qui libère en contact avec l'eau de la chaleur d'hydratation en une quantité totale de 10 à 30 % en poids et de préférence de 15 à 25 % en poids.

11. Procédé pour l'hygiène personnelle non thérapeutique et/ou pour les soins personnels non thérapeutiques, **caractérisé par** les étapes opératoires suivantes :
(i) humidification légère du produit selon l'une quelconque des revendications 1 à 10 avec de l'eau,
(ii) application du produit légèrement humidifié sur la surface de la peau et/ou sur la surface des cheveux à nettoyer et/ou à entretenir ;
(iii) le cas échéant élimination de la composition bénéfique par lavage et/ou par essuyage et/ou par rinçage.

12. Utilisation d'un produit selon l'une quelconque des revendications 1 à 10, pour l'hygiène personnelle non thérapeutique et/ou pour les soins personnels non thérapeutiques, le produit étant humidifié légèrement avec de l'eau avant l'étape de traitement.
